# EUROPEAN PATENT SPECIFICATION

(11) **EP 3 313 442 B1**
(45) Date of publication and mention of the grant of the patent: **16.10.2019**
(21) Application number: 16732589.3
(22) Date of filing: 23.06.2016
(51) Int. Cl.: A61K 39/395

(54) **ANTIBODIES AGAINST HUMAN CSF-1R FOR USE IN INDUCING LYMPHOCYTOSIS IN LYMPHOMAS OR LEUKEMIAS**
ANTIKÖRPER GEGEN MENSCHLICHES CSF-1R FÜR DIE INDUZIERUNG VON LYMPHOZYTOSE IN LYMPHOMEN ODER LEUKÄMIEN
ANTICORPS CONTRE LE CSF-1R HUMAIN PERMETTANT D'INDUIRE UNE LYMPHOCYTOSE DANS LES LYMPHOMES OU LES LEUCÉMIES

(30) Priority: 24.06.2015 EP 15173638; 13.01.2016 EP 16151129
(43) Date of publication of application: 02.05.2018
(73) Proprietor: F. Hoffmann-La Roche AG, 4070 Basel (CH)
(72) Inventor: BERTILACCIO, Maria Teresa Sabrina, 20146 Milano (IT); CALIGARIS-CAPPIO, Federico, 20090 Segrate (IT); GALLETTI, Giovanni, 20092 Cinisello Balsamo (MI) (IT); KLEIN, Christian, 8906 Bonstetten (CH); RIES, Carola, 82377 Penzberg (DE)
(74) Representative: Burger, Alexander
(86) International application number: PCT/EP2016/064611
(87) International publication number: WO 2016/207312

(56) References cited:
- WO-A1-2013/004806
- WO-A2-2005/044859
- US-A1- 2011 243 947
- US-A1- 2015 073 129

## Description

The present invention relates antibodies against human CSF-1R useful in inducing lymphocytosis of lymphomas or leukemias. The invention further relates to the combination therapy of specific antibodies which bind human CSF-1R with specific antibodies which bind human CD20 and to a method of targeting a CD20 expressing cancer with an anti-CD20 antibody in combination with a CSF-1R inhibitor for preventing escape from CD20 targeting therapies by targeting macrophages.

### Background of the Invention

### CSF-1R and CSF-1R antibodies

The human CSF-1 receptor (CSF-1R; colony stimulating factor 1 receptor; synonyms: M-CSF receptor; Macrophage colony-stimulating factor 1 receptor, Fms proto-oncogene, c-fms, SEQ ID NO: 62) is known since 1986 (Coussens, L., et al., Nature 320 (1986) 277-280). CSF-1R is a growth factor and encoded by the c-fms proto-oncogene (reviewed e.g. in Roth, P., and Stanley, E.R., Curr. Top. Microbiol. Immunol. 181 (1992) 141-167).

CSF-1R is the receptor for CSF-1 (colony stimulating factor 1, also called M-CSF, macrophage colony-stimulating factor) and mediates the biological effects of this cytokine (Sherr, C.J., et al., Cell 41 (1985) 665-676). The cloning of the colony stimulating factor-1 receptor (CSF-1R) (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628). Recently a second ligand for CSF-1R termed interleukin-34 (IL-34) was identified (Lin, H., et al, Science 320 (2008) 807-811).

Currently two CSF-1R ligands that bind to the extracellular domain of CSF-1R are known. The first one is CSF-1 (colony stimulating factor 1, also called M-CSF, macrophage; SEQ ID NO: 86) and is found extracellularly as a disulfide-linked homodimer (Stanley, E.R. et al., Journal of Cellular Biochemistry 21 (1983) 151-159; Stanley, E.R. et al., Stem Cells 12 Suppl. 1 (1995) 15-24). The second one is IL-34 (Human IL-34; SEQ ID NO: 87) (Hume, D. A., et al, Blood 119 (2012) 1810-1820). The main biological effects of CSF-1R signaling are the differentiation, proliferation, migration, and survival of hematopoietic precursor cells to the macrophage lineage (including osteoclast). Activation of CSF-1R is mediated by its CSF-1R ligands, CSF-1 (M-CSF) and IL-34. Binding of CSF-1 (M-CSF) to CSF-1R induces the formation of homodimers and activation of the kinase by tyrosine phosphorylation (Li, W. et al, EMBO Journal. 10 (1991) 277-288; Stanley, E.R., et al., Mol. Reprod. Dev. 46 (1997) 4-10).

The biologically active homodimer CSF-1 binds to the CSF-1R within the subdomains D1 to D3 of the extracellular domain of the CSF-1 receptor (CSF-1R-ECD). The CSF-1R-ECD comprises five immunoglobulin-like subdomains (designated D1 to D5). The subdomains D4 to D5 of the extracellular domain (CSF-1R-ECD) are not involved in the CSF-1 binding (Wang, Z., et al Molecular and Cellular Biology 13 (1993) 5348-5359). The subdomain D4 is involved in dimerization (Yeung, Y-G., et al Molecular & Cellular Proteomics 2 (2003) 1143-1155; Pixley, F. J., et al., Trends Cell Biol. 14 (2004) 628-638).

Further signaling is mediated by the p85 subunit of PI3K and Grb2 connecting to the PI3K/AKT and Ras/MAPK pathways, respectively. These two important signaling pathways can regulate proliferation, survival and apoptosis. Other signaling molecules that bind the phosphorylated intracellular domain of CSF-1R include STAT1, STAT3, PLCy, and Cbl (Bourette, R.P. and Rohrschneider, L.R., Growth Factors 17 (2000) 155-166).

CSF-1R signaling has a physiological role in immune responses, in bone remodeling and in the reproductive system. The knockout animals for either CSF-1 (Pollard, J.W., Mol. Reprod. Dev. 46 (1997) 54-61) or CSF-1R (Dai, X.M., et al., Blood 99 (2002) 111-120) have been shown to have osteopetrotic, hematopoietic, tissue macrophage, and reproductive phenotypes consistent with a role for CSF-1R in the respective cell types.

Sherr, C.J., et al., Blood 73 (1989) 1786-1793 relates to some antibodies against CSF-1R that inhibit the CSF-1 activity. Ashmun, R.A., et al., Blood 73 (1989) 827-837 relates to CSF-1R antibodies. Lenda, D., et al., Journal of Immunology 170 (2003) 3254-3262 relates to reduced macrophage recruitment, proliferation, and activation in CSF-1-deficient mice results in decreased tubular apoptosis during renal inflammation. Kitaura, H., et al., Journal of Dental Research 87 (2008) 396-400 refers to an anti-CSF-1 antibody which inhibits orthodontic tooth movement. WO 2001/030381 mentions CSF-1 activity inhibitors including antisense nucleotides and antibodies while disclosing only CSF-1 antisense nucleotides. WO 2004/045532 relates to metastases and bone loss prevention and treatment of metastatic cancer by a CSF-1 antagonist disclosing as antagonist anti-CSF-1-antibodies only. WO 2005/046657 relates to the treatment of inflammatory bowel disease by anti-CSF-1-antibodies. US 2002/0141994 relates to inhibitors of colony stimulating factors. WO 2006/096489 relates to the treatment of rheumatoid arthritis by anti-CSF-1-antibodies. WO 2009/026303 and WO 2009/112245 relate to certain anti-CSF-1R antibodies binding to CSF-1R within the first three subdomains (D1 to D3) of the Extracellular Domain (CSF-1R-ECD). WO2011/123381(A1) relates to antibodies against CSF-1R. WO2011/070024 relate to certain anti-CSF-1R antibodies binding to CSF-1R within the dimerization domain (D4 to D5). WO 2013/004806 describes anti-CD20 and anti-GM-CSF antibodies for use in combined therapy against B-cell-related cancer.

### CD20 and CD20 antibodies

The CD20 molecule (also called human B-lymphocyte-restricted differentiation antigen or Bp35) is a hydrophobic transmembrane protein located on pre-B and mature B lymphocytes that has been described extensively (Valentine, M.A., et al., J. Biol. Chem. 264 (1989) 11282-11287; and Einfeld, D.A., et al., EMBO J. 7 (1988) 711-717; Tedder, T.F., et al., Proc. Natl. Acad. Sci. U.S.A. 85 (1988) 208-212; Stamenkovic, I., et al., J. Exp. Med. 167 (1988) 1975-1980; Tedder, T.F., et al., J. Immunol. 142 (1989) 2560-2568). CD20 is expressed on greater than 90 % of B cell non-Hodgkin's lymphomas (NHL) (Anderson, K.C., et al., Blood 63 (1984) 1424-1433) but is not found on hematopoietic stem cells, pro-B cells, normal plasma cells, or other normal tissues (Tedder, T.F., et al., J, Immunol. 135 (1985) 973- 979).

There exist two different types of anti-CD20 antibodies differing significantly in their mode of CD20 binding and biological activities (Cragg, M.S., et al., Blood 103 (2004) 2738-2743; and Cragg, M.S., et al., Blood 101 (2003) 1045-1052). Type I antibodies, as, e.g., rituximab (a non-afucosylated antibody with an amount of fucose of 85 % or higher), are potent in complement mediated cytotoxicity.

Type II antibodies, as e.g. Tositumomab (B1), 11B8, AT80 or humanized B-Ly1 antibodies, effectively initiate target cell death via caspase-independent apoptosis with concomitant phosphatidylserine exposure.

The sharing common features of type I and type II anti-CD20 antibodies are summarized in Table 1.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

### Summary of the Invention

The invention is defined in the appended claims and any other aspects set forth herein not falling within the scope of the claims are for information only.

The invention relates to a combination of an antibody which binds to CSF-1R and an antibody which binds CD 20 for use in inducing lymphocytosis of leukemic cells in lymphomas or leukemias,
wherein the lymphocytosis increases the percentage of CD20 expressing circulating leukemic cells and renders the lymphoma or leukemia susceptible to a treatment with an anti-CD20 antibody, and
wherein the antibody which binds to human CSF-1R used in the combination therapy comprises
   a) a heavy chain variable domain VH of SEQ ID NO:23 and a light chain variable domain VL of SEQ ID NO:24, or
   b) a heavy chain variable domain VH of SEQ ID NO:31 and a light chain variable domain VL of SEQ ID NO:32, or
   c) a heavy chain variable domain VH of SEQ ID NO:39 and a light chain variable domain VL of SEQ ID NO:40, or
   d) a heavy chain variable domain VH of SEQ ID NO:47 and a light chain variable domain VL of SEQ ID NO:48, or
   e) a heavy chain variable domain VH of SEQ ID NO:55 and a light chain variable domain VL of SEQ ID NO:56; and
wherein the antibody which binds to human CD20 used in the combination therapy comprises
   a) a heavy chain variable domain VH of SEQ ID NO:69 and a light chain variable domain VL of SEQ ID NO:76, or
   b) a heavy chain variable domain VH of SEQ ID NO:70 and a light chain variable domain VL of SEQ ID NO:76, or
   c) a heavy chain variable domain VH of SEQ ID NO:71 and a light chain variable domain VL of SEQ ID NO:76, or
   d) a heavy chain variable domain VH of SEQ ID NO:72 and a light chain variable domain VL of SEQ ID NO:76, or
   e) a heavy chain variable domain VH of SEQ ID NO:73 and a light chain variable domain VL of SEQ ID NO:76, or
   f) a heavy chain variable domain VH of SEQ ID NO:74 and a light chain variable domain VL of SEQ ID NO:76, or
   g) a heavy chain variable domain VH of SEQ ID NO:75 and a light chain variable domain VL of SEQ ID NO:76.

The invention further relates to a combination of an antibody which binds to human C SF-1R and an antibody which binds to human CD20 wherein the combination is
i) for use in the treatment of a CD20 expressing cancer; or
ii) for use in stimulating an immune response or function, such as T cell activity; or
iii) for use in stimulating a cell mediated immune response, particularly stimulating cytotoxic T-lymphocytes, stimulating T cell activity, or stimulating macrophage activity; or
iv) for use in delaying progression of cancer; or
v) for use in prolonging the survival of a patient suffering from cancer.
wherein the antibody which binds to human CSF-1R used in the combination therapy is characterized in comprising
a) a heavy chain variable domain VH of SEQ ID NO:23 and a light chain variable domain VL of SEQ ID NO:24, or
b) a heavy chain variable domain VH of SEQ ID NO:31 and a light chain variable domain VL of SEQ ID NO:32, or
c) a heavy chain variable domain VH of SEQ ID NO:39 and a light chain variable domain VL of SEQ ID NO:40, or
d) a heavy chain variable domain VH of SEQ ID NO:47 and a light chain variable domain VL of SEQ ID NO:48, or
e) a heavy chain variable domain VH of SEQ ID NO:55 and a light chain variable domain VL of SEQ ID NO:56;
and wherein the antibody which binds to human CD20 used in the combination therapy is characterized in comprising
a) a heavy chain variable domain VH of SEQ ID NO:69 and a light chain variable domain VL of SEQ ID NO:76, or
b) a heavy chain variable domain VH of SEQ ID NO:70 and a light chain variable domain VL of SEQ ID NO:76, or
c) a heavy chain variable domain VH of SEQ ID NO:71 and a light chain variable domain VL of SEQ ID NO:76, or
d) a heavy chain variable domain VH of SEQ ID NO:72 and a light chain variable domain VL of SEQ ID NO:76, or
e) a heavy chain variable domain VH of SEQ ID NO:73 and a light chain variable domain VL of SEQ ID NO:76, or
f) a heavy chain variable domain VH of SEQ ID NO:74 and a light chain variable domain VL of SEQ ID NO:76, or
g) a heavy chain variable domain VH of SEQ ID NO:75 and a light chain variable domain VL of SEQ ID NO:76.

In one preferred embodiment the combination is for use in the treatment of a CD20 expressing cancer as lymphomas (preferably B-Cell Non-Hodgkin's lymphomas (NHL)) and lymphocytic leukemias.

In one preferred embodiment the combination is for use in the treatment of multiple myeloma.

In one preferred embodiment the combination is for use in the treatment of follicular lymphoma.

In one preferred embodiment the combination is for use in the treatment of Hodgkin's disease.

In one embodiment the combination is for use in the prevention or treatment of metastasis.

In one embodiment the combination is for use in the treatment of inflammatory diseases.

In one embodiment the combination is for use in treating or delaying progression of an immune related disease such as tumor immunity.

In one embodiment the combination is for use in stimulating an immune response or function, such as T cell activity.

The inventors have found that a set of molecular interactions supporting the in vivo dependence of leukemic cells on monocytes/macrophages. The present invention relates to finding that CSF1R inhibition reduces leukemic cell load especially in the bone marrow and increases circulating CD20+ leukemic cells, thus suggesting the therapeutic combination of two different antibodies, one targeting TAMs and the other targeting CD20 molecule on leukemic cells.

Such combination therapies of the specific antibodies described herein show benefits for patients in need of an CSF-1R and CD20 targeting therapy. The inventors found that the administration of an anti-CSFIR antibody induced significant lymphocytosis, which renders lymphomas and leukemias more susceptiple to anti-CD20 and or anti-CD19 antibody treatment. Furthermore, they show that macrophage depletion-associated increase of circulating CD20+ leukemic cells represents a therapeutic opportunity for combination with anti-CD20 antibodies. Based on the increased percentage of CD19+CD20+ circulating leukemic cells, an effective combination therapy with specific anti-CSF-1R and anti-CD20 antibody can be administered to patients afflicted with lymphomas and leukemias, especially CD20 expressing lymphomas and leukemias. More strikingly, the leukemic cell depletion increased significantly when the anti-human CSF1R moAb was associated to GA101. This observation suggested a novel strategy of treatment based upon the combination of two different antibodies, one targeting TAMs and the other targeting CD20+ circulating cells.

### Description of the Figures

- **Figure la-b**: **1a:** Human Monocytes differentiated into macrophages with coculture of GM-CSF or CSF-1 (100ng/ml ligand). After 6 days differentiation addition of hMab 2F11-e7. Cell viability was measured at day 7 of antibody treatment in a CTG Viability Assay (CellTiterGlo® Promega). Calculation of % cell viability: RLU signals from treated cells divided by RLU signal from untreated control without antibody, (n =4).
**1b:** Human Monocytes differentiated into macrophages with GM-CSF (M1) or M-CSF (M2) for 7 days. Phenotype analyzed by indirect fluorescence analysis - staining with anti CD163-PE, anti CD80-PE or anti HLA-DR/DQ/DP-Zenon-Alexa647 labeled. The number in each histogram corresponds to mean ratio fluorescence intensity (MRFI); calculated ratio between mean fluorescence intensity (MFI) of cells stained with the selected antibody (empty histogram) and of corresponding isotype control (negative control; gray filled histogram) (mean ± SD; n ≥ 5).

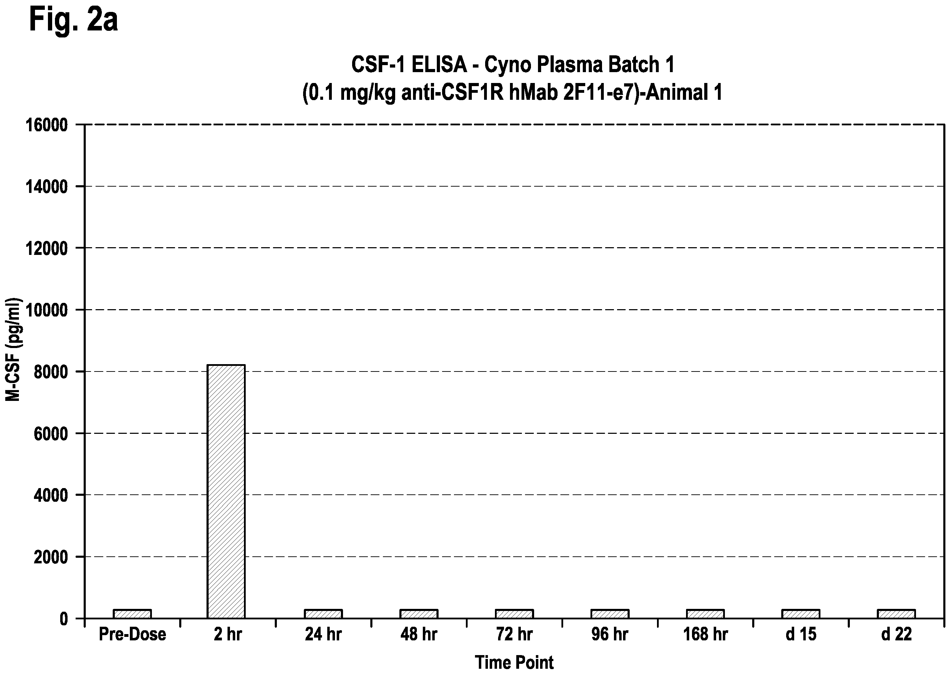

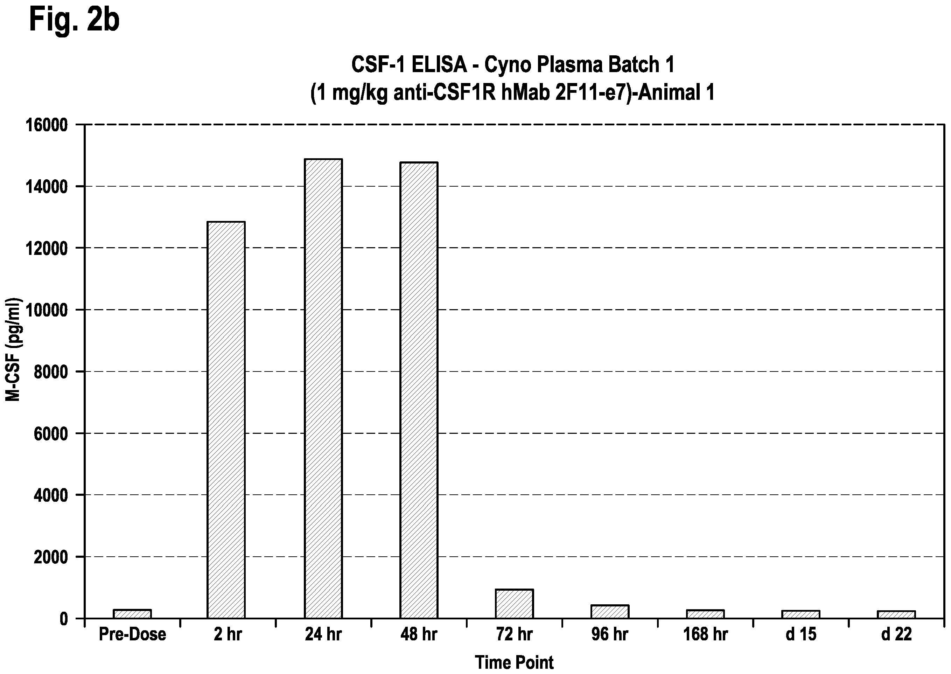

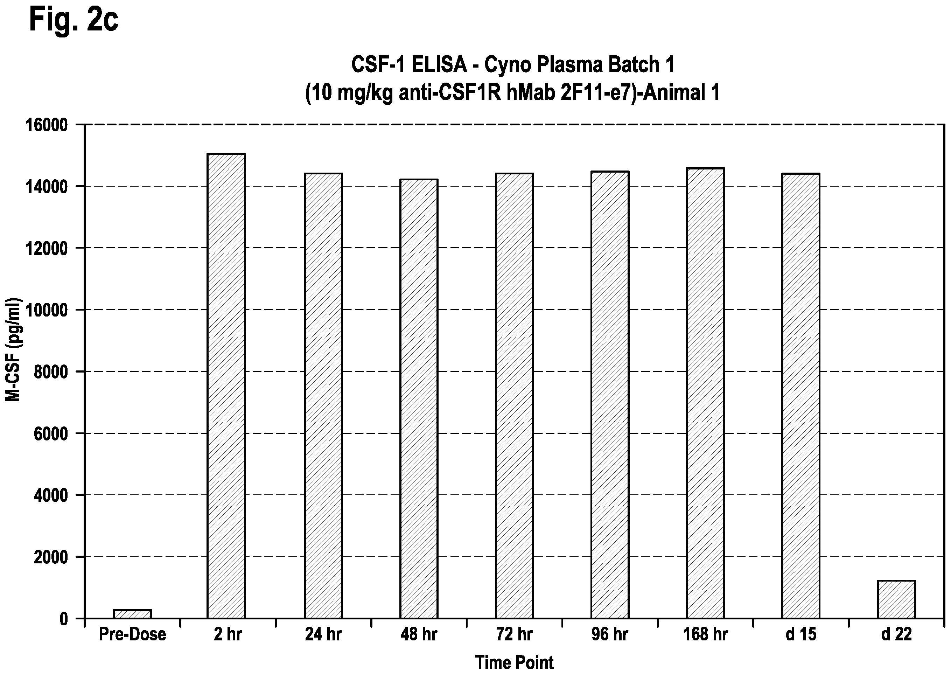

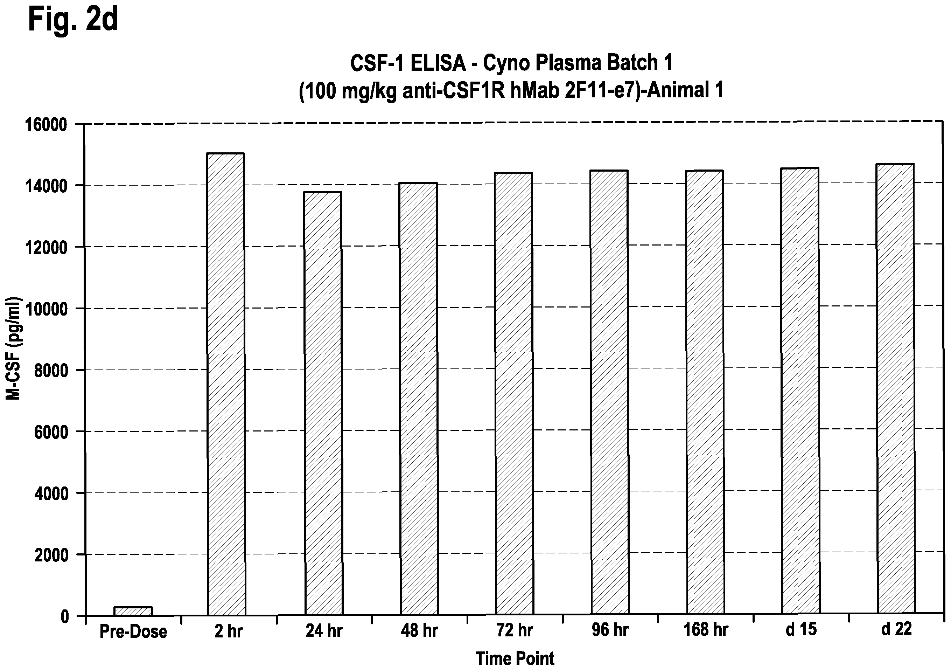

- **Figure 2a-d**: CSF-1 levels in Cynomolgus monkey after application of different dosages of anti-CSF-1R antibody hMab 2F11-e7.
- **Figure 3**: In the presence of TAMs, T cell expansion induced by activation of CD3 and CD28 was suppressed: TAM were isolated from MC38 tumors and co-cultured at the ratios indicated with CFSE-labeled CD8+ T cells in the presence of CD3/CD28 stimulation. T cell proliferation was analyzed after 3 days using bead quantification
of CFSElow dividing cells. One representative experiment out of two is depicted as means + SEM of triplicate wells.
- **Figure 4**: Anti-leukemic effect of anti-CSFIR moAb in the CLL xeno-transplantation system.
(A-B-C-D-E) i.v. MEC1 challenged (day 0) Rag2^{-/-}γ_{c}^{-/-} mice were treated i.v. (day +11, +25) with 30mg/Kg of anti-CSFIR moAb (n=3, white circles) or left untreated (n=4, black circles) and killed at day 27 (A). The mean value of the absolute number of CD11b+ F4/80+ cells gated on CD45+ in SP (B) and BM (C) is shown in graphs. The mean value of the absolute number of human CD19+ cells in the BM is shown in graph (D). The mean value of the absolute number of human CD19+ cells and of Ann- PI+ cells gated on hCD19+ cells in SP is shown in graphs (E). Data are from one representative experiment of three. Statistical analysis: *p < 0.05, **p < 0.01, Student's t test. (F-G-H-I) i.v. MEC1 challenged (day 0) Rag2^{-/-}γ_{c}^{-/-} mice were treated i.v. (day +11, +25) with 30mg/Kg of anti-CSFIR moAb) (n=7, white circles) or left untreated (n=7, black circles) and killed at days 27 and 29, 48h and 96h after the last moAb injection (F). The mean value of the absolute number of human CD19+ cells and of Ann- PI+ cells gated on hCD19+ cells in SP at days 27, 29 is shown in graphs (G). The mean value of the absolute number of CD11b+ F4/80+ cells gated on CD45+ in SP at day 27 is shown in graph (H). The mean value of the absolute number of CD11b+ MRC1+ cells to the CSF1R+ macrophage pool gated on CD45+ in SP at day 27 is shown in graph (I). Data are from one representative experiment of two. Statistical analysis: *p < 0.05, Student's t test. See also Figure S4.

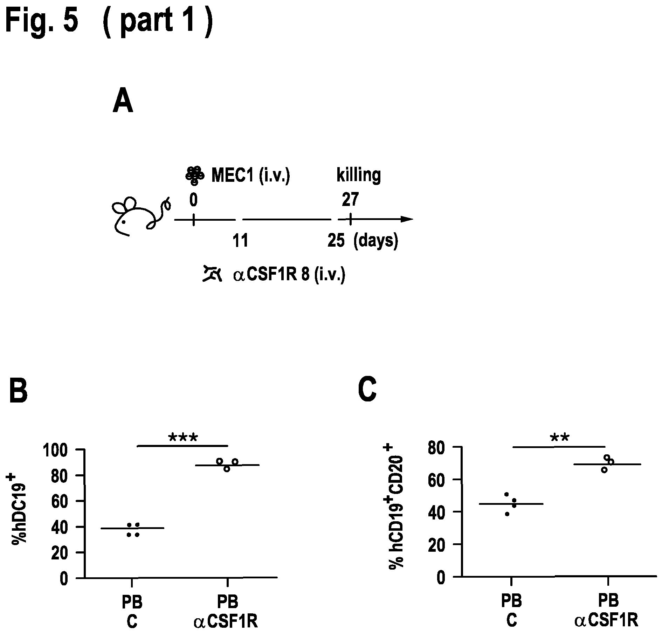

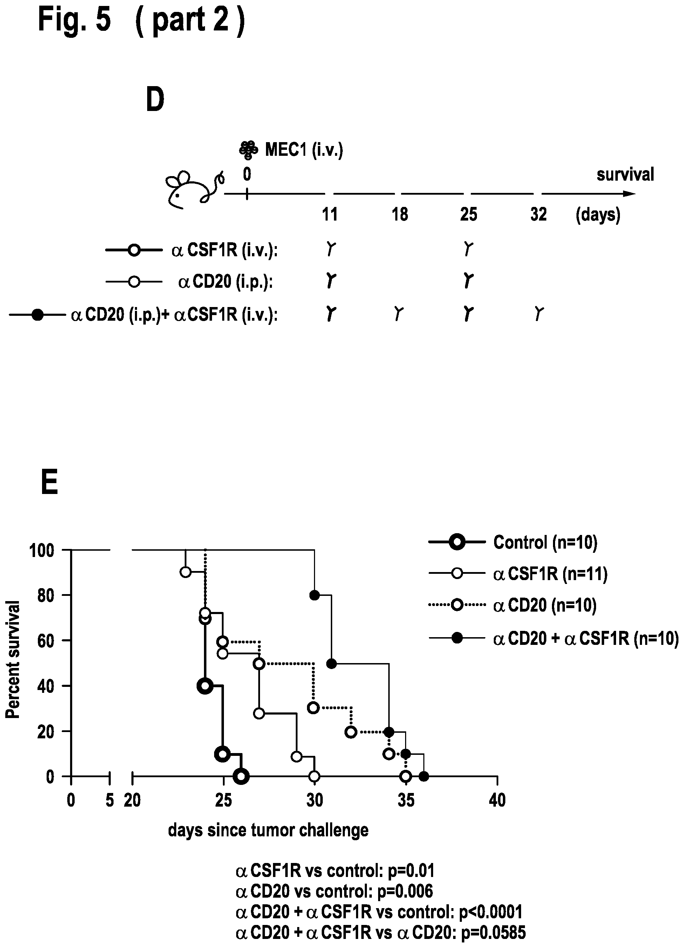

- **Figure** 5: Anti-leukemic effect and survival impact of anti-CSFIR moAb in the CLL xeno-transplantation system.
(A-B-C) i.v. MEC1 challenged (day 0) Rag2^{-/-}γ_{c}^{-/-} mice were treated i.v. (day +11, +25) with 30mg/Kg of anti-CSFIR moAb (n=3, white circles) or left untreated (n=4, black circles) and killed at day 27 (A). The mean value of the percentage of hCD19+ (B)
and of hCD19+ CD20+ (C) cells in PB is shown in graphs. Data are from one representative experiment of two. Statistically significant differences were calculated using the Student t test. **p < 0.01, ***p < 0.001. (D-E) i.v. MEC1 challenged (day 0) Rag2^{-/-} γ_{c}^{-/-}mice were left untreated (n=10, black circles) or treated with: 30mg/Kg i.v. of anti-CSFIR moAb (n=11, white circles; day + 11, +25); or 30mg/Kg i.p. of anti-CD20 moAb GA101 (n=10, blue circles; day +11, +25); or 30mg/Kg i.p. of anti-CD20 moAb GA101 (day +11, +25) + 30mg/Kg i.v. of anti-CSFIR moAb (day +18, +32) (n=10, violet circles) and monitored for survival (D). Kaplan-Meier survival curve is represented (E), statistical analysis was performed using Log-Rank test. αCSF1R vs control: p=0.01; αCD20 vs control: p=0.006; αCD20 + αCSF1R vs control: p<0.0001; αCD20 + αCSF1R vs αCD20: p=0.0585.

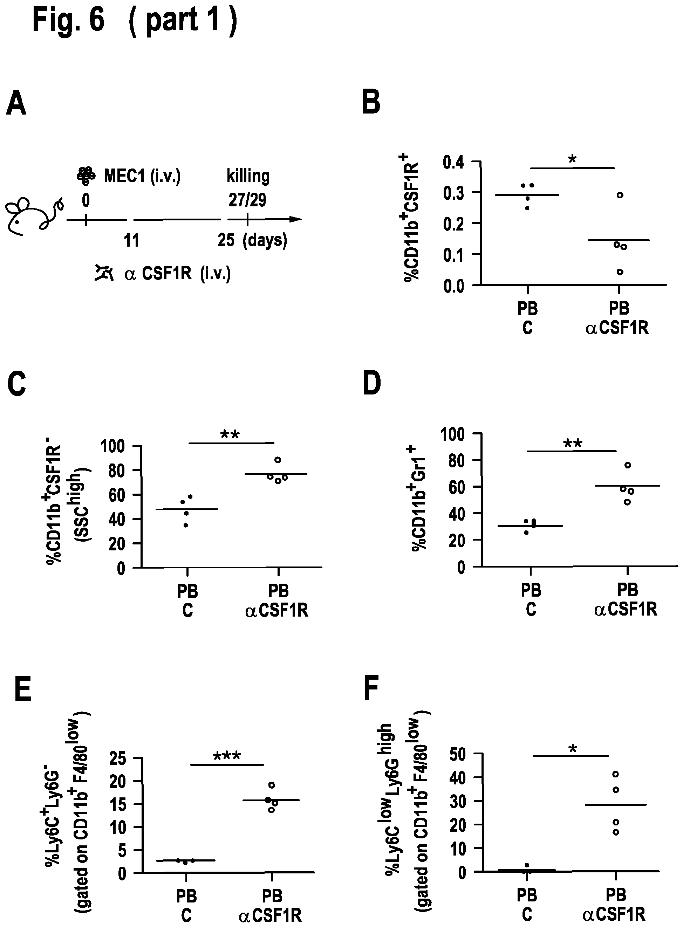

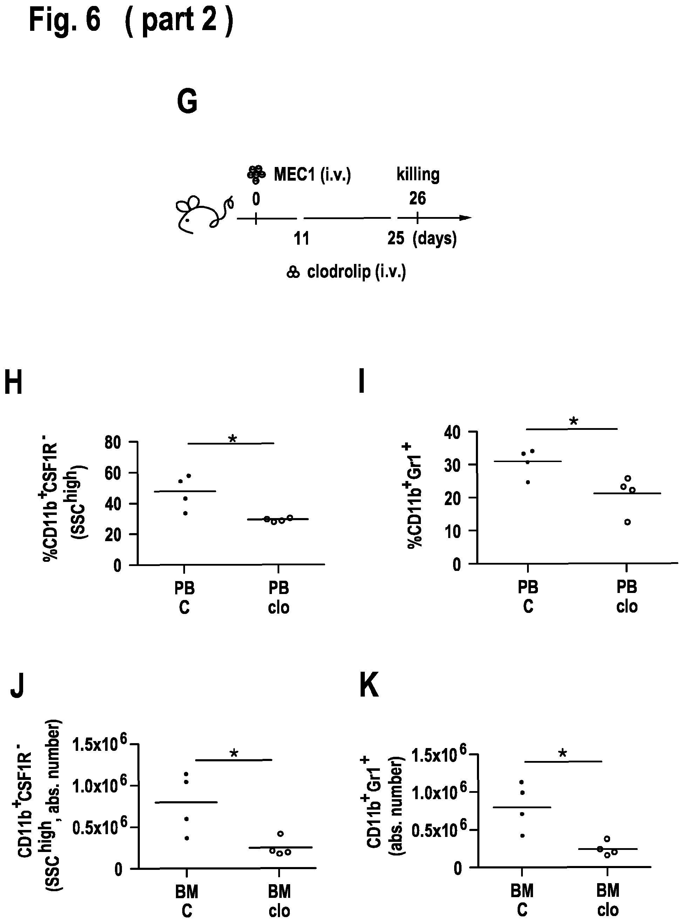

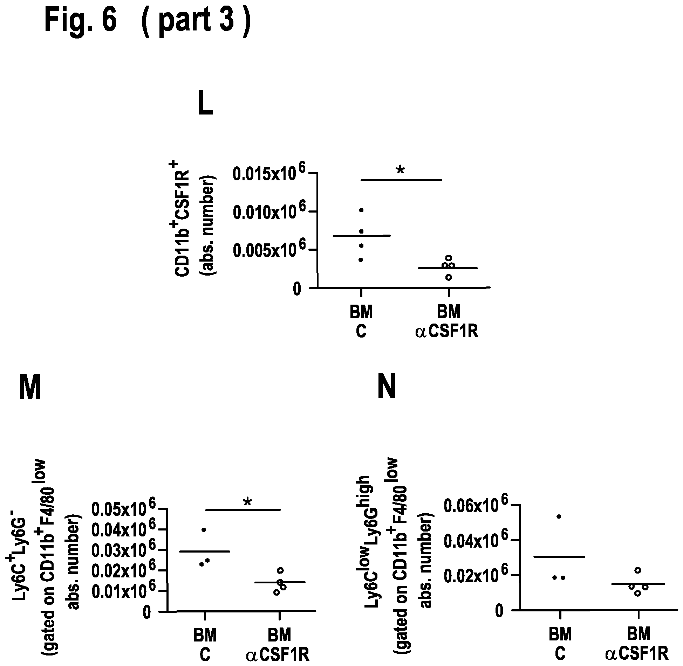

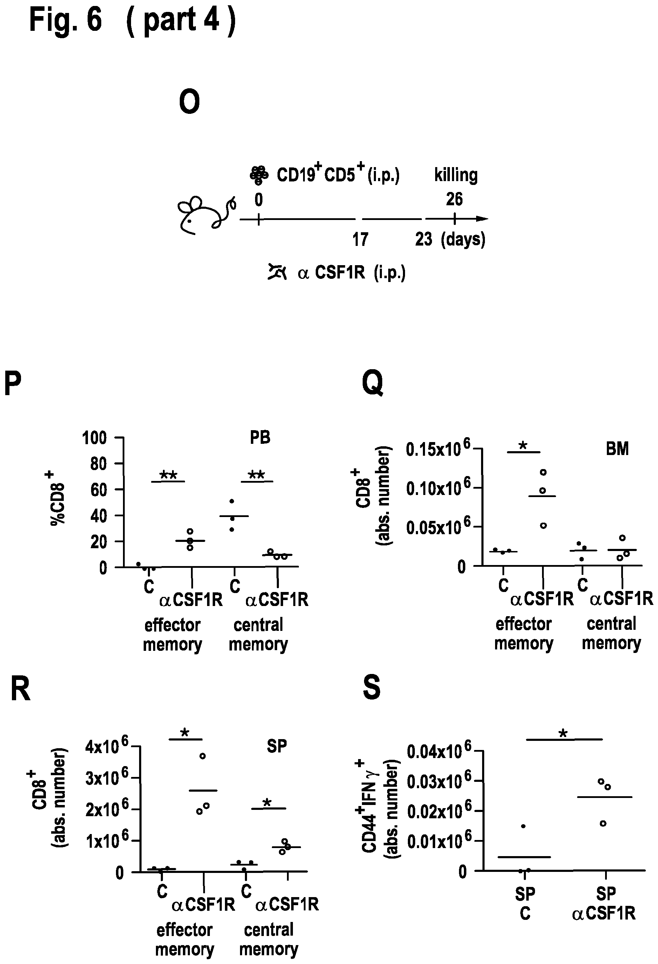

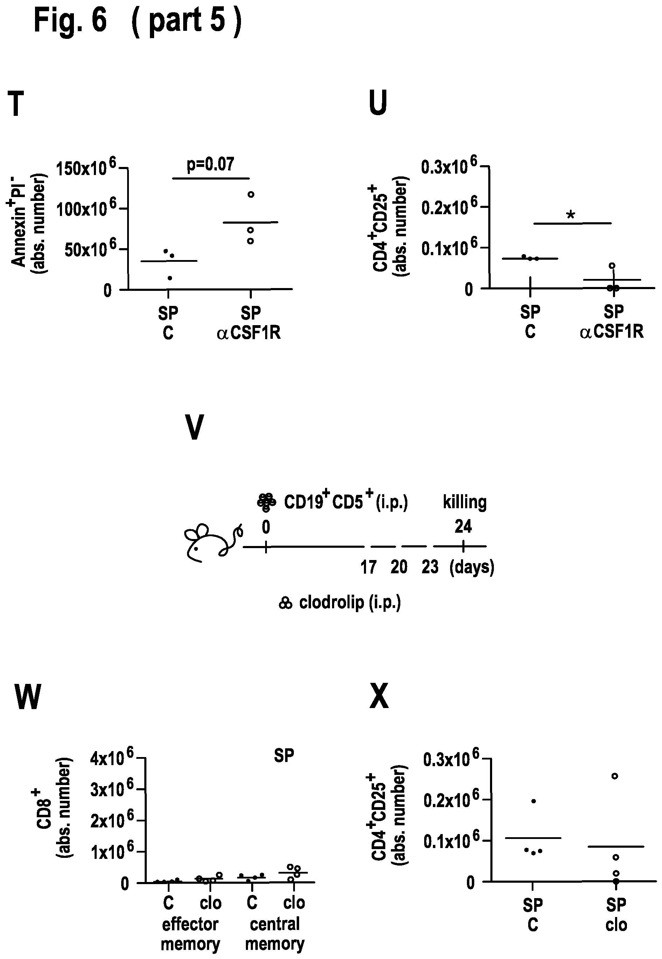

- **Figure** 6: The impact of macrophage targeting on the microenvironment of CLL mouse models.
(A-B-C-D-E-F) i.v. MEC1 challenged (day 0) Rag2^{-/-}γ_{c}^{-/-} mice were treated i.v. (day +11, +25) with 30mg/Kg of anti-CSFIR moAb (n=4, white circles) or left untreated (n=4, black circles) and killed at day 27 or 29 (A). The mean value of the relative contribution of CD11b+ CSF1R+ cells gated on CD45+ in PB is shown in graph (B). The mean value of the relative contribution of CD11b+ CSF1R- SSChigh neutrophils gated on CD45+ in PB is shown in graph (C). The mean value of the relative contribution of CD11b+ Gr1+ cells gated on CD45+ in PB is shown in graph (D). Data are from one representative experiment of two. The mean value of the relative contribution of monocytic Ly6C+ Ly6Ggated on CD11b+ F4/80low (E) and of granulocytic Ly6Clow Ly6Ghigh gated on CD11b+ F4/80low (F) cells in the PB is shown in graphs. Statistical analysis: *p < 0.05, **p < 0.01, ***p < 0.001, Student's t test. (G-H-I-J-K) i.v. MEC1 challenged (day 0) Rag2^{-/-}γ_{c}^{-/-} mice were treated i.v. (day +11, +25) with 60µl clodrolip (n=4, white circles) or left untreated (n=4, black circles) and killed at day 26 (G). The mean value of the relative contribution of CD11b+ CSF1R- SSChigh neutrophils gated on CD45+ in PB is shown in graph (H). The mean value of the relative contribution of CD11b+ Gr1+ cells gated on CD45+ in PB is shown in graph (I). The mean value of the absolute number of CD11b+ CSF1R- SSChigh neutrophils gated on CD45+ in BM is shown in graph (J). The mean value of the absolute number of CD11b+ Gr1+ cells gated on CD45+ in BM is shown in graph (K). Data are from one representative experiment of two. Statistical analysis: *p < 0.05, Student's t test. (L-M-N) i.v. MEC1 challenged (day 0) Rag2^{-/-}γ_{c}^{-/-} mice were treated i.v. (day +11, +25) with 30mg/Kg of anti-CSFIR moAb (n=4, white circles) or left untreated (n=4, black circles) and killed at day 27 or 29 (A). The mean value of the absolute number of CD11b+ CSF1R+ cells gated on CD45+ in BM is shown in graph (L). Data are from one representative experiment of two. The mean value of the absolute number of monocytic Ly6C+ Ly6G-gated on CD11b+ F4/80low (M) and of granulocytic Ly6Clow Ly6Ghigh gated on CD11b+ F4/80low (N) cells in the BM is shown in graphs. Statistical analysis: *p < 0.05, Student's t test. (O-P-Q-R-S-T-U) C57BL/6 mice transplanted i.p. with leukemic B cells from Eµ-TCL1 transgenic mouse were treated i.p. (day + 17, +23) with 30mg/Kg of anti-CSFIR moAb (n=3, white circles) or left untreated (n=3, black circles) and killed at day 26 (O). The mean value of the relative contribution of CD44+CD62L+ central and CD44+CD62Llow/neg effector memory CD8+ T cells in PB is shown in graph (P). The mean value of the absolute number of CD44+CD62L+ central and CD44+CD62Llow/neg effector memory CD8+ T cells in BM (Q) and SP (R) is shown in graphs. Intracellular IFNγ production was measured by flow cytometry and the mean value of the absolute number of CD8+ CD44+ IFNγ+ T cells is shown in graph (S). The mean value of the absolute number of Ann+ PI- cells gated on CD19+ CD5+ cells to the whole B cell pool in SP is shown in graph (T). The mean value of the absolute number of CD4+ CD25+ T cells is shown in graph (U). Statistical analysis: *p < 0.05, **p < 0.01, ***p < 0.001, Student's t test. (V-W-X) C57BL/6 mice transplanted i.p. with leukemic B cells from Eµ-TCL1 transgenic mouse were treated i.p. (starting at day 17, every 3 days) with 50µl of clodrolip (n=4, white circles) or left untreated (n=4, black circles) and killed at day 24 (V). The mean value of the absolute number of CD44+CD62L+ central and CD44+CD62Llow/neg effector memory CD8+ T cells in SP is shown in graph (W). The mean value of the absolute number of CD4+ CD25+ T cells in SP is shown in graph (X).

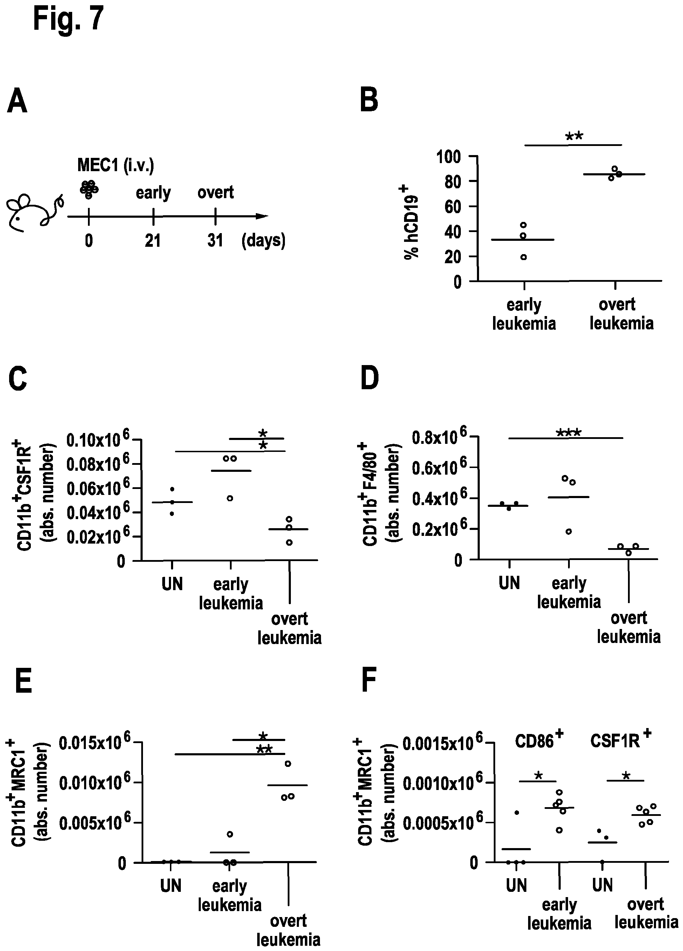

- **Figure** 7: BM microenvironment of xeno-transplanted mice: monocyte and macrophage populations
(A-B-C-D-E-F) Rag2-/-yc-/- mice, uninjected (UN, black circles) or injected i.v. with MEC1 (white circles) cells (day 0), were killed at early (n=3) and late (n=3) stage of leukemia and analyzed by flow cytometry (A). The mean value of the relative contribution of hCD19+ cells in BM is shown in graph (B). The mean value of the absolute number of CD11b+ CSF1R+ cells gated on CD45+ in BM is shown in graph (C). The mean value of the absolute number of CD11b+ F4/80+ cells gated on CD45+ in BM is shown in graph (D). The mean value of the absolute number of CD11b+ MRC1+ cells to the whole macrophage pool (CD11b+ F4/80+) gated on CD45+ in BM is shown in graph (E).Data are from one representative experiment of three. Statistical analysis: *p < 0.05, **p < 0.01, ***p < 0.001, Student's t test. The mean value of the absolute number of CD86+ MRC1+ and CSF1R+ MRC1+ cells to the whole CD45+ CD11b+ pool in BM is shown in graph (F). Statistical analysis: *p < 0.05, Student's t test.

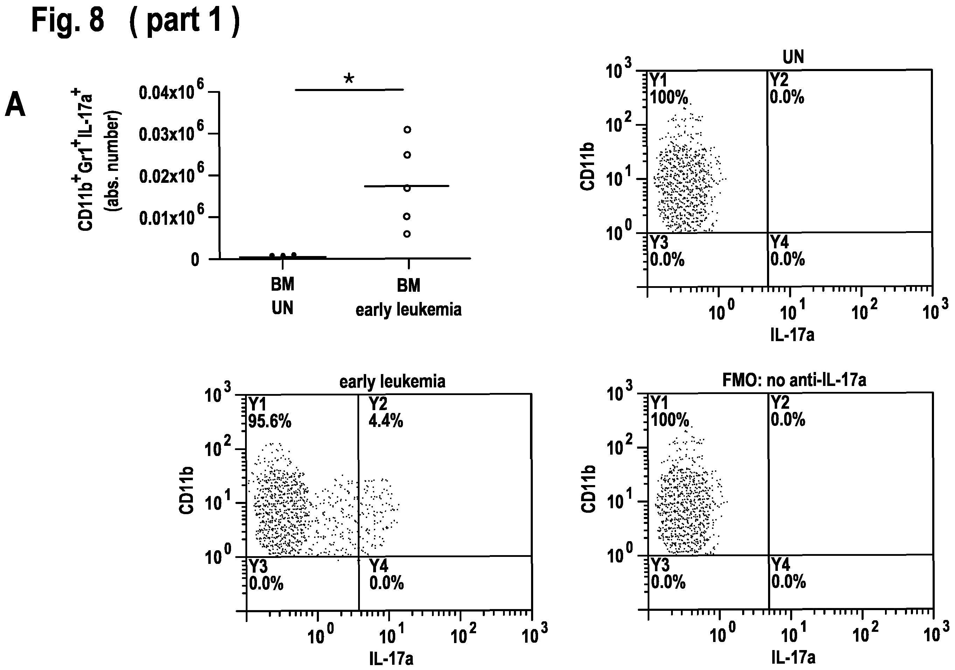

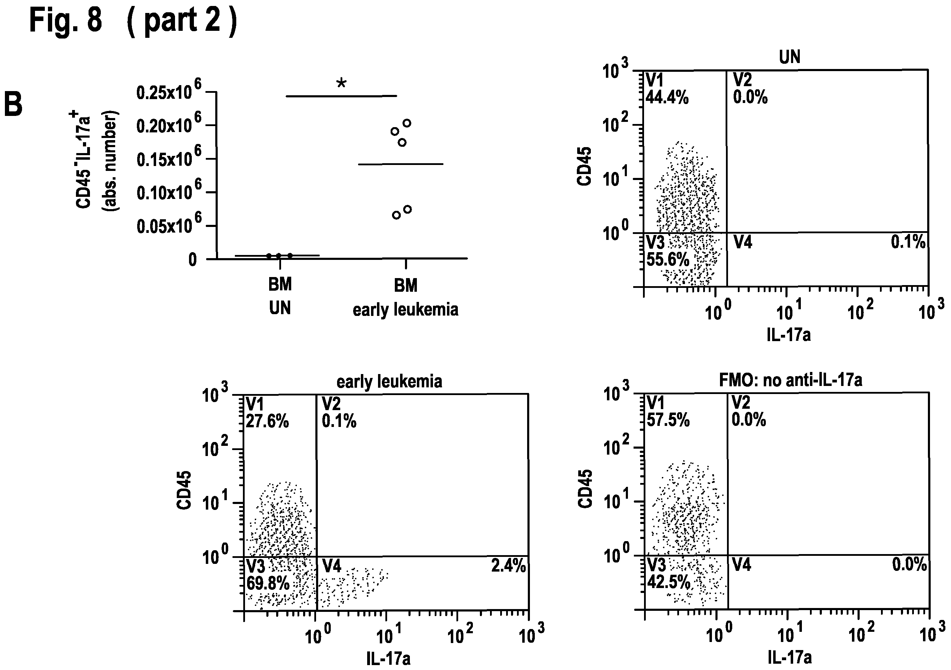

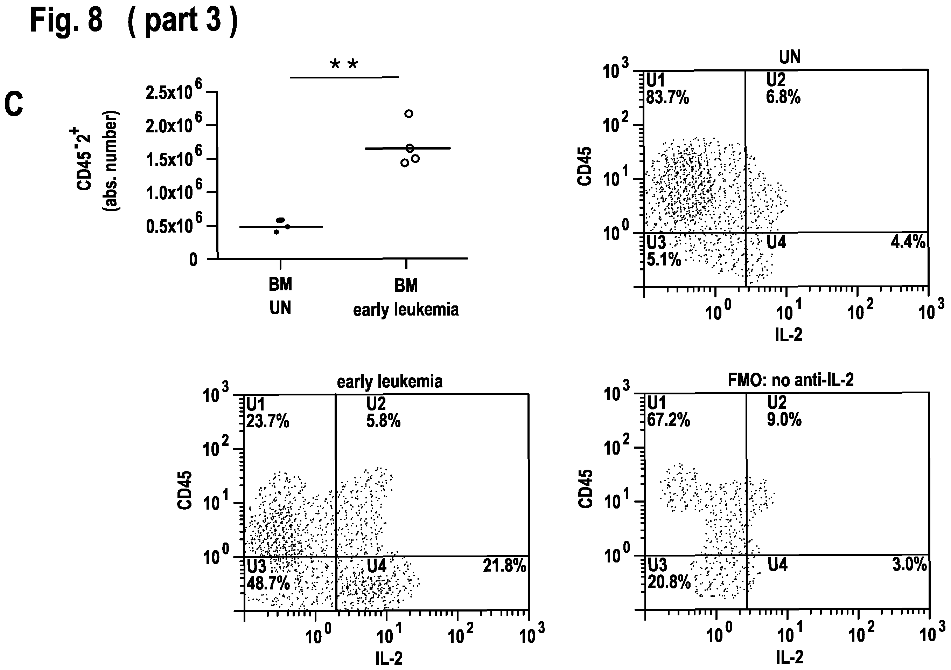

- **Figure** 8: Cytokine production by BM stromal cells from CLL xeno-transplanted mice.
(A-B-C) Rag2-/-yc-/- mice, uninjected (UN, black circles, n=3) or injected i.v. with MEC1 (white circles) cells (day 0), were killed at early stage of leukemia (n=5) and analyzed by flow cytometry. Intracellular cytokine production was measured by flow cytometry. Left: the mean value of the absolute number of CD11b+ Gr1+ cells producing IL-17a gated on CD45+ in BM is shown in graph; Right: flow cytometry representative plots of CD11b+ Gr1+ cells producing IL- 17a gated on CD45+ in BM (A). Left: the mean value of the absolute number of non-hematopoietic CD45- cells producing IL-17a; Right: flow cytometry representative plots of non-hematopoietic CD45- cells producing IL-17a in BM (B). Left: the mean value of the absolute number of non-hematopoietic CD45- cells producing IL-2; Right: flow cytometry representative plots of nonhematopoietic CD45- cells producing IL-2 in BM (C). Statistical analysis: *p < 0.05, **p < 0.01, Student's t test.

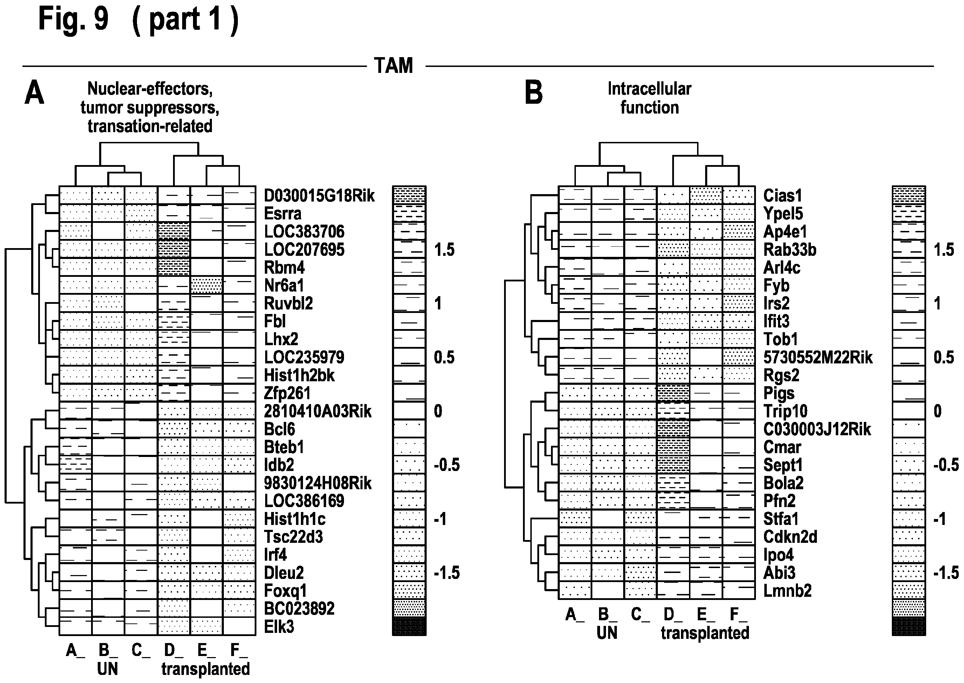

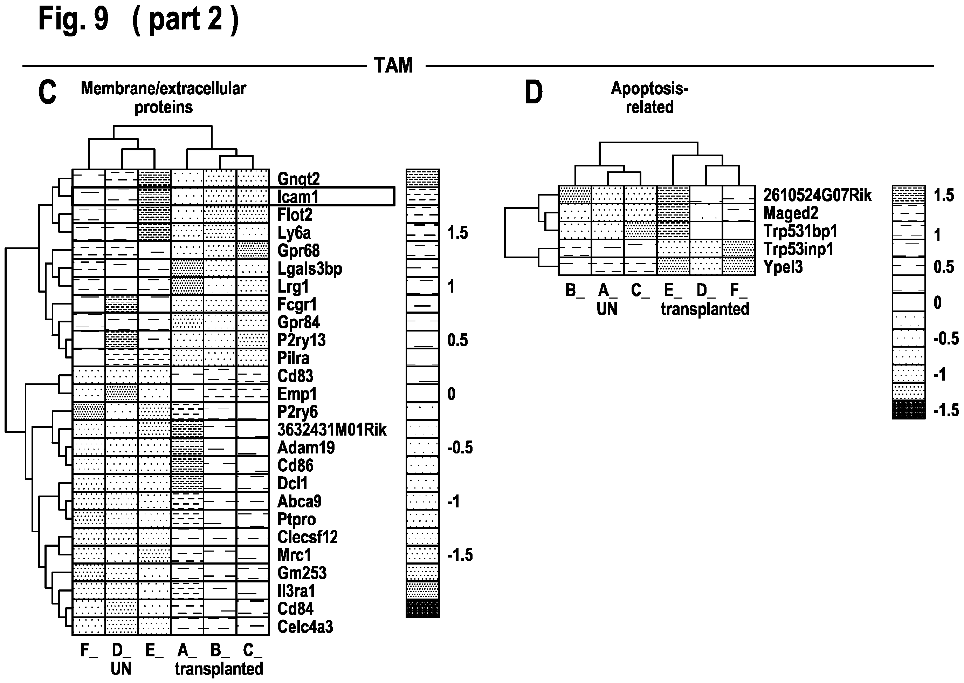

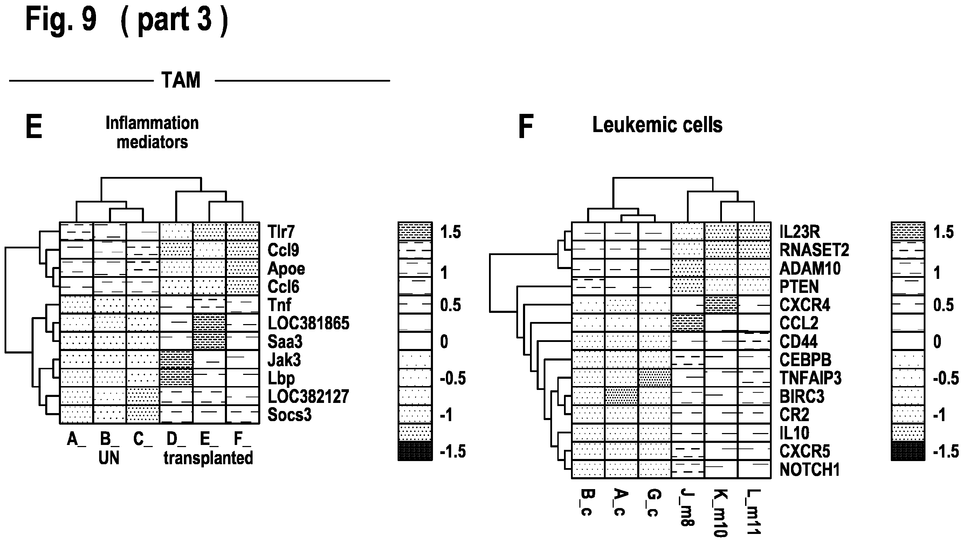

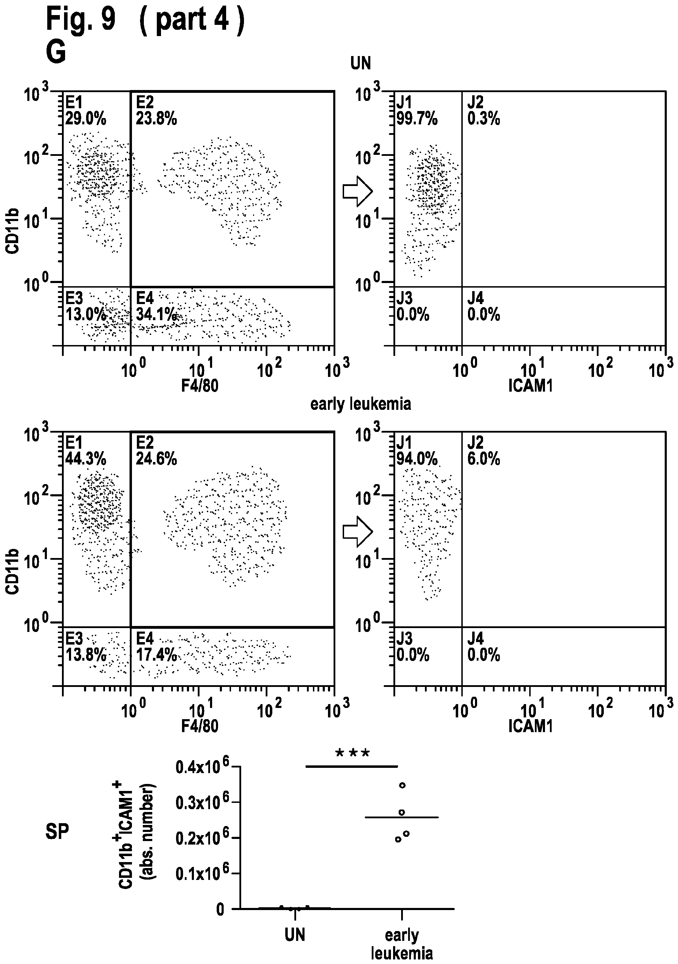

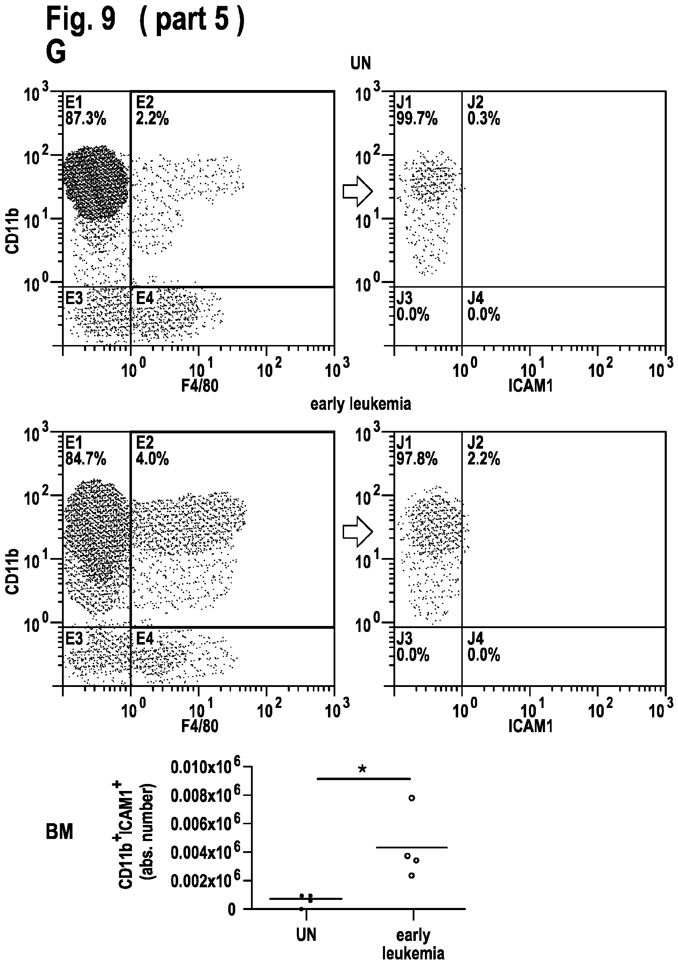

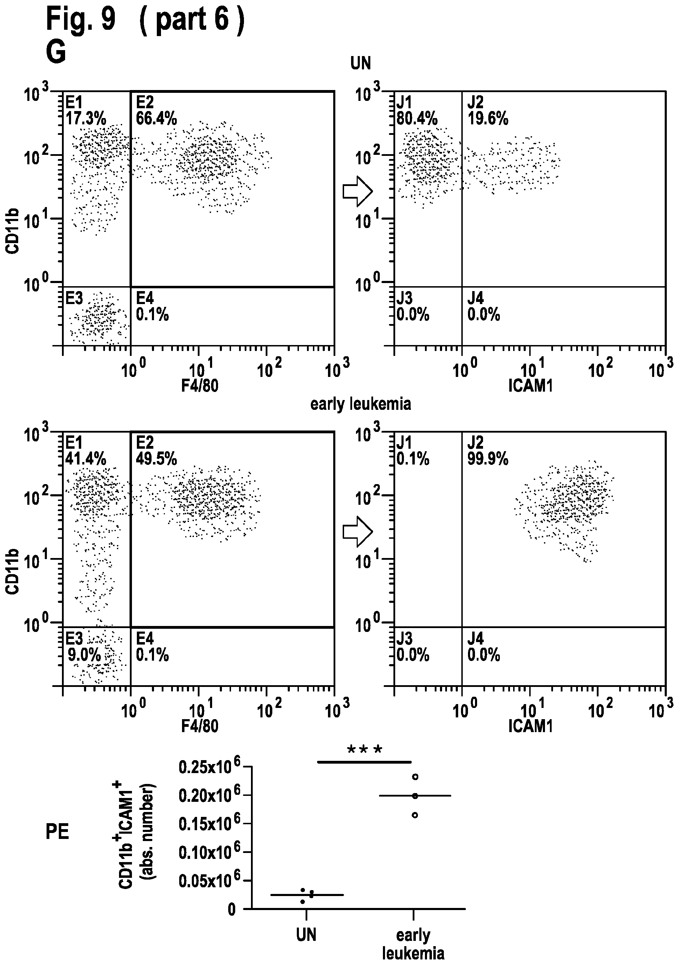

- **Figure** 9: Transcriptome analysis of MEC1 leukemic cells and monocytes/macrophages of xeno-transplanted mice
(A-B-C-D-E-F) Rag2-/-yc-/- mice were injected i.v. with MEC1 cells and sacrificed at early stage of leukemia with age-matched untransplanted Rag2-/-yc-/- mice (n=3/group). RNA was isolated from BM murine monocytes/macrophages purified by magnetic separation and processed for Illumina whole-genome gene expression direct-hybridization assay. Supervised analysis between monocytes/macrophages from xeno-transplanted and untransplanted (UN) mice was performed. The heatmaps represent the hierarchical clustering of differentially expressed genes (transplanted vs UN) (adjusted P-Value < 0.05). The expression level of each gene has been standardized by subtracting the gene's mean expression and then dividing by the standard deviation across all samples. This scaled expression value, denoted as the Row Z-score, is plotted in red-blue scale color, with red indicating high expression. The gene expression differences among the groups were functionally classified as follows: nuclear-effectors, tumor suppressors and translation-related (A); intracellular function (B); membrane/extracellular proteins (C), apoptosis-related (D) and inflammation mediators (E). RNA was isolated from BMinfiltrating human CD19+ MEC1 cells purified by magnetic separation and processed for Illumina whole-genome gene expression direct-hybridization assay. Supervised analysis between xeno-transplanted and in vitro pre-injected MEC1 cells was performed. The heatmap represents the hierarchical clustering of differentially expressed genes (xenotransplanted vs pre-injected) (adjusted P-Value < 0.05). The gene expression differences among the groups involved in monocytes/macrophages and B cells interaction are described (F). (G) Rag2-/-yc-/- mice injected i.v. with MEC1 cells and sacrificed at day 21 (n=4, white circles) and age-matched uninjected (UN) Rag2-/-yc-/- mice (n=4, black circles) were analyzed by flow cytometry. Left: Flow cytometry representative plots of CD11b+ ICAM1+ cells to the whole macrophage pool (CD11b+ F4/80+) gated on CD45+, in the SP, BM and PE. Right: the mean value of the absolute number of CD11b+ ICAM1+ cells to the whole macrophage pool (CD11b+ F4/80+) gated on CD45+, in SP, BM, PE is shown in graphs. Statistical significance was analyzed by Student's t test. *p < 0.05, **p < 0.01, ***p < 0.001.

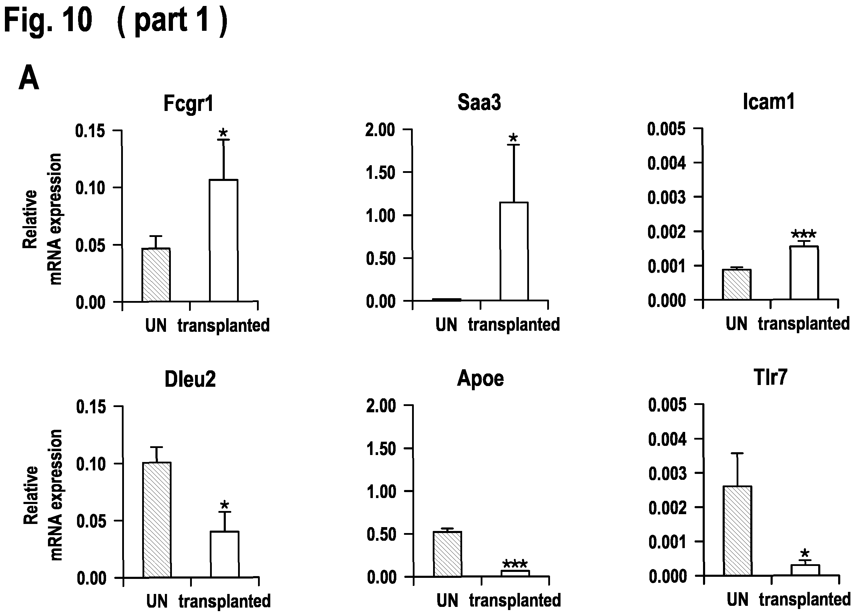

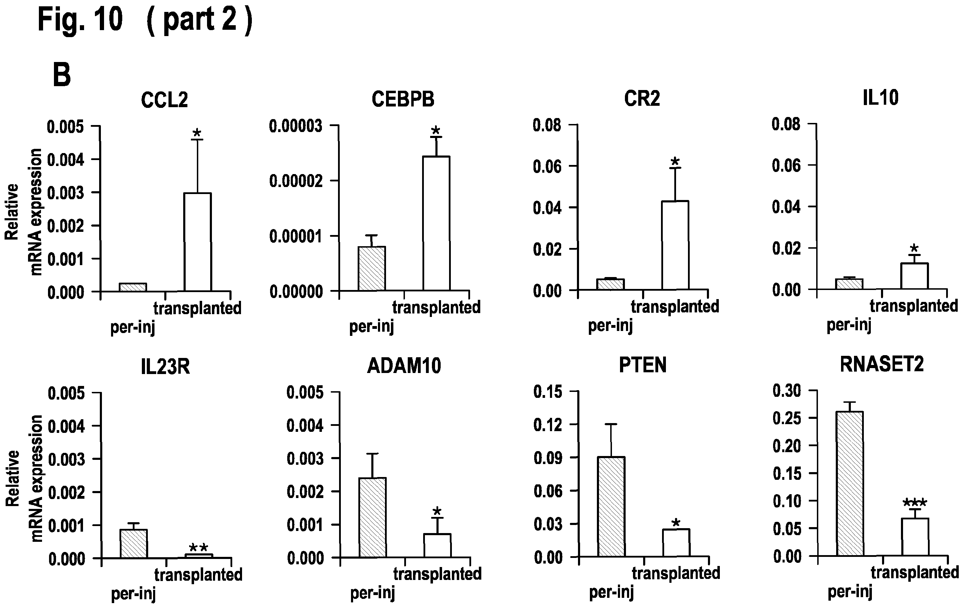

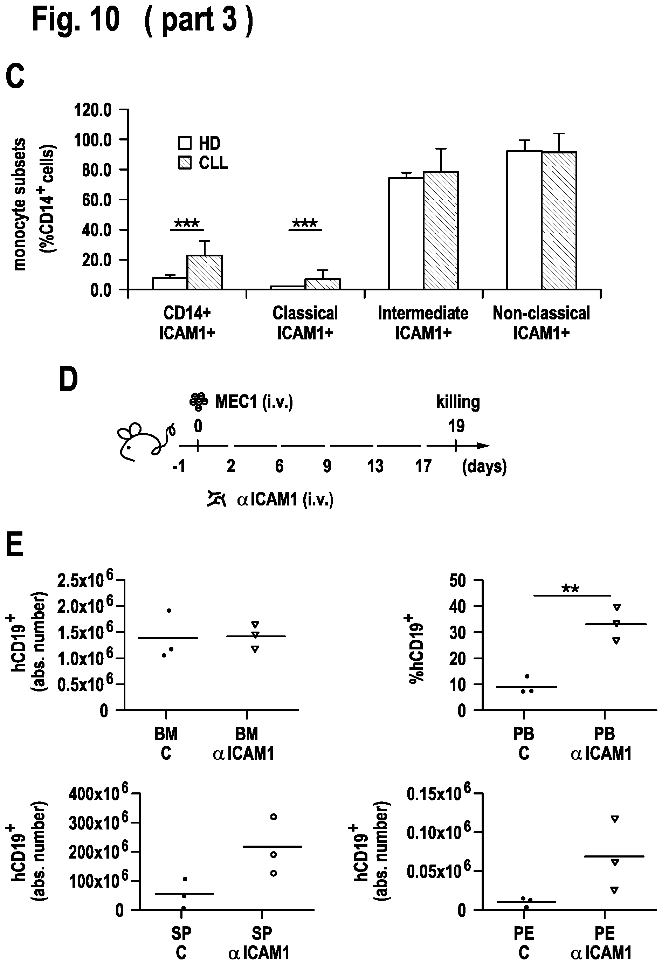

- **Figure 10**: Transcriptome analysis suggests a role for the monocyte/macrophage cross talk with leukemic cells (related to Figure 9).
(A) Relative mRNA expression of Fcgr1, Saa3, Icam1, Dleu2, Apoe, Tlr7 in monocytes/macrophages from BM of uninjected (UN) and Rag2-/-yc-/- mice injected i.v. with MEC1 cells and sacrificed at early stage of leukemia. The expression was normalized to β-actin levels. Data are shown as mean ± SD (n=3/group). (B) Relative mRNA expression of CCL2, CEBPB, CR2, IL10, IL23R, ADAM10, PTEN, RNASET2 in MEC1 B cells from BM of xeno-transplanted Rag2-/-yc-/- mice sacrificed at early stage of leukemia (n=3/group) and in vitro pre-injection cells. The expression was normalized to β-actin levels. Data are shown as mean ± SD (n=3/group). (C) Fresh peripheral blood samples were collected from 18 CLL patients and 4 healthy donors. Monocytes were analyzed by flow cytometry for the expression of ICAM1. CD14+ monocytes were subdivided in CD14++ CD16- classical monocytes, CD14++ CD16+ intermediate monocytes and CD14+ CD16++ non-classical monocytes. The mean value ± SD of the relative contribution of ICAM1 + cells to the monocyte populations gated on CD14 is shown in graph. (D-E) i.v. MEC1 challenged (day 0) Rag2-/-yc-/- mice were either pre-treated starting from day -1 every 3/4 days with 2mg/Kg i.v. of ICAM1 blocking moAb clone YN1/1.7.4 (n=3, white triangles) or left untreated (n=3, black circles) and were analyzed by flow cytometry (D). The mean value of the percentage of human CD19+ cells in the PB and of the absolute number of human CD19+ cells in the BM, SP and PE is shown in graph (E). Statistical analysis: *p < 0.05, **p < 0.01, ***p < 0.001, Student's t test.

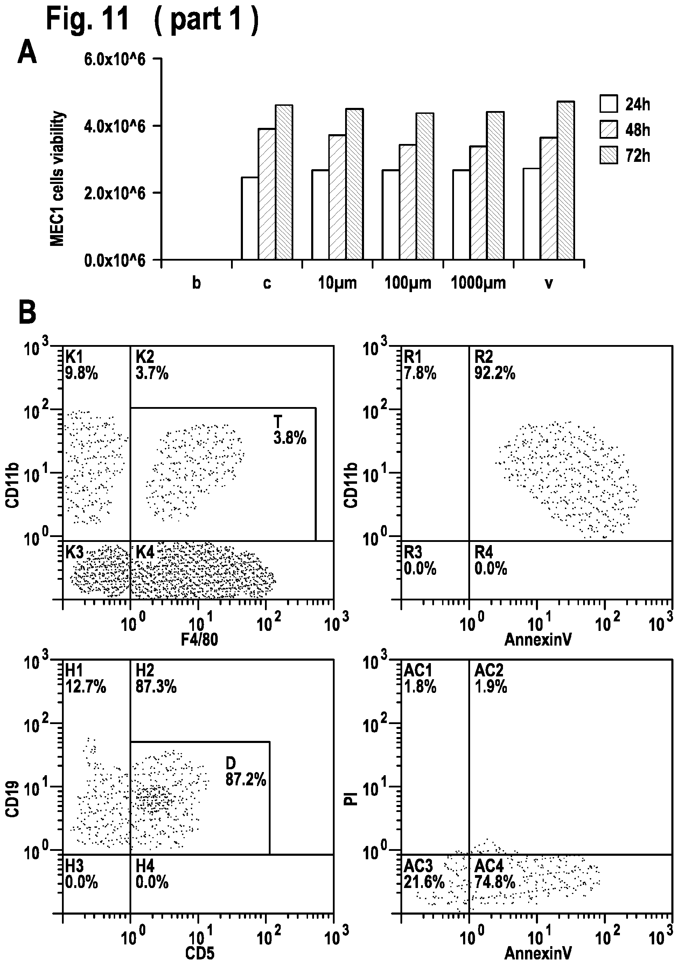

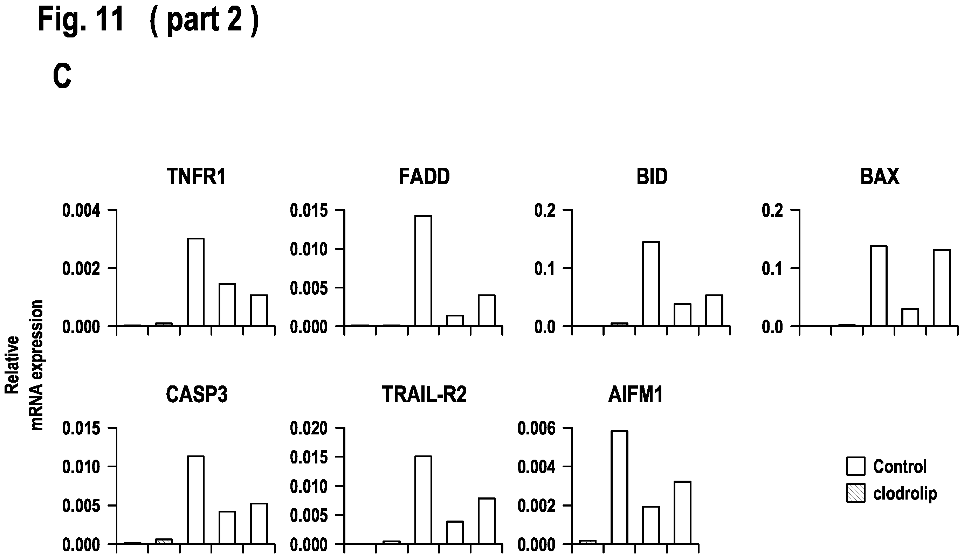

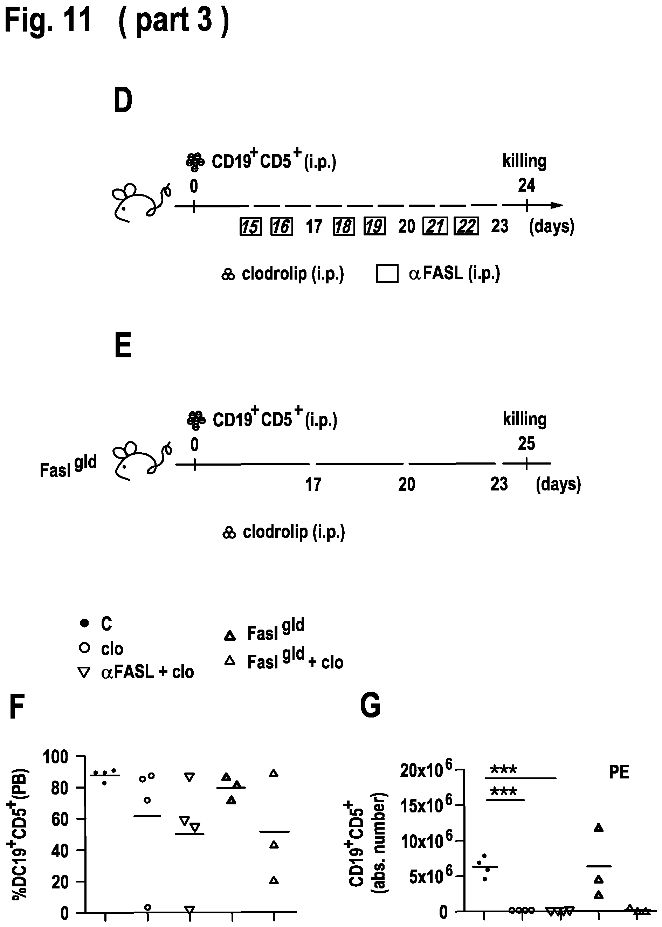

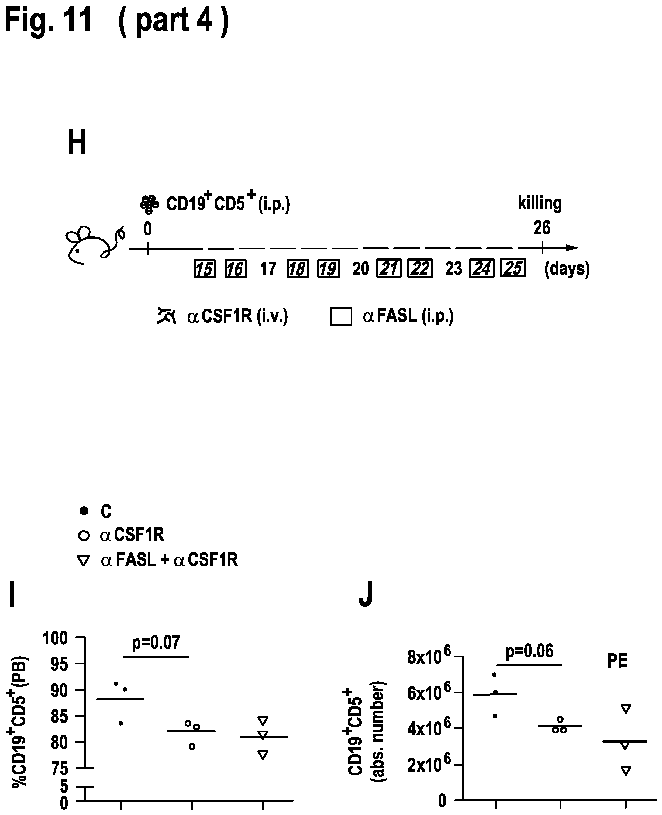

- **Figure 11**: Cell death mechanisms induced on leukemic cells by macrophage targeting
(A) Left: MEC1 cells were plated in 96-well plates alone (c), with increasing concentrations of clodrolip (10 µM, 100 µM, 1000 µM) and with PBS liposomes (v, 1000 µM) and a luminescent assay was performed 24, 48 and 72 hours later to evaluate MEC1 cells' sensitivity to the drug. Right: MEC1 cells were plated in 96-well plates alone (c), with anti-human CSF1R moAb RG7155 (hMab 2F11-e7 IgG1 isotype) (1-10µg/ml) and a luminescent assay was performed 48 and 72 hours later to evaluate MEC1 cells' sensitivity to the drug.

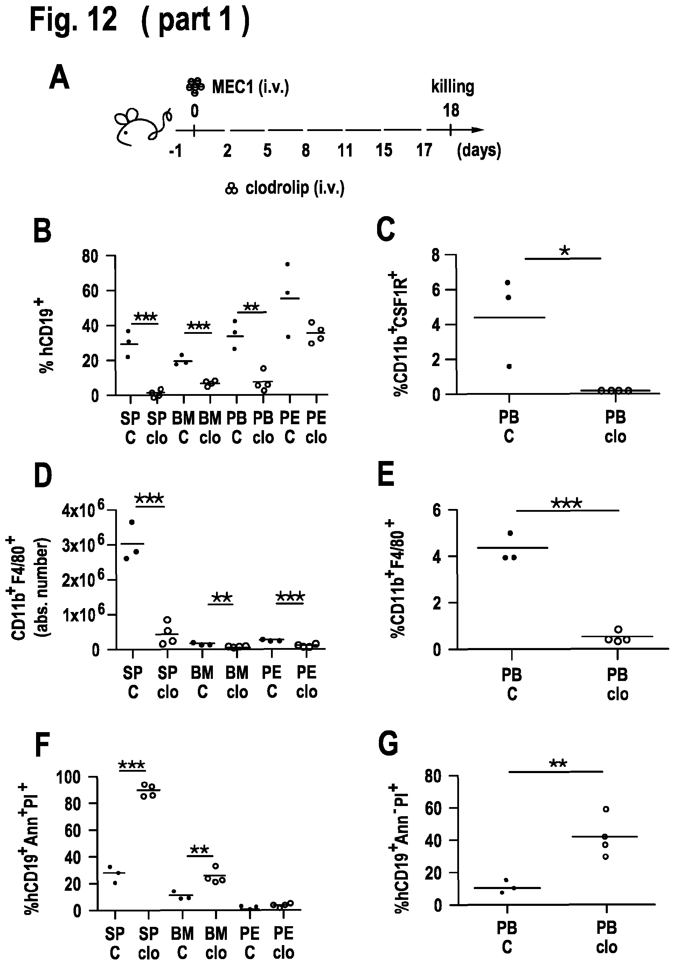

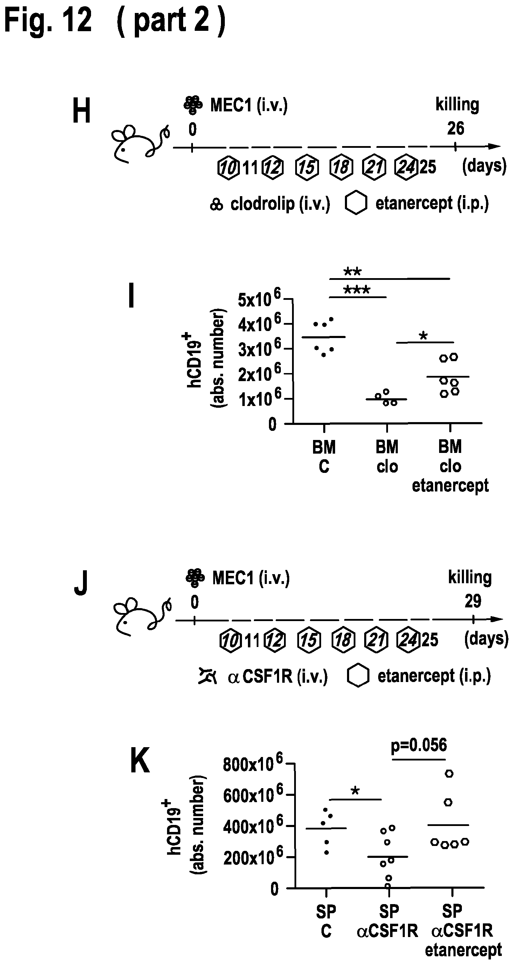

- **Figure 12**: TNF-dependent macrophage-mediated mechanism of in vivo leukemic cell death
(A-B-C-D-E-F-G) i.v. MEC1 challenged (day 0) Rag2-/-yc-/- mice were either pre-treated starting from day -1 every 3 days with 200µl i.v. of clodrolip (n=4, white circles) or untreated (n=3, black circles) and were analyzed by flow cytometry (A). The mean value of the percentage of human CD19+ cells in SP, BM, PB, PE is shown in graph (B). The mean value of the relative contribution of CD11b+ CSF1R+ cells gated on CD45 in PB is shown in graph (C). The mean value of the absolute number or of the percentage of CD11b+ F4/80+ cells gated on CD45+ in SP, BM, PE (D) and PB (E) is shown in graphs. The mean value of the relative contribution of Ann+ PI+ cells gated on hCD19+ cells in SP, BM, PE (F) and of the relative contribution of Ann- PI+ cells gated on hCD19+ cells in PB (G) is shown in graphs. Statistical significance was analyzed by Student's t test. *p < 0.05, **p < 0.01, ***p < 0.001.
(H-I) i.v. MEC1 challenged (day 0) Rag2-/-yc-/- mice were left untreated (n=6, black circles) or treated i.v. (day +11, +25) with 60µl clodrolip (n=4, white circles) or with 60µl clodrolip (day +11, +25) + 10mg/Kg etanercept (i.p., day +10,+12,+15,+18,+21,+24) (n=6, red triangles) and killed at day 26 (H). The mean value of the absolute number of human CD19+ cells in the BM is shown in graph (I).Statistical significance was analyzed by Student's t test. *p < 0.05, **p < 0.01, ***p < 0.001.
(J-K) i.v. MEC1 challenged (day 0) Rag2-/-yc-/- mice were left untreated (n=5, black circles) or treated i.v. (day +11, +25) with 30mg/Kg anti-CSFIR moAb (n=7, white circles) or with 30mg/Kg anti-CSF1R moAb (day + 11, +25) + 11mg/Kg etanercept (i.p., day +10,+12,+15,+18,+21,+24) (n=6, red triangles) and killed at day 29 (J). The mean value of the absolute number of human CD19+ cells in the SP is shown in graph (K). Statistical significance was analyzed by Student's t test. *p < 0.05.

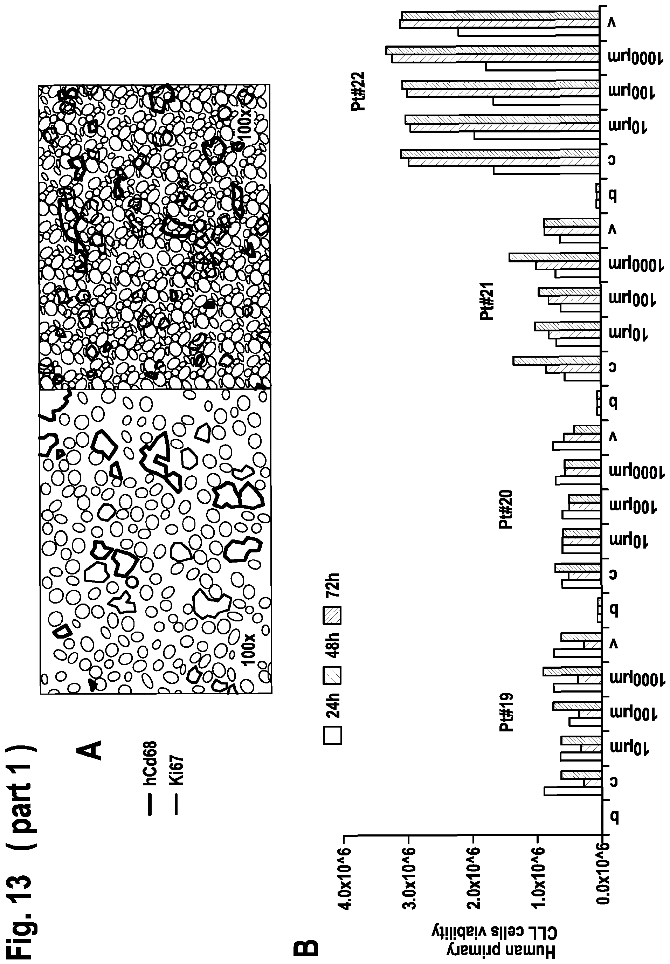

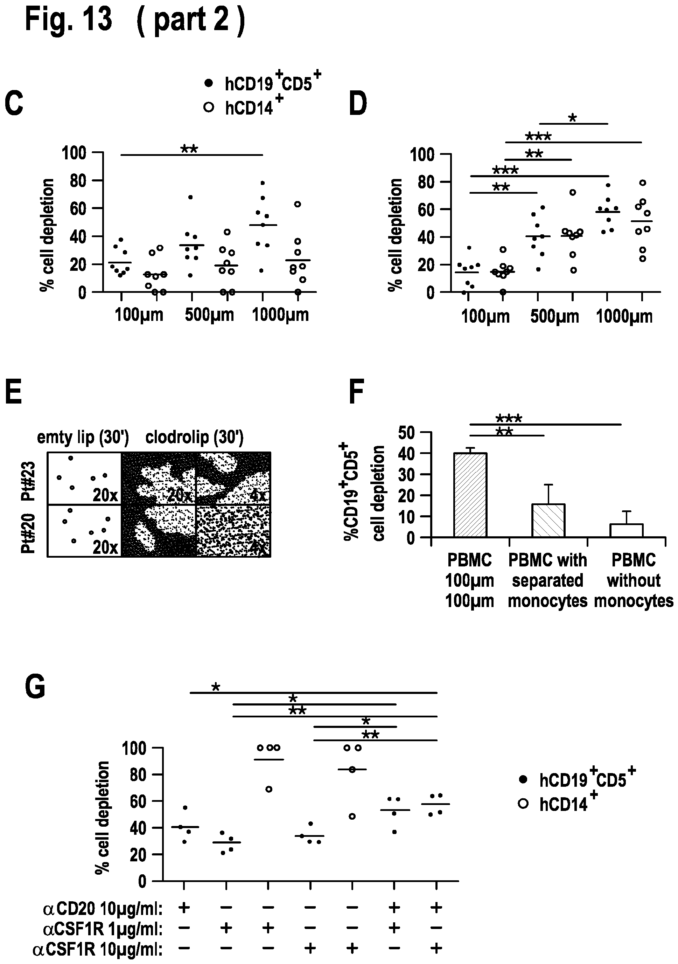

- **Figure 13**: Monocytes/macrophages and leukemic cells in humans
(G) hCD19+ CD5+ (black circles) and hCD14+ (white circles) depletion after 48h (n=4) with anti-CD20 moAb GA101 10µg/ml, anti-human CSF1R moAb RG7155 (hMab 2F11-e7 IgG1 isotype) (1-10µg/ml), anti-CD20 moAb GA101 10µg/ml + anti-human CSFIRmoAb RG7155 (hMab 2F11-e7 IgG1 isotype) (1-10p,g/ml) was calculated by the following formula: 100 - % remaining cells, where % remaining cells = (Absolute number in treated samples/Absolute number in untreated samples) × 100. Horizontal bars represent the mean value (*p<0.05; **p<0.01), Student's t test.

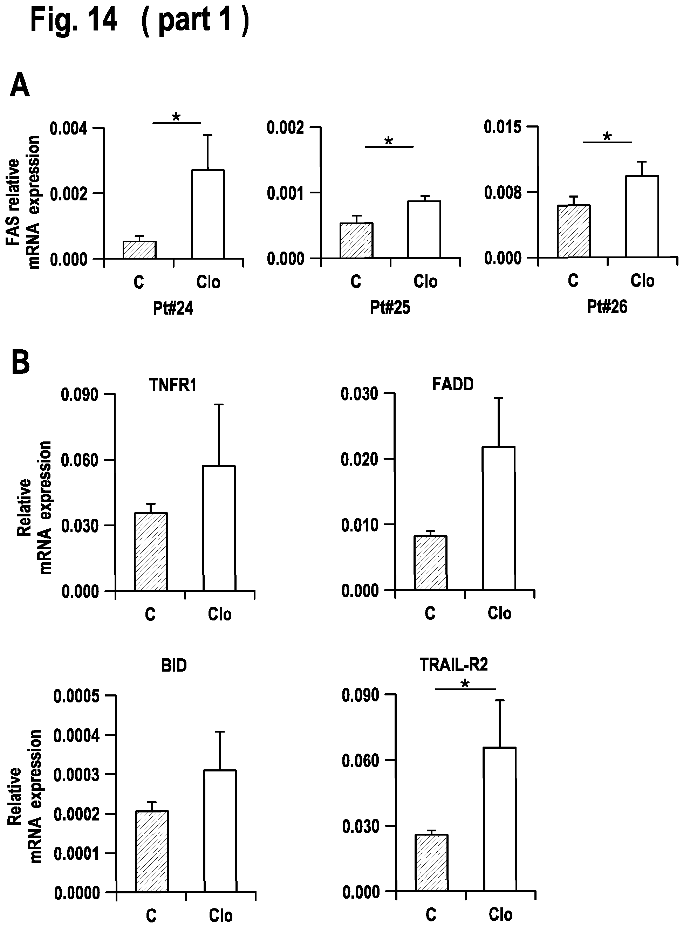

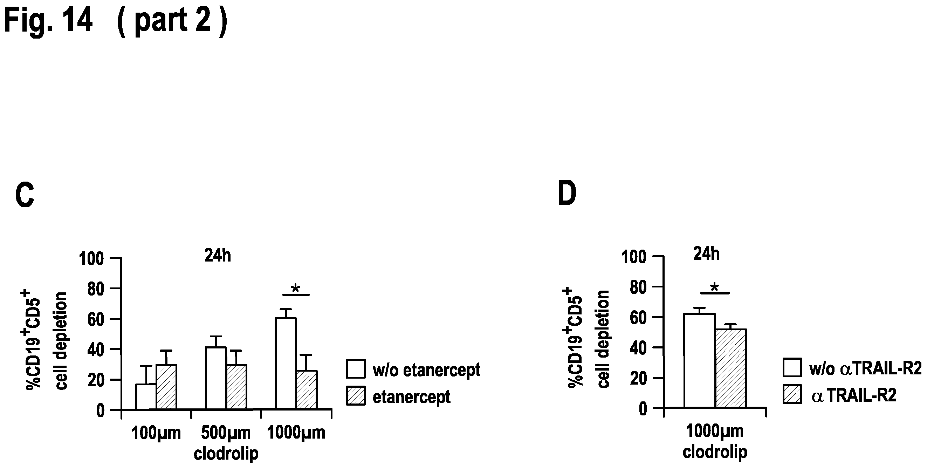

- **Figure 14**: Cell death mechanisms induced by clodrolip on primary leukemic cells from CLL patients (related to Figure 13).
(A) Relative mRNA expression of FAS in human primary CLL cells purified by negative selection from PBMCs of CLL patients (n=3, Pt #24-26), plated (in triplicates) 24h in 6-well plates alone (C, black bars) or with 1000 µM of clodrolip (Clo, white bars). The expression was normalized to β-actin levels. Data are shown as mean ± SD (n=3/group). Statistically significant differences were calculated using the Student t test. *p < 0.05.
(B) Relative mRNA expression of TNFR1, FADD, BID, TRAIL-R2 in human primary CLL cells purified by negative selection from PBMC of one CLL patient, plated (in triplicates) 24h in 6-well plates alone (C, black bars) or with 1000 µM of clodrolip (Clo, white bars). The expression was normalized to β- actin levels. Data are shown as mean ± SD (n=3/group). Statistically significant differences were calculated using the Student t test. *p < 0.05
(C) hCD19+ CD5+ depletion after 24h treatment (n=3) with 100µM, 500µM, 1000µM of clodrolip, with (black bar) or without (white bar) etanercept (10µg/ml) was calculated by the following formula: 100 - % remaining cells, where % remaining cells = (Absolute number in treated samples/Absolute number in untreated samples) × 100. (*p<0.05), Student's t test.
(D) hCD19+ CD5+ depletion after 24h treatment (n=3) with clodrolip 1000µM, with (black bar) or without (white bar) blocking anti-TRAIL-R2 moAb (1µg/ml) was calculated by the following formula: 100 - % remaining cells, where % remaining cells = (Absolute number in treated samples/Absolute number in untreated samples) × 100. (*p<0.05), Student's t test.

### Detailed Description of the Invention

The invention is defined in the appended claims and any other aspects set forth herein not falling within the scope of the claims are for information only.

Many tumors are characterized by a prominent immune cell infiltrate, including macrophages. Initially, the immune cells were thought to be part of a defense mechanism against the tumor, but recent data support the notion that several immune cell populations including macrophages may, in fact, promote tumor progression. Macrophages are characterized by their plasticity. Depending on the cytokine microenvironment, macrophages can exhibit so-called M1 or M2-subtypes. M2 macrophages are engaged in the suppression of tumor immunity. They also play an important role in tissue repair functions such as angiogenesis and tissue remodeling which are coopted by the tumor to support growth. In contrast to tumor promoting M2 macrophages, M1 macrophages exhibit antitumor activity via the secretion of inflammatory cytokines and their engagement in antigen presentation and phagocytosis (Mantovani, A. et al., Curr. Opin. Immunol. 2 (2010) 231-237).

By secreting various cytokines such as colony stimulating factor 1 (CSF-1) and IL-10, tumor cells are able to recruit and shape macrophages into the M2- subtype, whereas cytokines such as granulocyte macrophage colony stimulating factor (GM-CSF), IFN-gamma program macrophages towards the M1 subtype. Using immunohistochemistry, it is possible to distinguish between a macrophage subpopulation co-expressing CD68 and CD163, which is likely to be enriched for M2 Macrophages, and a subset showing the CD68+/MHC II+, or CD68+/CD80+ immunophenotype, likely to include M1 macrophages. Cell shape, size, and spatial distribution of CD68 and CD163 positive macrophages is consistent with published hypotheses on a tumor-promoting role of M2 macrophages, for example by their preferential location in tumor intersecting stroma, and vital tumor areas. In contrast, CD68+/MHC class II+ macrophages are ubiquitously found. Their hypothetical role in phagocytosis is reflected by clusters of the CD68+/MHC class II+, but CD163-immunophenotype near apoptotic cells and necrotic tumor areas.

The subtype and marker expression of different macrophage subpopulations is linked with their functional state. M2 macrophages can support tumorigenesis by:
a) enhancing angiogenesis via the secretion of angiogenic factors such as VEGF or bFGF,
b) supporting metastasis formation via secretion of matrix metalloproteinases(MMPs), growth factors and migratory factors guiding the tumor cells to the blood stream and setting up the metastatic niche (Wyckoff, J. et al., Cancer Res. 67 (2007) 2649-2656),
c) playing a role in building an immunosuppressive milieu by secreting immunosuppressive cytokines such as IL-4, I1-13, IL-1ra and IL-10, which in turn regulate T regulatory cell function. Conversely CD4 positive T cells have been shown to enhance the activity of tumor promoting macrophages in preclinical models (Mantovani, A. et al., Eur. J. Cancer 40 (2004) 1660-1667; DeNardo, D. et al., Cancer Cell 16 (2009) 91-102).

Accordingly, in several types of cancer (e.g. breast, ovarian, Hodgkin's lymphoma) the prevalence of M2 subtype tumor associated macrophages (TAMs) has been associated with poor prognosis (Bingle, L. et al., J. Pathol. 3 (2002) 254-265; Orre, M., and Rogers, P.A., Gynecol. Oncol. 1 (1999) 47-50; Steidl, C. et al., N. Engl. J. Med. 10 (2010) 875-885). Recent data show a correlation of CD163 positive macrophage infiltrate in tumors and tumor grade (Kawamura, K. et al., Pathol. Int. 59 (2009) 300-305). TAMs isolated from patient tumors had a tolerant phenotype and were not cytotoxic to tumor cells (Mantovani, A. et al., Eur. J. Cancer 40 (2004) 1660-1667). However, infiltration of TAMs in the presence of cytotoxic T cells correlates with improved survival in non small cell lung cancer and hence reflects a more prominent M1 macrophage infiltrate in this tumor type (Kawai, O. et al., Cancer 6 (2008) 1387-1395).

Recently, a so-called immune signature comprising high numbers of macrophages and CD4 positive T cells, but low numbers of cytotoxic CD8 positive T cells was shown to correlate with reduced overall survival (OS) in breast cancer patients and to represent an independent prognostic factor (DeNardo, D. et al., Cancer Discovery 1 (2011) 54-67).

Consistent with a role for CSF-1 in driving the pro-tumorigenic function of M2 macrophages, high CSF-1 expression in rare sarcomas or locally aggressive connective tissue tumors, such as pigmented villonodular synovitis (PVNS) and tenosynovial giant cell tumor (TGCT) due in part to a translocation of the CSF-1 gene, leads to the accumulation of monocytes and macrophages expressing the receptor for CSF-1, the colony-stimulating factor 1 receptor (CSF-1R) forming the majority of the tumor mass (West, R.B. et al., Proc. Natl. Acad. Sci. USA 3 (2006) 690-695). These tumors were subsequently used to define a CSF-1 dependent macrophage signature by gene expression profiling. In breast cancer and leiomyosarcoma patient tumors this CSF-1 response gene signature predicts poor prognosis (Espinosa, I. et al., Am. J. Pathol. 6 (2009) 2347-2356; Beck, A. et al., Clin. Cancer Res. 3 (2009) 778-787).

CSF-1 R belongs to the class III subfamily of receptor tyrosine kinases and is encoded by the c-fms proto-oncogene. Binding of CSF-1 or IL-34 induces receptor dimerization, followed by autophosphorylation and activation of downstream signaling cascades. Activation of CSF-1R regulates the survival, proliferation and differentiation of monocytes and macrophages (Xiong, Y. et al., J. Biol. Chem. 286 (2011) 952-960).

In addition to cells of the monocytic lineage and osteoclasts, which derive from the same hematopoietic precursor as the macrophage, CSF-1R/c-fms has also been found to be expressed by several human epithelial cancers such as ovarian and breast cancer and in leiomyosarcoma and TGCT/PVNS, albeit at lower expression levels compared to macrophages. As with TGCT/PVNS, elevated levels of CSF-1, the ligand for CSF-1R, in serum as well as ascites of ovarian cancer patients have been correlated with poor prognosis (Scholl, S. et al., Br. J. Cancer 62 (1994) 342-346; Price, F. et al., Am. J. Obstet. Gynecol. 168 (1993) 520-527). Furthermore, a constitutively active mutant form of CSF 1R is able to transform NIH3T3 cells, one of the properties of an oncogene (Chambers, S., Future Oncol 5 (2009) 1429-1440).

Preclinical models provide validation of CSF-1R as an oncology target. Blockade of CSF-1 as well as CSF-1R activity results in reduced recruitment of TAMs. Chemotherapy resulted in elevated CSF-1 expression in tumor cells leading to enhanced TAM recruitment. Blockade of CSF-1R in combination with paclitaxel resulted in activation of CD8 positive cytotoxic T cells leading to reduced tumor growth and metastatic burden in a spontaneous transgenic breast cancer model (DeNardo, D. et al., Cancer Discovery 1 (2011) 54-67).

The human CSF-1R (CSF-1 receptor; synonyms: M-CSF receptor; Macrophage colony-stimulating factor 1 receptor, Fms proto-oncogene, c-fms, SEQ ID NO: 22)) is known since 1986 (Coussens, L., et al., Nature 320 (1986) 277-280). CSF-1R is a growth factor and encoded by the c-fms proto-oncogene (reviewed e.g. in Roth, P. and Stanley, E.R., Curr. Top. Microbiol. Immunol. 181 (1992) 141-167).

CSF-1R is the receptor for the CSF-1R ligands CSF-1 (macrophage colony stimulating factor, also called M-CSF) (SEQ ID No.: 86) and IL-34 (SEQ ID No.: 87) and mediates the biological effects of these cytokines (Sherr, C.J., et al., Cell 41 (1985) 665-676; Lin, H., et al., Science 320 (2008) 807-811). The cloning of the colony stimulating factor-1 receptor (also called c-fms) was described for the first time in Roussel, M.F., et al., Nature 325 (1987) 549-552. In that publication, it was shown that CSF-1R had transforming potential dependent on changes in the C-terminal tail of the protein including the loss of the inhibitory tyrosine 969 phosphorylation which binds Cbl and thereby regulates receptor down regulation (Lee, P.S., et al., Embo J. 18 (1999) 3616-3628).

CSF-1R is a single chain, transmembrane receptor tyrosine kinase (RTK) and a member of the family of immunoglobulin (Ig) motif containing RTKs characterized by 5 repeated Ig-like subdomains D1-D5 in the extracellular domain (ECD) of the receptor (Wang, Z., et al Molecular and Cellular Biology 13 (1993) 5348-5359). The human CSF-1R Extracellular Domain (CSF-1R-ECD) (SEQ ID NO: 64) comprises all five extracellular Ig-like subdomains D1 -D5. The human CSF-1R fragment delD4 (SEQ ID NO: 65) comprises the extracellular Ig-like subdomains D1-D3 and D5, but is missing the D4 subdomain. The human CSF-1R fragment D1-D3 (SEQ ID NO: 66) comprises the respective subdomains D1-D3. The sequences are listed without the signal peptide MGSGPGVLLL LLVATAWHGQ G (SEQ ID NO: 67). The human CSF-1R fragment D4-D3 (SEQ ID NO: 85) comprises the respective subdomains D4-D3.

Currently two CSF-1R ligands that bind to the extracellular domain of CSF-1R are known. The first one is CSF-1 (colony stimulating factor 1, also called M-CSF, macrophage; human CSF-1, SEQ ID NO: 86) and is found extracellularly as a disulfide-linked homodimer (Stanley, E.R. et al., Journal of Cellular Biochemistry 21 (1983) 151-159; Stanley, E.R. et al., Stem Cells 12 Suppl. 1 (1995) 15-24). The second one is IL-34 (human IL-34; SEQ ID NO: 87) (Hume, D. A., et al, Blood 119 (2012) 1810-1820). Thus in one embodiment the term "CSF-1R ligand" refers to human CSF-1 (SEQ ID NO: 86) and/or human IL-34 (SEQ ID NO: 87).

For experiments often the active 149 amino acid (aa) fragment of human CSF-1 (aa 33-181 of SEQ ID NO: 86) is used. This active 149 aa fragment of human CSF-1 (aa 33-181 of SEQ ID NO: 86) is contained in all 3 major forms of CSF-1 and is sufficient to mediate binding to CSF-1R (Hume, D. A., et al, Blood 119 (2012) 1810-1820).

The main biological effects of CSF-1R signaling are the differentiation, proliferation, migration, and survival of hematopoietic precursor cells to the macrophage lineage (including osteoclast). Activation of CSF-1R is mediated by its CSF-1R ligands, CSF-1 (M-CSF) and IL-34. Binding of CSF-1 (M-CSF) to CSF-1R induces the formation of homodimers and activation of the kinase by tyrosine phosphorylation (Li, W. et al, EMBO Jounal. 10 (1991) 277-288; Stanley, E.R., et al., Mol. Reprod. Dev. 46 (1997) 4-10).

The intracellular protein tyrosine kinase domain is interrupted by a unique insert domain that is also present in the other related RTK class III family members that include the platelet derived growth factor receptors (PDGFR), stem cell growth factor receptor (c-Kit) and fins-like cytokine receptor (FLT3). In spite of the structural homology among this family of growth factor receptors, they have distinct tissue-specific functions.

CSF-1R is mainly expressed on cells of the monocytic lineage and in the female reproductive tract and placenta. In addition expression of CSF-1R has been reported in Langerhans cells in skin, a subset of smooth muscle cells (Inaba, T., et al., J. Biol. Chem. 267 (1992) 5693-5699), B cells (Baker, A.H., et al., Oncogene 8 (1993) 371-378) and microglia (Sawada, M., et al., Brain Res. 509 (1990) 119-124). Cells with mutant human CSF-1R ((SEQ ID NO: 23) are known to proliferate independently of ligand stimulation.

As used herein, "binding to human CSF-1R" or "specifically binding to human CSF-1R" or "which binds to human CSF-1R" or "anti-CSF-1R antibody" refers to an antibody specifically binding to the human CSF-1R antigen with a binding affinity of KD-value of 1.0 x 10⁻⁸ mol/l or lower, in one embodiment of a KD-value of 1.0 x10⁻⁹ mol/l or lower. The binding affinity is determined with a standard binding assay, such as surface plasmon resonance technique (BIAcore®, GE-Healthcare Uppsala, Sweden). Thus an "antibody binding to human CSF-1R" as used herein refers to an antibody specifically binding to the human CSF-1R antigen with a binding affinity of KD 1.0 x 10⁻⁸ mol/l or lower (in one embodiment 1.0 x 10⁻⁸ mol/l - 1.0 x 10⁻¹³ mol/1), in on embodiment of a KD 1.0 x10⁻⁹ mol/l or lower (in one embodiment 1.0 x 10⁻⁹ mol/l - 1.0 x 10⁻¹³ mol/l).

CD20 (also known as B-lymphocyte antigen CD20, B-lymphocyte surface antigen B1, Leu-16, Bp35, BM5, and LF5; the sequence is characterized by the SwissProt database entry P11836) is a hydrophobic transmembrane protein with a molecular weight of approximately 35 kD located on pre-B and mature B lymphocytes (Valentine, M.A. et al., J. Biol. Chem. 264 (1989) 11282-11287; Tedder, T.F., et al., Proc. Natl. Acad. Sci. U.S.A. 85 (1988) 208-212; Stamenkovic, I., et al., J. Exp. Med. 167 (1988) 1975-1980; Einfeld, D.A., et al., EMBO J. 7 (1988) 711-717; Tedder, T.F., et al., J. Immunol. 142 (1989) 2560-2568). The corresponding human gene is Membrane-spanning 4-domains, subfamily A, member 1, also known as MS4A1. This gene encodes a member of the membrane-spanning 4A gene family. Members of this nascent protein family are characterized by common structural features and similar intron/exon splice boundaries and display unique expression patterns among hematopoietic cells and nonlymphoid tissues. This gene encodes the B-lymphocyte surface molecule which plays a role in the development and differentiation of B-cells into plasma cells. This family member is localized to 11q12, among a cluster of family members. Alternative splicing of this gene results in two transcript variants which encode the same protein.

The terms "CD20" and "CD20 antigen" are used interchangeably herein, and include any variants, isoforms and species homologs of human CD20 which are naturally expressed by cells or are expressed on cells transfected with the CD20 gene. Binding of an antibody of the invention to the CD20 antigen mediate the killing of cells expressing CD20 (e.g., a tumor cell) by inactivating CD20. The killing of the cells expressing CD20 may occur by one or more of the following mechanisms: Cell death/apoptosis induction, ADCC and CDC.

Synonyms of CD20, as recognized in the art, include B-lymphocyte antigen CD20, B-lymphocyte surface antigen B1, Leu-16, Bp35, BM5, and LF5.

The term "anti-CD20 antibody" according to the invention is an antibody that binds specifically to human CD20 antigen. Depending on binding properties and biological activities of anti-CD20 antibodies to the CD20 antigen, two types of anti-CD20 antibodies (type I and type II anti-CD20 antibodies) can be distinguished according to Cragg, M.S., et al., Blood 103 (2004) 2738-2743; and Cragg, M.S., et al., Blood 101 (2003) 1045-1052, see Table 2.

**Table 1: Properties of type I and type II anti-CD20 antibodies**

| **type I anti-CD20 antibodies** | **type II anti-CD20 antibodies** |
|---|---|
| type I CD20 epitope | type II CD20 epitope |
| Localize CD20 to lipid rafts | Do not localize CD20 to lipid rafts |
| Increased CDC (if IgG1 isotype) | Decreased CDC (if IgG1 isotype) |
| ADCC activity (if IgG1 isotype) | ADCC activity (if IgG1 isotype) |
| Full binding capacity | Reduced binding capacity |
| Homotypic aggregation | Stronger homotypic aggregation |
| Apoptosis induction upon cross-linking | Strong cell death induction without cross-linking |

Examples of type II anti-CD20 antibodies include e.g. humanized B-Ly1 antibody IgG1 (a chimeric humanized IgG1 antibody as disclosed in WO 2005/044859), 11B8 IgG1 (as disclosed in WO 2004/035607), and AT80 IgG1. Typically type II anti-CD20 antibodies of the IgG1 isotype show characteristic CDC properties. Type II anti-CD20 antibodies have a decreased CDC (if IgG1 isotype) compared to type I antibodies of the IgG1 isotype.

Examples of type I anti-CD20 antibodies include e.g. rituximab, HI47 IgG3 (ECACC, hybridoma), 2C6 IgG1 (as disclosed in WO 2005/103081), 2F2 IgG1 (as disclosed and WO 2004/035607 and WO 2005/103081) and 2H7 IgG1 (as disclosed in WO 2004/056312).

The "rituximab" antibody (reference antibody; example of a type I anti-CD20 antibody) is a genetically engineered chimeric human gamma 1 murine constant domain containing monoclonal antibody directed against the human CD20 antigen. This chimeric antibody contains human gamma 1 constant domains and is identified by the name "C2B8" in US 5,736,137 (Andersen et. al.) issued on April 17, 1998, assigned to IDEC Pharmaceuticals Corporation. Rituximab is approved for the treatment of patients with relapsed or refracting low-grade or follicular, CD20 positive, B cell non-Hodgkin's lymphoma. In vitro mechanism of action studies have shown that rituximab exhibits human complement--dependent cytotoxicity (CDC) (Reff, M.E., et. al., Blood 83 (1994) 435-445). Additionally, it exhibits significant activity in assays that measure antibody-dependent cellular cytotoxicity (ADCC). Rituximab is not afucosylated.

| Antibody | Amount of fucose |
|---|---|
| Rituximab (non-afucosylated) | >85 % |
| Wild type afucosylated glyco-engineered humanized B-Ly1 (B-HH6-B-KV1) (non-afucosylated) | >85 % |
| afucosylated glyco-engineered humanized B-Lyl (B-HH6-B-KV1 GE) | 45-55 % |

As used herein, "binding to human CD20" or "specifically binding to human CD20" or "which binds to human CD20" or "anti- CD20 antibody" refers to an antibody specifically binding to the human CD20 antigen with a binding affinity of KD-value of 1.0 x 10⁻⁸ mol/l or lower, in one embodiment of a KD-value of 1.0 x10⁻⁹ mol/l or lower. The binding affinity is determined with a standard binding assay, such as surface plasmon resonance technique (BIAcore®, GE-Healthcare Uppsala, Sweden). Thus an "antibody binding to human CD20" as used herein refers to an antibody specifically binding to the human CD20 antigen with a binding affinity of KD 1.0 x 10⁻⁸ mol/l or lower (in one embodiment 1.0 x 10⁻⁸ mol/l - 1.0 x 10⁻¹³ mol/l), in on embodiment of a KD 1.0 x10⁻⁹ mol/l or lower (in one embodiment 1.0 x 10⁻⁹ mol/l - 1.0 x 10⁻¹³ mol/l).

In one embodiment the antibody which binds to human CSF-1R used in the combination therapy described herein is selected from the group consisting of hMab 2F11-c11 , hMab 2F11-d8 , hMab 2F11-e7 , hMab 2F11-f12 , and hMab 2F11-g1.

These antibodies are described in WO2011/070024 and are characterized in comprising the following VH and VL sequences as described herein:

**Table 2:**

| anti-CSF-1R antibody | amino acid sequence of the heavy chain variable domain VH, SEQ ID NO: | amino acid sequence of the light chain variable domain VL, SEQ ID NO: |
|---|---|---|
| hMab 2F11-c11 | 23 | 24 |
| hMab 2F11-d8 | 31 | 32 |
| hMab 2F11-e7 | 39 | 40 |
| hMab 2F11-f12 | 47 | 48 |
| hMab 2F11-g1 | 55 | 56 |

In one preferred embodiment the antibody which binds to human CSF-1R used in the combination therapy described herein is hMab 2F11-e7 (VH, SEQ ID NO:39; VL, SEQ ID NO:40).

In one embodiment the antibody which binds to human CD20 used in the combination therapy described herein is a humanized B-Ly1 antibody:
The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

In one embodiment, the "humanized B-Ly1 antibody" has variable region of the heavy chain (VH) selected from group of SEQ ID No.69 to SEQ ID No.75 (B-HH2, BHH-3, B-HH6, B-HH8, B-HL8, B-HL11 and B-HL13 of WO 2005/044859 and WO 2007/031875). In one embodiment, the "humanized B-Ly1 antibody" has variable region of the light chain (VL) of SEQ ID No. 76 (B-KV1 of WO 2005/044859 and WO 2007/031875).

These antibodies are characterized in comprising the following VH and VL sequences as described herein:

**Table 3:**

| anti-CD20 antibody IgG1 VH/VL | amino acid sequence of the heavy chain variable domain VH, SEQ ID NO: | amino acid sequence of the light chain variable domain VL, SEQ ID NO: |
|---|---|---|
| B-HH2 / B-KV1 | 69 | 76 |
| B-HH3/ B-KV1 | 70 | 76 |
| B-HH6/ B-KV1 | 71 | 76 |
| B-HH8/ B-KV 1 | 72 | 76 |
| B-HL8/ B-KV1 | 73 | 76 |
| B-HL11/ B-KV1 | 74 | 76 |
| B-HL13/ B-KV1 | 75 | 76 |

In one preferred embodiment the antibody which binds to human CD20 used in the combination therapy described herein is selected the humanized B-Ly1 antibody B-HH6/B-KV1 (comprising a variable region of the heavy chain (VH) of SEQ ID No.71 (B-HH6)and a variable region of the light chain (VL) of SEQ ID No. 76 (B-KV1).

Furthermore in one embodiment, the humanized B-Ly1 antibody is an afucosylated antibody of IgG1 isotype.

According to the invention such afucosylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one embodiment, the afucosylated glyco-engineered humanized B-Ly1 is B-HH6-B-KV1 GE. Such glycoengineered humanized B-Ly1 antibodies have an altered pattern of glycosylation in the Fc region, preferably having a reduced level of fucose residues. In one embodiment, the amount of fucose is 60% or less of the total amount of oligosaccharides at Asn297 (in one embodiment the amount of fucose is between 40% and 60%, in another embodiment the amount of fucose is 50% or less, and in still another embodiment the amount of fucose is 30% or less). In another embodiment, the oligosaccharides of the Fc region are preferably bisected. These glycoengineered humanized B-Ly1 antibodies have an increased ADCC.

In one preferred embodiment the antibody which binds to human CD20 used in the combination therapy described herein is selected the humanized B-Ly1 antibody B-HH6/B-KV1(comprising a variable region of the heavy chain (VH) of SEQ ID No.71 (B-HH6)and a variable region of the light chain (VL) of SEQ ID No. 76 (B-KV1) and is an afucosylated antibody of IgG1 isotype having an altered pattern of glycosylation in the Fc region wherein the amount of fucose containing oligosaccharides is between 40% and 60% of the total amount of oligosaccharides at Asn297.

The afucosylated anti-CD20 antibodies according to the invention have an increased antibody dependent cellular cytotoxicity (ADCC) unlike anti-CD20 antibodies having no reduced fucose.

By "afucosylated anti-CD20 antibody with increased antibody dependent cellular cytotoxicity (ADCC)" is meant an afucosylated anti-CD20 antibody, as that term is defined herein, having increased ADCC as determined by any suitable method known to those of ordinary skill in the art. One accepted in vitro ADCC assay is as follows:
1) the assay uses target cells that are known to express the target antigen recognized by the antigen-binding region of the antibody;
2) the assay uses human peripheral blood mononuclear cells (PBMCs), isolated from blood of a randomly chosen healthy donor, as effector cells;
3) the assay is carried out according to following protocol:
   i) the PBMCs are isolated using standard density centrifugation procedures and are suspended at 5 x 10⁶ cells/ml in RPMI cell culture medium;
   ii) the target cells are grown by standard tissue culture methods, harvested from the exponential growth phase with a viability higher than 90%, washed in RPMI cell culture medium, labeled with 100 micro-Curies of ⁵¹Cr, washed twice with cell culture medium, and resuspended in cell culture medium at a density of 10⁵ cells/ml;
   iii) 100 microliters of the final target cell suspension above are transferred to each well of a 96-well microtiter plate;
   iv) the antibody is serially-diluted from 4000 ng/ml to 0.04 ng/ml in cell culture medium and 50 microliters of the resulting antibody solutions are added to the target cells in the 96-well microtiter plate, testing in triplicate various antibody concentrations covering the whole concentration range above;
   v) for the maximum release (MR) controls, 3 additional wells in the plate containing the labeled target cells, receive 50 microliters of a 2% (VN) aqueous solution of non-ionic detergent (Nonidet, Sigma, St. Louis), instead of the antibody solution (point iv above);
   vi) for the spontaneous release (SR) controls, 3 additional wells in the plate containing the labeled target cells, receive 50 microliters of RPMI cell culture medium instead of the antibody solution (point iv above);
   vii) the 96-well microtiter plate is then centrifuged at 50 x g for 1 minute and incubated for 1 hour at 4°C;
   viii) 50 microliters of the PBMC suspension (point i above) are added to each well to yield an effector:target cell ratio of 25: 1 and the plates are placed in an incubator under 5% CO2 atmosphere at 37 C for 4 hours;
   ix) the cell-free supernatant from each well is harvested and the experimentally released radioactivity (ER) is quantified using a gamma counter;
   x) the percentage of specific lysis is calculated for each antibody concentration according to the formula (ER-MR)/(MR-SR) x 100, where ER is the average radioactivity quantified (see point ix above) for that antibody concentration, MR is the average radioactivity quantified (see point ix above) for the MR controls (see point V above), and SR is the average radioactivity quantified (see point ix above) for the SR controls (see point vi above);
4) "increased ADCC" is defined as either an increase in the maximum percentage of specific lysis observed within the antibody concentration range tested above, and/or a reduction in the concentration of antibody required to achieve one half of the maximum percentage of specific lysis observed within the antibody concentration range tested above. The increase in ADCC is relative to the ADCC, measured with the above assay, mediated by the same antibody, produced by the same type of host cells, using the same standard production, purification, formulation and storage methods, which are known to those skilled in the art, but that has not been produced by host cells engineered to overexpress GnTIII.

Said "increased ADCC" can be obtained by glycoengineering of said antibodies, that means enhance said natural, cell-mediated effector functions of monoclonal antibodies by engineering their oligosaccharide component as described in Umana, P., et al., Nature Biotechnol. 17 (1999) 176-180 and US 6,602,684.

The term "complement-dependent cytotoxicity (CDC)" refers to lysis of human tumor target cells by the antibody according to the invention in the presence of complement. CDC is measured preferably by the treatment of a preparation of CD20 expressing cells with an anti-CD20 antibody according to the invention in the presence of complement. CDC is found if the antibody induces at a concentration of 100 nM the lysis (cell death) of 20% or more of the tumor cells after 4 hours. The assay is performed preferably with ⁵¹Cr or Eu labeled tumor cells and measurement of released ⁵¹Cr or Eu. Controls include the incubation of the tumor target cells with complement but without the antibody.

The term "afucosylated antibody" refers to an antibody of IgG1 or IgG3 isotype (preferably of IgG1 isotype) with an altered pattern of glycosylation in the Fc region at Asn297 having a reduced level of fucose residues. Glycosylation of human IgG1 or IgG3 occurs at Asn297 as core fucosylated bianntennary complex oligosaccharide glycosylation terminated with up to 2 Gal residues. These structures are designated as G0, G1 (α1,6 or α1,3) or G2 glycan residues, depending from the amount of terminal Gal residues (Raju, T.S., BioProcess Int. 1 (2003) 44-53). CHO type glycosylation of antibody Fc parts is e.g. described by Routier, F.H., Glycoconjugate J. 14 (1997) 201-207. Antibodies which are recombinantely expressed in non glycomodified CHO host cells usually are fucosylated at Asn297 in an amount of at least 85%. It should be understood that the term an afucosylated antibody as used herein includes an antibody having no fucose in its glycosylation pattern. It is commonly known that typical glycosylated residue position in an antibody is the asparagine at position 297 according to the EU numbering system ("Asn297").

The "EU numbering system" or "EU index" is generally used when referring to a residue in an immunoglobulin heavy chain constant region (e.g., the EU index reported in Kabat et al., Sequences of Proteins of Immunological Interest, 5th ed., Public Health Service, National Institutes of Health, Bethesda, MD (1991)).

Thus an afucosylated antibody according to the invention means an antibody of IgG1 or IgG3 isotype (preferably of IgG1 isotype) wherein the amount of fucose is 60% or less of the total amount of oligosaccharides (sugars) at Asn297 (which means that at least 40% or more of the oligosaccharides of the Fc region at Asn297 are afucosylated). In one embodiment the amount of fucose is between 40% and 60% of the oligosaccharides of the Fc region at Asn297. In another embodiment the amount of fucose is 50% or less, and in still another embodiment the amount of fucose is 30% or less of the oligosaccharides of the Fc region at Asn297. According to the invention "amount of fucose" means the amount of said oligosaccharide (fucose) within the oligosaccharide (sugar) chain at Asn297, related to the sum of all oligosaccharides (sugars) attached to Asn 297 (e. g. complex, hybrid and high mannose structures) measured by MALDI-TOF mass spectrometry and calculated as average value (for a detailed procedure to determine the amount of fucose, see e.g. WO 2008/077546). Furthermore in one embodiment, the oligosaccharides of the Fc region are bisected. The afucosylated antibody according to the invention can be expressed in a glycomodified host cell engineered to express at least one nucleic acid encoding a polypeptide having GnTIII activity in an amount sufficient to partially fucosylate the oligosaccharides in the Fc region. In one embodiment, the polypeptide having GnTIII activity is a fusion polypeptide. Alternatively α1,6-fucosyltransferase activity of the host cell can be decreased or eliminated according to US 6,946,292 to generate glycomodified host cells. The amount of antibody fucosylation can be predetermined e.g. either by fermentation conditions (e.g. fermentation time) or by combination of at least two antibodies with different fucosylation amount. Such afucosylated antibodies and respective glycoengineering methods are described in WO 2005/044859, WO 2004/065540, WO 2007/031875, Umana, P., et al., Nature Biotechnol. 17 (1999) 176-180, WO 99/154342, WO 2005/018572, WO 2006/116260, WO 2006/114700, WO 2005/011735, WO 2005/027966, WO 97/028267, US 2006/0134709, US 2005/0054048, US 2005/0152894, WO 2003/035835, WO 2000/061739. These glycoengineered antibodies have an increased ADCC. Other glycoengineering methods yielding afucosylated antibodies according to the invention are described e.g. in Niwa, R.. et al., J. Immunol. Methods 306 (2005) 151-160; Shinkawa, T., et al., J. Biol. Chem, 278 (2003) 3466-3473; WO 03/055993 or US 2005/0249722.

Thus one aspect of the invention is an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the treatment of cancer in combination with an CSF1R antibody as described herein. In another aspect of the invention is the use of an afucosylated anti-CD20 antibody of IgG1 or IgG3 isotype (preferably of IgG1 isotype) specifically binding to CD20 with an amount of fucose of 60% or less of the total amount of oligosaccharides (sugars) at Asn297, for the manufacture of a medicament for the treatment of cancer in combination with an CSF1R antibody as described herein. In one embodiment the amount of fucose is between 60% and 20% of the total amount of oligosaccharides (sugars) at Asn297. In one embodiment the amount of fucose is between 60% and 40% of the total amount of oligosaccharides (sugars) at Asn297.

In one embodiment the amount of fucose is between 0% of the total amount of oligosaccharides (sugars) at Asn297.

In one embodiment of the invention the antibody which binds to human CSF-1R used in the combination therapy described herein is characterized in comprising
a) a heavy chain variable domain VH of SEQ ID NO:23 and a light chain variable domain VL of SEQ ID NO:24, or
b) a heavy chain variable domain VH of SEQ ID NO:31 and a light chain variable domain VL of SEQ ID NO:32, or
c) a heavy chain variable domain VH of SEQ ID NO:39 and a light chain variable domain VL of SEQ ID NO:40, or
d) a heavy chain variable domain VH of SEQ ID NO:47 and a light chain variable domain VL of SEQ ID NO:48, or
e) a heavy chain variable domain VH of SEQ ID NO:55 and a light chain variable domain VL of SEQ ID NO:56; and
wherein the antibody which binds to human CD20 used in the combination therapy is characterized in comprising
a) a heavy chain variable domain VH of SEQ ID NO:69 and a light chain variable domain VL of SEQ ID NO:76, or
b) a heavy chain variable domain VH of SEQ ID NO:70 and a light chain variable domain VL of SEQ ID NO:76, or
c) a heavy chain variable domain VH of SEQ ID NO:71 and a light chain variable domain VL of SEQ ID NO:76, or
d) a heavy chain variable domain VH of SEQ ID NO:72 and a light chain variable domain VL of SEQ ID NO:76, or
e) a heavy chain variable domain VH of SEQ ID NO:73 and a light chain variable domain VL of SEQ ID NO:76, or
f) a heavy chain variable domain VH of SEQ ID NO:74 and a light chain variable domain VL of SEQ ID NO:76, or
g) a heavy chain variable domain VH of SEQ ID NO:75 and a light chain variable domain VL of SEQ ID NO:76.

In one embodiment the antibody which binds to human CSF-1R used in the combination therapy is characterized in comprising
a heavy chain variable domain VH of SEQ ID NO:39 and a light chain variable domain VL of SEQ ID NO:40;
   and
   wherein the antibody which binds to human CD20 used in the combination therapy is characterized in comprising
a heavy chain variable domain VH of SEQ ID NO:71 and a light chain variable domain VL of SEQ ID NO:76

The term "epitope" denotes a protein determinant of human CSF-1R or CD20 capable of specifically binding to an antibody. Epitopes usually consist of chemically active surface groupings of molecules such as amino acids or sugar side chains and usually epitopes have specific three dimensional structural characteristics, as well as specific charge characteristics. Conformational and nonconformational epitopes are distinguished in that the binding to the former but not the latter is lost in the presence of denaturing solvents.

The "variable domain" (light chain variable domain VL, heavy chain variable domain VH) as used herein denotes each of the pair of light and heavy chain domains which are involved directly in binding the antibody to the antigen. The variable light and heavy chain domains have the same general structure and each domain comprises four framework (FR) regions whose sequences are widely conserved, connected by three "hypervariable regions" (or complementary determining regions, CDRs). The framework regions adopt a beta-sheet conformation and the CDRs may form loops connecting the beta-sheet structure. The CDRs in each chain are held in their three-dimensional structure by the framework regions and form together with the CDRs from the other chain the antigen binding site. The antibody's heavy and light chain CDR3 regions play a particularly important role in the binding specificity/affinity of the antibodies according to the invention and therefore provide a further object of the invention.

The term "antigen-binding portion of an antibody" when used herein refer to the amino acid residues of an antibody which are responsible for antigen-binding. The antigen-binding portion of an antibody comprises amino acid residues from the "complementary determining regions" or "CDRs". "Framework" or "FR" regions are those variable domain regions other than the hypervariable region residues as herein defined. Therefore, the light and heavy chain variable domains of an antibody comprise from N- to C-terminus the domains FR1, CDR1, FR2, CDR2, FR3, CDR3, and FR4. Especially, CDR3 of the heavy chain is the region which contributes most to antigen binding and defines the antibody's properties. CDR and FR regions are determined according to the standard definition of Kabat et al., Sequences of Proteins of Immunological Interest, 5th ed., Public Health Service, National Institutes of Health, Bethesda, MD (1991) and/or those residues from a "hypervariable loop".

The terms "nucleic acid" or "nucleic acid molecule", as used herein, are intended to include DNA molecules and RNA molecules. A nucleic acid molecule may be single-stranded or double-stranded, but preferably is double-stranded DNA.

The term "amino acid" as used within this application denotes the group of naturally occurring carboxy alpha-amino acids comprising alanine (three letter code: ala, one letter code: A), arginine (arg, R), asparagine (asn, N), aspartic acid (asp, D), cysteine (cys, C), glutamine (gln, Q), glutamic acid (glu, E), glycine (gly, G), histidine (his, H), isoleucine (ile, I), leucine (leu, L), lysine (lys, K), methionine (met, M), phenylalanine (phe, F), proline (pro, P), serine (ser, S), threonine (thr, T), tryptophan (trp, W), tyrosine (tyr, Y), and valine (val, V).

The "Fc part" of an antibody is not involved directly in binding of an antibody to an antigen, but exhibit various effector functions. A "Fc part of an antibody" is a term well known to the skilled artisan and defined on the basis of papain cleavage of antibodies. Depending on the amino acid sequence of the constant region of their heavy chains, antibodies or immunoglobulins are divided in the classes: IgA, IgD, IgE, IgG and IgM, and several of these may be further divided into subclasses (isotypes), e.g. IgG1, IgG2, IgG3, and IgG4, IgA1, and IgA2. According to the heavy chain constant regions the different classes of immunoglobulins are called α, δ, ε, γ, and µ, respectively. The Fc part of an antibody is directly involved in ADCC (antibody-dependent cell-mediated cytotoxicity) and CDC (complement-dependent cytotoxicity) based on complement activation, C1q binding and Fc receptor binding. Complement activation (CDC) is initiated by binding of complement factor C1q to the Fc part of most IgG antibody subclasses. While the influence of an antibody on the complement system is dependent on certain conditions, binding to C1q is caused by defined binding sites in the Fc part. Such binding sites are known in the state of the art and described e.g. by Boackle, R.J., et al., Nature 282 (1979) 742-743; Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol.164 (2000) 4178-4184; Hezareh, M., et al., J. Virology 75 (2001) 12161-12168; Morgan, A., et al., Immunology 86 (1995) 319-324; EP 0 307 434. Such binding sites are e.g. L234, L235, D270, N297, E318, K320, K322, P331 and P329 (numbering according to EU index of Kabat, E.A., see below). Antibodies of subclass IgG1, IgG2 and IgG3 usually show complement activation and C1q and C3 binding, whereas IgG4 do not activate the complement system and do not bind C1q and C3.

In one embodiment the antibody according to the invention comprises an Fc part derived from human origin and preferably all other parts of the human constant regions. As used herein the term "Fc part derived from human origin" denotes a Fc part which is either a Fc part of a human antibody of the subclass IgG1, IgG2, IgG3 or IgG4, preferably a Fc part from human IgG1 subclass, a mutated Fc part from human IgG1 subclass (in one embodiment with a mutation on L234A + L235A), a Fc part from human IgG4 subclass or a mutated Fc part from human IgG4 subclass (in one embodiment with a mutation on S228P). In one preferred embodiment the human heavy chain constant region is SEQ ID NO: 58 (human IgG1 subclass), in another preferred embodiment the human heavy chain constant region is SEQ ID NO: 59 (human IgG1 subclass with mutations L234A and L235A), in another preferred embodiment the human heavy chain constant region is SEQ ID NO: 60 (human IgG4 subclass), and in another preferred embodiment the human heavy chain constant region is SEQ ID NO: 61 (human IgG4 subclass with mutation S228P). In one embodiment said antibodies have reduced or minimal effector function. In one embodiment the minimal effector function results from an effectorless Fc mutation. In one embodiment the effectorless Fc mutation is L234A/L235A or L234A/L235A/P329G or N297A or D265A/N297A. In one embodiment the effectorless Fc mutation is selected for each of the antibodies independently of each other from the group comprising (consisting of) L234A/L235A, L234A/L235A/P329G, N297A and D265A/N297A.

In one embodiment the antibodies described herein are of human IgG class (i.e. of IgG1, IgG2, IgG3 or IgG4 subclass).

In a preferred embodiment the antibodies described herein are of human IgG1 subclass or of human IgG4 subclass. In one embodiment the described herein are of human IgG1 subclass. In one embodiment the antibodies described herein are of human IgG4 subclass.

In one embodiment the antibody described herein is characterized in that the constant chains are of human origin. Such constant chains are well known in the state of the art and e.g. described by Kabat, E.A., (see e.g. Johnson, G. and Wu, T.T., Nucleic Acids Res. 28 (2000) 214-218). For example, a useful human heavy chain constant region comprises an amino acid sequence of SEQ ID NO: 58. For example, a useful human light chain constant region comprises an amino acid sequence of a kappa-light chain constant region of SEQ ID NO: 57.

The invention comprises a method for the treatment of a patient in need of therapy, characterized by administering to the patient a therapeutically effective amount of an antibody according to the invention.

The invention comprises the use of an antibody according to the invention for the described therapy.

One preferred embodiment of the invention are the CSF-1R antibodies of the present invention for use in the treatment of "CSF-1R mediated diseases" or the CSF-1R antibodies of the present invention for use for the manufacture of a medicament in the treatment of "CSF-1R mediated diseases", which can be described as follows:
There are 3 distinct mechanisms by which CSF-1R signaling is likely involved in tumor growth and metastasis. The first is that expression of CSF-ligand and receptor has been found in tumor cells originating in the female reproductive system (breast, ovarian, endometrium, cervical) (Scholl, S.M., et al., J. Natl. Cancer Inst. 86 (1994) 120-126; Kacinski, B.M., Mol. Reprod. Dev. 46 (1997) 71-74; Ngan, H.Y., et al., Eur. J. Cancer 35 (1999) 1546-1550; Kirma, N., et al., Cancer Res 67 (2007) 1918-1926) and the expression has been associated with breast cancer xenograft growth as well as poor prognosis in breast cancer patients. Two point mutations were seen in CSF-1R in about 10-20% of acute myelocytic leukemia, chronic myelocytic leukemia and myelodysplasia patients tested in one study, and one of the mutations was found to disrupt receptor turnover (Ridge, S.A., et al., Proc. Natl. Acad. Sci USA 87 (1990) 1377-1380). However the incidence of the mutations could not be confirmed in later studies (Abu-Duhier, F.M., et al., Br. J. Haematol. 120 (2003) 464-470). Mutations were also found in some cases of hepatocellular cancer (Yang, D.H., et al., Hepatobiliary Pancreat. Dis. Int. 3 (2004) 86-89) and idiopathic myelofibrosis (Abu-Duhier, F.M., et al., Br. J. Haematol. 120 (2003) 464-470). Recently, in the GDM-1 cell line derived from a patient with myelomonoblastic leukemia the Y571D mutation in CSF-1R was identified (Chase, A., et al., Leukemia 23 (2009) 358-364).

The second mechanism is based on blocking signaling through M-CSF/CSF-1R at metastatic sites in bone which induces osteoclastogenesis, bone resorption and osteolytic bone lesions. Breast, multiple myeloma and lung cancers are examples of cancers that have been found to metastasize to the bone and cause osteolytic bone disease resulting in skeletal complications. M-CSF released by tumor cells and stroma induces the differentiation of hematopoietic myeloid monocyte progenitors to mature osteoclasts in collaboration with the receptor activator of nuclear factor kappa-B ligand-RANKL. During this process, M-CSF acts as a permissive factor by giving the survival signal to osteoclasts (Tanaka, S., et al., J. Clin. Invest. 91 (1993) 257-263). Inhibition of CSF-1R activity during osteoclast differentiation and maturation with an anti-CSF-1R antibody is likely to prevent unbalanced activity of osteoclasts that cause osteolytic disease and the associated skeletal related events in metastatic disease. Whereas breast, lung cancer and multiple myeloma typically result in osteolytic lesions, metastasis to the bone in prostate cancer initially has an osteoblastic appearance in which increased bone forming activity results in 'woven bone' which is different from typical lamellar structure of normal bone. During disease progression bone lesions display a significant osteolytic component as well as high serum levels of bone resorption and suggests that anti-resorptive therapy may be useful. Bisphosphonates have been shown to inhibit the formation of osteolytic lesions and reduced the number of skeletal-related events only in men with hormone-refractory metastatic prostate cancer but at this point their effect on osteoblastic lesions is controversial and bisphosphonates have not been beneficial in preventing bone metastasis or hormone responsive prostate cancer to date. The effect of anti-resorptive agents in mixed osteolytic/osteoblastic prostate cancer is still being studied in the clinic (Choueiri, M.B., et al., Cancer Metastasis Rev. 25 (2006) 601-609; Vessella, R.L. and Corey, E., Clin. Cancer Res. 12 (20 Pt 2) (2006) 6285s-6290s).

The third mechanism is based on the recent observation that tumor associated macrophages (TAM) found in solid tumors of the breast, prostate, ovarian and cervical cancers correlated with poor prognosis (Bingle, L., et al., J. Pathol. 196 (2002) 254-265; Pollard, J.W., Nat. Rev. Cancer 4 (2004) 71-78). Macrophages are recruited to the tumor by M-CSF and other chemokines. The macrophages can then contribute to tumor progression through the secretion of angiogenic factors, proteases and other growth factors and cytokines and may be blocked by inhibition of CSF-1R signaling. Recently it was shown by Zins et a1 (Zins, K., et al., Cancer Res. 67 (2007) 1038-1045) that expression of siRNA of Tumor necrosis factor alpha (TNF alpha), M-CSF or the combination of both would reduce tumor growth in a mouse xenograft model between 34% and 50% after intratumoral injection of the respective siRNA. SiRNA targeting the TNF alpha secreted by the human SW620 cells reduced mouse M-CSF levels and led to reduction of macrophages in the tumor. In addition treatment of MCF7 tumor xenografts with an antigen binding fragment directed against M-CSF did result in 40% tumor growth inhibition, reversed the resistance to chemotherapeutics and improved survival of the mice when given in combination with chemotherapeutics (Paulus, P., et al., Cancer Res. 66 (2006) 4349-4356).

TAMs are only one example of an emerging link between chronic inflammation and cancer. There is additional evidence for a link between inflammation and cancer as many chronic diseases are associated with an increased risk of cancer, cancers arise at sites of chronic inflammation, chemical mediators of inflammation are found in many cancers; deletion of the cellular or chemical mediators of inflammation inhibits development of experimental cancers and long-term use of anti-inflammatory agents reduce the risk of some cancers. A link to cancer exists for a number of inflammatory conditions among- those H.pylori induced gastritis for gastric cancer, Schistosomiasis for bladder cancer, HHVX for Kaposi's sarcoma, endometriosis for ovarian cancer and prostatitis for prostate cancer (Balkwill, F., et al., Cancer Cell 7 (2005) 211-217). Macrophages are key cells in chronic inflammation and respond differentially to their microenvironment. There are two types of macrophages that are considered extremes in a continuum of functional states: M1 macrophages are involved in Type 1 reactions. These reactions involve the activation by microbial products and consequent killing of pathogenic microorganisms that result in reactive oxygen intermediates. On the other end of the extreme are M2 macrophages involved in Type 2 reactions that promote cell proliferation, tune inflammation and adaptive immunity and promote tissue remodeling, angiogenesis and repair (Mantovani, A., et al., Trends Immunol. 25 (2004) 677-686). Chronic inflammation resulting in established neoplasia is usually associated with M2 macrophages. A pivotal cytokine that mediates inflammatory reactions is TNF alpha that true to its name can stimulate anti-tumor immunity and hemorrhagic necrosis at high doses but has also recently been found to be expressed by tumor cells and acting as a tumor promoter (Zins, K., et al., Cancer Res. 67 (2007) 1038-1045; Balkwill, F., Cancer Metastasis Rev. 25 (2006) 409-416). The specific role of macrophages with respect to the tumor still needs to be better understood including the potential spatial and temporal dependence on their function and the relevance to specific tumor types.

Thus one embodiment of the invention are the CSF-1R antibodies described herein in for use in the treatment of cancer in combination with an anti-CD20 antibody as described herein.

### Detailed description of the embodiments of the invention

In one embodiment, the invention relates to a combination of an antibody which binds to CSF-1R and an antibody which binds CD 20 for use in inducing lymphocytosis of leukemic cells in lymphomas or leukemias.

"Lymphocytosis of leukemic cells in lymphomas or leukemias" as used herein refers to an absolute or relative increase of circulating leukemic cells in the peripheral blood of patients suffering from/afflicted with lymphomas or leukemias. In one embodiment the lymphocytosis increases the percentage of CD19 expressing and/or CD20 expressing circulating leukemic cells in the peripheral blood. In one preferred embodiment the lymphocytosis increases the percentage of CD19 expressing and/or CD20 expressing circulating leukemic cells in the peripheral blood (see also Hallek M, Cheson BD, Catovsky D, et al. Response assessment in chronic lymphocytic leukemia treated with novel agents causing an increase of peripheral blood lymphocytes. Blood. Epub June 4, 2012). Examples of such lmphocytosis are e.g. described below where two administrations of the anti-CSFIR moAb (Figure 6A) induced significant lymphocytosis (Figure 6B), a treatment-related compartment shift from infiltrated tissues into peripheral blood (PB). Similar lymphocytosis was also described for CLL patients with agents targeting BCR signaling (e.g. ibrutinib) (Byrd et al., N Engl J Med. 2013 Jul 4;369(1):32-42.), but never seen before for CSF-1R targeting agents.

In a further embodiment, the invention relates to the use of an antibody which binds to CSF-1R for inducing lymphocytosis of leukemic cells in lymphomas or leukemias.

The invention further relates to the antibody, use or method as described herein before, wherein the lymphocytosis increases the percentage of CD19 expressing and/or CD20 expressing circulating leukemic cells in the peripheral blood. In a further embodiment, the invention relates the antibody, use or method as described herein, wherein the lymphocytosis increases the percentage of CD19 expressing and/or CD20 expressing circulating leukemic cells in the peripheral blood and renders the lymphoma or leukemia susceptible to a treatment with an anti-CD 19 antibody and /or an anti-CD20 antibody.

The invention also relates to the antibody, use or method according to any of the preceding embodiments, wherein the lymphocytosis increases the circulating leukemic cells expressing CD20, in particular, wherein the lymphocytosis increases the percentage of CD20 expressing circulating leukemic cells and renders the lymphoma or leukemia susceptible to a treatment with an anti-CD20 antibody.

In another embodiment, the invention relates to a combination therapy, encompassing the co-administration of an antibody that binds to CSF-1R with an antibody that binds to CD20. The combination therapy is applicable in
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity. In one embodiment of the invention, the stimulation of the cell mediated immune response occurs during the treatment of cancer.

In one embodiment, stimulating the cell mediated immune response as indicated under d) encompasses stimulating the activity of cytotoxic T-lymphocytes, or stimulating the activity of macrophages.

It has been shown that applying said combination therapy was potent to improve CSF-1R based cancer therapy in treated individuals suffering from cancer. The inventors of the present invention have found that CSF-1R antibodies enhance the efficacy of CD20 antibodies to treat cancers, delay progression of a tumor, or prolonging the survival of a patient afflicted with cancer e.g. with lymphoma or leukemia. The delay of cancer progression, as well as the longer overall survival represent a major benefit for patients.

### Antibody for use

One aspect of the invention relates to an antibody that binds to CSF-1R, or an antibody that binds to CSF-1R for use in a combination therapy according to the invention.

### anti-CSF-1R antibody for use

In one embodiment the invention relates to an antibody that binds to CSF-1R, wherein the antibody is administered in a combination therapy with an antibody that binds to CD20, for use in
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity.

In one embodiment the invention relates to an antibody that binds to CSF-1R, wherein the antibody is administered in a combination therapy with an antibody that binds to CD20, for use in
a) treating cancer,
b) delaying progression of cancer, or
c) prolonging the survival of a patient suffering from cancer.

In one embodiment the invention relates to an antibody that binds to CSF-1R, wherein the antibody is administered in a combination therapy with an antibody that binds to CD20, for use in treating cancer. In one embodiment the invention relates to an antibody that binds to CSF-1R, wherein the antibody is administered in a combination therapy with an antibody that binds to CD20, for use in prolonging the survival of a patient suffering from cancer.

### anti-CD20 antibody for use

In one embodiment the invention relates to an antibody that binds to CD20, wherein the antibody is administered in a combination therapy with an antibody that binds to CSF-1R, for use in
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity. In one embodiment of the invention, the stimulation of the cell mediated immune response occurs during the treatment of cancer.

In one embodiment the invention relates to an antibody that binds to CD20, wherein the antibody is administered in a combination therapy with an antibody that binds to CSF-1R, for use in
a) treating cancer,
b) delaying progression of cancer, or
c) prolonging the survival of a patient suffering from cancer.

In one embodiment the invention relates to an antibody that binds to CD20, wherein the antibody is administered in a combination therapy with an antibody that binds to CSF-1R, for use in treating cancer. In one embodiment the invention relates to an antibody that binds to CD20, wherein the antibody is administered in a combination therapy with an antibody that binds to CSF-1R, for use in prolonging the survival of a patient suffering from cancer.

### Use of antibody for manufacture of a medicament

One aspect of the invention relates to an antibody that binds to CSF-1R, or an antibody that binds to CD20 for use in the manufacture of a medicament for the use in a combination therapy according to the invention.

### Use of anti-CSF-1R antibody

In one embodiment the invention relates to the use of an antibody that binds to CSF-1R for the manufacture of a medicament for
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity,
wherein the antibody is for administration in a combination therapy with an antibody that binds to CD20.

In one embodiment the invention relates to the use of an antibody that binds to CSF-1R for the manufacture of a medicament for
a) treating cancer,
b) delaying progression of cancer, or
c) prolonging the survival of a patient suffering from cancer,
wherein the antibody is for administration in a combination therapy with an antibody that binds to CD20.

In one embodiment the invention relates to the use of an antibody that binds to CSF-1R for the manufacture of a medicament for treating cancer, wherein the antibody is for administration in a combination therapy with an antibody that binds to CD20. In one embodiment the invention relates to the use of an antibody that binds to CSF-1R for the manufacture of a medicament for prolonging the survival of a patient suffering from cancer, wherein the antibody is for administration in a combination therapy with an antibody that binds to CD20.

### Use of anti-CD20 antibody

In one embodiment the invention relates to the use of an antibody that binds to CD20 for the manufacture of a medicament for
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity,
wherein the antibody is for administration in a combination therapy with an antibody that binds to CSF-1R.

In one embodiment the invention relates to the use of an antibody that binds to CD20 for the manufacture of a medicament for
a) treating cancer,
b) delaying progression of cancer, or
c) prolonging the survival of a patient suffering from cancer,
wherein the antibody is for administration in a combination therapy with an antibody that binds to CSF-1R.

In one embodiment the invention relates to the use of an antibody that binds to CD20 for the manufacture of a medicament for treating cancer, wherein the antibody is for administration in a combination therapy with an antibody that binds to CSF-1R. In one embodiment the invention relates to the use of an antibody that binds to CD20 for the manufacture of a medicament for prolonging the survival of a patient suffering from cancer, wherein the antibody is for administration in a combination therapy with an antibody that binds to CSF-1R.

### Use of an anti-CSF-1R antibody and an anti-CD20 antibody

In one embodiment the invention relates to the use of an antibody that binds to CSF-1R and an antibody that binds to CD20 for the manufacture of a medicament for
a) treating cancer,
b) delaying progression of cancer,
c) prolonging the survival of a patient suffering from cancer, or
d) stimulating a cell mediated immune response, particularly stimulating cytotoxic T-lymphocytes, stimulating T cell activity, or stimulating macrophage activity.

In one embodiment the invention relates to the use of an antibody that binds to CSF-1R and an antibody that binds to CD20 for the manufacture of a medicament for
a) treating cancer,
b) delaying progression of cancer, or
c) prolonging the survival of a patient suffering from cancer.

In one embodiment the invention relates to the use of an antibody that binds to CSF-1R and an antibody that binds to CD20 for the manufacture of a medicament for treating cancer, wherein the antibody is for administration in a combination therapy with an antibody that binds to CD20. In one embodiment the invention relates to the use of an antibody that binds to CSF-1R and an antibody that binds to CD20 for the manufacture of a medicament for prolonging the survival of a patient suffering from cancer, wherein the antibody is for administration in a combination therapy with an antibody that binds to CD20.

### Article of manufacture

One aspect of the invention relates to an article of manufacture comprising a container, a composition within the container comprising an antibody that binds to CSF-1R, or an antibody that binds to CD20, and a package insert instructing the user of the composition to administer the respective antibody in a combination therapy according to the invention.

### Article of manufacture comprising an anti-CSF-1R antibody

In one embodiment the invention relates to an article of manufacture comprising (a) a container, a composition within the container comprising an antibody that binds to CSF-1R, and (b) a package insert instructing the user of the composition to administer the antibody that binds to CSF-1R to a patient
a) in the treatment of cancer,
b) to delay progression of cancer,
c) to prolong the survival of a patient suffering from cancer, or
d) to stimulate a cell mediated immune response, particularly stimulating cytotoxic T-lymphocytes, stimulating T cell activity, or stimulating macrophage activity
wherein the administration of the antibody that binds to CSF-1R is in a combination therapy with an antibody that binds to CD20.

In one embodiment the invention relates to an article of manufacture comprising (a) a container, a composition within the container comprising an antibody that binds to CSF-1R, and (b) a package insert instructing the user of the composition to administer the antibody that binds to CD20 to a patient
a) in the treatment of cancer,
b) to delay progression of cancer, or
c) to prolong the survival of a patient suffering from cancer,
wherein the administration of the antibody that binds to CSF-1R is in a combination therapy with an antibody that binds to CD20.

In one embodiment the invention relates to an article of manufacture comprising (a) a container, a composition within the container comprising an antibody that binds to CSF-1R, and (b) a package insert instructing the user of the composition to administer the antibody that binds to CSF-1R to a patient in the treatment of cancer, wherein the administration of the antibody that binds to CSF-1R is in a combination therapy with an antibody that binds to CD20. In one embodiment the invention relates to an article of manufacture comprising (a) a container, a composition within the container comprising an antibody that binds to CSF-1R, and (b) a package insert instructing the user of the composition to administer the antibody that binds to CSF-1R to a patient to prolong the survival of a patient suffering from cancer, wherein the administration of the antibody that binds to CSF-1R is in a combination therapy with an antibody that binds to CD20.

### Article of manufacture comprising an anti-CD20 antibody

In one embodiment the invention relates to an article of manufacture comprising (a) a container, a composition within the container comprising an antibody that binds to CD20, and (b) a package insert instructing the user of the composition to administer the antibody that binds to CD20 to a patient
a) in the treatment of cancer,
b) to delay progression of cancer,
c) to prolong the survival of a patient suffering from cancer, or
d) to stimulate a cell mediated immune response, particularly stimulating cytotoxic T-lymphocytes, stimulating T cell activity, or stimulating macrophage activity,
wherein the administration of the antibody that binds to CD20 is in a combination therapy with an antibody that binds to CSF-1R.

In one embodiment the invention relates to an article of manufacture comprising (a) a container, a composition within the container comprising an antibody that binds to CD20, and (b) a package insert instructing the user of the composition to administer the antibody that binds to CD20 to a patient
a) in the treatment of cancer,
b) to delay progression of cancer, or
c) to prolong the survival of a patient suffering from cancer,
wherein the administration of the antibody that binds to CD20 is in a combination therapy with an antibody that binds to CSF-1R.

In one embodiment the invention relates to an article of manufacture comprising (a) a container, a composition within the container comprising an antibody that binds to CD20, and (b) a package insert instructing the user of the composition to administer the antibody that binds to CD20 to a patient in the treatment of cancer, wherein the administration of the antibody that binds to CD20 is in a combination therapy with an antibody that binds to CSF-1R. In one embodiment the invention relates to an article of manufacture comprising (a) a container, a composition within the container comprising an antibody that binds to CD20, and (b) a package insert instructing the user of the composition to administer the antibody that binds to CD20 to a patient to prolong the survival of a patient suffering from cancer, wherein the administration of the antibody that binds to PD-1 is in a combination therapy with an antibody that binds to CSF-1R.

### Article of manufacture comprising an anti-CSF-1R antibody and an anti-CD20 antibody

In one embodiment the invention relates to an article of manufacture comprising (a) a container, a composition within the container comprising an antibody that binds to CSF-1R, and (b) a container, a composition within the container comprising an antibody that binds to CD20, and (c) a package insert instructing the user of the composition to administer the antibody that binds to CSF-1R and the antibody that binds to CD20 to a patient
a) in the treatment of cancer,
b) to delay progression of cancer,
c) to prolong the survival of a patient suffering from cancer, or
d) to stimulate a cell mediated immune response, particularly stimulating cytotoxic T-lymphocytes, stimulating T cell activity, or stimulating macrophage activity.

### Pharmaceutical composition

In one embodiment the invention relates to a pharmaceutical composition, comprising an antibody that binds to CSF-1R, and an antibody that binds to CD20, wherein each antibody is formulated together with a pharmaceutically acceptable carrier.

In one embodiment of said pharmaceutical composition, the antibody that binds to CSF-1R and the antibody that binds to CD20are formulated separately. In another embodiment of said pharmaceutical composition, the antibody that binds to CSF-1R and the antibody that binds to CD20 are formulated together.

### Method for the manufacture of a pharmaceutical composition

In one embodiment the invention relates to a method for the manufacture of a pharmaceutical composition, comprising (a) formulating an antibody that binds to CSF-1R with a pharmaceutically acceptable carrier, and (b) formulating separately an antibody that binds to CD20with a pharmaceutically acceptable carrier.

In one embodiment the invention relates to a method for the manufacture of a pharmaceutical composition, comprising formulating an antibody that binds to CSF-1R together with an antibody that binds to CD20 in combination with a pharmaceutically acceptable carrier.

### Specific antibodies used in the above embodiments

In one embodiment the antibody which binds to human CSF-1R used in the combination therapy described herein is selected from the group consisting of hMab 2F11-c11, hMab 2F11-d8, hMab 2F11-e7 , hMab 2F11-f12, and hMab 2F11-g1.

These antibodies are described in WO2011/070024 and are characterized in comprising the following VH and VL sequences as described herein:

**Table 2:**

| anti-CSF-1R antibody | amino acid sequence of the heavy chain variable domain VH, SEQ ID NO: | amino acid sequence of the light chain variable domain VL, SEQ ID NO: |
|---|---|---|
| hMab 2F11-c11 | 23 | 24 |
| hMab 2F11-d8 | 31 | 32 |
| hMab 2F11-e7 | 39 | 40 |
| hMab 2F11-f12 | 47 | 48 |
| hMab 2F11-g1 | 55 | 56 |

In one preferred embodiment the antibody which binds to human CSF-1R used in the combination therapy described herein is hMab 2F11-e7 (VH, SEQ ID NO:39; VL, SEQ ID NO:40)

In one embodiment the antibody which binds to human CD20 used in the combination therapy described herein is a humanized B-Ly1 antibody:
The term "humanized B-Ly1 antibody" refers to humanized B-Ly1 antibody as disclosed in WO 2005/044859 and WO 2007/031875, which were obtained from the murine monoclonal anti-CD20 antibody B-Ly1 (see Poppema, S. and Visser, L., Biotest Bulletin 3 (1987) 131-139) by chimerization with a human constant domain from IgG1 and following humanization (see WO 2005/044859 and WO 2007/031875). These "humanized B-Ly1 antibodies" are disclosed in detail in WO 2005/044859 and WO 2007/031875.

In one embodiment, the "humanized B-Ly1 antibody" has variable region of the heavy chain (VH) selected from group of SEQ ID No.69 to SEQ ID No.75 (B-HH2, BHH-3, B-HH6, B-HH8, B-HL8, B-HL11 and B-HL13 of WO 2005/044859 and WO 2007/031875). In one embodiment, the "humanized B-Ly1 antibody" has variable region of the light chain (VL) of SEQ ID No. 76 (B-KV1 of WO 2005/044859 and WO 2007/031875).

These antibodies are characterized in comprising the following VH and VL sequences as described herein:

**Table 3:**

| anti-CD20 antibody IgG1 VH/VL | amino acid sequence of the heavy chain variable domain VH, SEQ ID NO: | amino acid sequence of the light chain variable domain VL, SEQ ID NO: |
|---|---|---|
| B-HH2 / B-KV1 | 69 | 76 |
| B-HH3/ B-KV 1 | 70 | 76 |
| B-HH6/ B-KV 1 | 71 | 76 |
| B-HH8/ B-KV 1 | 72 | 76 |
| B-HL8/ B-KV1 | 73 | 76 |
| B-HL11/ B-KV1 | 74 | 76 |
| B-HL13/ B-KV1 | 75 | 76 |

In one preferred embodiment the antibody which binds to human CD20 used in the combination therapy described herein is selected the humanized B-Ly1 antibody B-HH6/B-KV1(comprising a variable region of the heavy chain (VH) of SEQ ID No.71 (B-HH6)and a variable region of the light chain (VL) of SEQ ID No. 76 (B-KV1).

Furthermore in one embodiment, the humanized B-Ly1 antibody is an afucosylated antibody of IgG1 isotype.

According to the invention such afucosylated humanized B-Ly1 antibodies are glycoengineered (GE) in the Fc region according to the procedures described in WO 2005/044859, WO 2004/065540, WO 2007/031875, Umana, P. et al., Nature Biotechnol. 17 (1999) 176-180 and WO 99/154342. In one embodiment, the afucosylated glyco-engineered humanized B-Ly1 is B-HH6-B-KV1 GE. Such glycoengineered humanized B-Ly1 antibodies have an altered pattern of glycosylation in the Fc region, preferably having a reduced level of fucose residues. In one embodiment, the amount of fucose is 60% or less of the total amount of oligosaccharides at Asn297 (in one embodiment the amount of fucose is between 40% and 60%, in another embodiment the amount of fucose is 50% or less, and in still another embodiment the amount of fucose is 30% or less). In another embodiment, the oligosaccharides of the Fc region are preferably bisected. These glycoengineered humanized B-Ly1 antibodies have an increased ADCC.

In one preferred embodiment the antibody which binds to human CD20 used in the combination therapy described herein is selected the humanized B-Ly1 antibody B-HH6/B-KV1(comprising a variable region of the heavy chain (VH) of SEQ ID No.71 (B-HH6)and a variable region of the light chain (VL) of SEQ ID No. 76 (B-KV1) and is an afucosylated antibody of IgG1 isotype having an altered pattern of glycosylation in the Fc region wherein the amount of fucose containing oligosaccharides is between 40% and 60% of the total amount of oligosaccharides at Asn297.

The term "cancer" as used herein may be, for example, lung cancer, non small cell lung (NSCL) cancer, bronchioloalviolar cell lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, gastric cancer, colon cancer, breast cancer, uterine cancer, carcinoma of the fallopian tubes, carcinoma of the endometrium, carcinoma of the cervix, carcinoma of the vagina, carcinoma of the vulva, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, mesothelioma, hepatocellular cancer, biliary cancer, neoplasms of the central nervous system (CNS), spinal axis tumors, brain stem glioma, glioblastoma multiforme, astrocytomas, schwanomas, ependymonas, medulloblastomas, meningiomas, squamous cell carcinomas, pituitary adenoma, lymphoma, lymphocytic leukemia, including refractory versions of any of the above cancers, or a combination of one or more of the above cancers. In one preferred embodiment the cancer refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

In another preferred embodiment the term cancer refers to a "CD20 expressing cancer" in which the cancer cells show an expression of the CD20 antigen. In another preferred embodiment the term "CD20 expressing cancer" as used herein refers to lymphomas (preferably B-Cell Non-Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma) c) marginal zone lymphomas (including extranodal marginal zone B cell lymphoma (Mucosa-associated lymphatic tissue lymphomas, MALT), nodal marginal zone B cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma-Pulmonary B-Cell Lymphoma) f) hairy cell leukemia, g) lymphocytic lymphoma, waldenstrom's macroglobulinemia, h) acute lymphocytic leukemia (ALL), chronic lymphocytic leukemia (CLL)/ small lymphocytic lymphoma (SLL), B-cell prolymphocytic leukemia, i) plasma cell neoplasms, plasma cell myeloma, multiple myeloma, plasmacytoma j) Hodgkin's disease.

In another preferred embodiment, the CD20 expressing cancer is a B-Cell Non-Hodgkin's lymphomas (NHL). In another embodiment, the CD20 expressing cancer is a Mantle cell lymphoma (MCL), acute lymphocytic leukemia (ALL), chronic lymphocytic leukemia (CLL), diffuse large B-cell lymphoma (DLBCL), Burkitt's lymphoma, hairy cell leukemia, follicular lymphoma, multiple myeloma, marginal zone lymphoma, post transplant lymphoproliferative disorder (PTLD), HIV associated lymphoma, waldenstrom's macroglobulinemia, or primary CNS lymphoma.

In one preferred embodiment the cancer or CD20 expressing cancer is B-Cell Non-Hodgkin's lymphoma (NHL). In another preferred embodiment the cancer or CD20 expressing cancer is chronic lymphocytic leukemia (CLL), follicular lymphoma, multiple myeloma. In another preferred embodiment the cancer or CD20 expressing cancer is Hodgkin's disease

Rheumatoid arthritis, psioratic arthritis and inflammatory arthridities are in itself potential indications for CSF-1R signaling inhibitors in that they consist of a macrophage component and to a varying degree bone destruction (Ritchlin, C.T., et al., J. Clin. Invest. 111 (2003) 821-831). Osteoarthritis and rheumatoid arthritis are inflammatory autoimmune disease caused by the accumulation of macrophages in the connective tissue and infiltration of macrophages into the synovial fluid, which is at least partially mediated by M-CSF. Campbell, I., K., et al., J. Leukoc. Biol. 68 (2000) 144-150, demonstrated that M-CSF is produced by human-joint tissue cells (chondrocytes, synovial fibroblasts) in vitro and is found in synovial fluid of patients with rheumatoid arthritis, suggesting that it contributes to the synovial tissue proliferation and macrophage infiltration which is associated with the pathogenesis of the disease. Inhibition of CSF-1R signaling is likely to control the number of macrophages in the joint and alleviate the pain from the associated bone destruction. In order to minimize adverse effects and to further understand the impact of the CSF-1R signaling in these indications, one method is to specifically inhibit CSF-1R without targeting a myriad other kinases, such as Raf kinase.

Thus another embodiment of the invention are the CSF-1R antibodies being characterized by the above mentioned amino acid sequences and amino acid sequence in combination with an anti-CD20 antibody being characterized by the above mentioned amino acid sequences and amino acid sequence for use in the treatment of rheumatoid arthritis, psioratic arthritis, osteoarthritis, inflammatory arthridities, and inflammation.

The invention comprises the combination therapy with an antibody binding to human CSF-1R being characterized by the above mentioned amino acid sequences and amino acid sequence fragments with an anti-CD20 antibody being characterized by the above mentioned amino acid sequences and amino acid sequence fragments for the treatment of cancer.

The invention comprises the combination therapy with an antibody binding to human CSF-1R being characterized by the above mentioned amino acid sequences and amino acid sequence fragments with an anti-CD20 antibody being characterized by the above mentioned amino acid sequences and amino acid sequence fragments for the prevention or treatment of metastasis.

The invention comprises the combination therapy of antibody binding to human CSF-1R being characterized by the above mentioned amino acid sequences and amino acid sequence fragments with an anti-CD20 antibody being characterized by the above mentioned amino acid sequences and amino acid sequence fragments for treatment of inflammatory diseases.

The invention comprises the combination therapy of antibody binding to human CSF-1R being characterized by the above mentioned amino acid sequences and amino acid sequence fragments with an anti-CD20 antibody being characterized by the above mentioned amino acid sequences and amino acid sequence fragments for use in treating or delaying progression of an immune related disease such as tumor immunity.

The invention comprises the combination therapy of antibody binding to human CSF-1R being characterized by the above mentioned amino acid sequences and amino acid sequence fragments with an anti-CD20 antibody being characterized by the above mentioned amino acid sequences and amino acid sequence fragments for use in stimulating an immune response or function, such as T cell activity.

The invention comprises the use of an antibody characterized in comprising the antibody binding to human CSF-1R being characterized by the above mentioned amino acid sequences and amino acid sequence fragments for the combination treatment of cancer with an anti-CD20 antibody or alternatively for the manufacture of a medicament for the combination treatment of cancer with an anti-CD20 antibody as described herein.

The invention comprises the use of an antibody characterized in comprising the antibody binding to human CSF-1R being characterized by the above mentioned amino acid sequences and amino acid sequence fragments for the prevention or treatment of metastasis in the combination with an anti-CD20 antibody as described herein or alternatively for the manufacture of a medicament for the prevention or treatment of metastasis in the combination with an anti-CD20 antibody as described herein.

The invention comprises the use of an antibody characterized in comprising the antibody binding to human CSF-1R being characterized by the above mentioned amino acid sequences and amino acid sequence fragments for combination treatment of inflammatory diseases with an anti-CD20 antibody as described herein or alternatively for the manufacture of a medicament for the combination treatment of inflammatory diseases with an anti-CD20 antibody as described herein.

The invention comprises the use of an antibody characterized in comprising the antibody binding to human CSF-1R being characterized by the above mentioned amino acid sequences and amino acid sequence fragments for use in treating or delaying progression of an immune related disease such as tumor immunity in combination with an anti-CD20 antibody as described herein or alternatively for the manufacture of a medicament for use in treating or delaying progression of an immune related disease such as tumor immunity in combination with an anti-CD20 antibody as described herein.

The invention comprises the use of an antibody characterized in comprising the antibody binding to human CSF-1R being characterized by the above mentioned amino acid sequences and amino acid sequence fragments for use in stimulating an immune response or function, such as T cell activity in combination with an anti-CD20 antibody as described herein or alternatively for the manufacture of a medicament for use in stimulating an immune response or function, such as T cell activity in combination with an anti-CD20 antibody as described herein.

In one preferred embodiment of the invention the antibody which binds to human CSF-1R used in the above described combination treatments and medical uses of different diseases is characterized in comprising
a heavy chain variable domain VH of SEQ ID NO:39 and a light chain variable domain VL of SEQ ID NO:40, and
wherein the antibody which binds to human CD20 used in such combination treatments is characterized in comprising
a heavy chain variable domain VH of SEQ ID NO:71 and a light chain variable domain VL of SEQ ID NO:76.

The antibodies described herein are preferably produced by recombinant means. Such methods are widely known in the state of the art and comprise protein expression in prokaryotic and eukaryotic cells with subsequent isolation of the antibody polypeptide and usually purification to a pharmaceutically acceptable purity. For the protein expression nucleic acids encoding light and heavy chains or fragments thereof are inserted into expression vectors by standard methods. Expression is performed in appropriate prokaryotic or eukaryotic host cells, such as CHO cells, NS0 cells, SP2/0 cells, HEK293 cells, COS cells, yeast, or E. coli cells, and the antibody is recovered from the cells (from the supernatant or after cells lysis).

Recombinant production of antibodies is well-known in the state of the art and described, for example, in the review articles of Makrides, S.C., Protein Expr. Purif. 17 (1999) 183-202; Geisse, S., et al., Protein Expr. Purif. 8 (1996) 271-282; Kaufman, R.J., Mol. Biotechnol. 16 (2000) 151-161; Werner, R.G., Drug Res. 48 (1998) 870-880.

The antibodies may be present in whole cells, in a cell lysate, or in a partially purified, or substantially pure form. Purification is performed in order to eliminate other cellular components or other contaminants, e.g. other cellular nucleic acids or proteins, by standard techniques, including alkaline/SDS treatment, CsCl banding, column chromatography, agarose gel electrophoresis, and others well known in the art. See Ausubel, F., et al., ed. Current Protocols in Molecular Biology, Greene Publishing and Wiley Interscience, New York (1987).

Expression in NS0 cells is described by, e.g., Barnes, L.M., et al., Cytotechnology 32 (2000) 109-123; Barnes, L.M., et al., Biotech. Bioeng. 73 (2001) 261-270. Transient expression is described by, e.g., Durocher, Y., et al., Nucl. Acids. Res. 30 (2002) E9. Cloning of variable domains is described by Orlandi, R., et al., Proc. Natl. Acad. Sci. USA 86 (1989) 3833-3837; Carter, P., et al., Proc. Natl. Acad. Sci. USA 89 (1992) 4285-4289; Norderhaug, L., et al., J. Immunol. Methods 204 (1997) 77-87. A preferred transient expression system (HEK 293) is described by Schlaeger, E.-J. and Christensen, K., in Cytotechnology 30 (1999) 71-83, and by Schlaeger, E.-J., in J. Immunol. Methods 194 (1996) 191-199.

The heavy and light chain variable domains according to the invention are combined with sequences of promoter, translation initiation, constant region, 3' untranslated region, polyadenylation, and transcription termination to form expression vector constructs. The heavy and light chain expression constructs can be combined into a single vector, co-transfected, serially transfected, or separately transfected into host cells which are then fused to form a single host cell expressing both chains.

The control sequences that are suitable for prokaryotes, for example, include a promoter, optionally an operator sequence, and a ribosome binding site. Eukaryotic cells are known to utilize promoters, enhancers and polyadenylation signals.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading frame. However, enhancers do not have to be contiguous. Linking is accomplished by ligation at convenient restriction sites. If such sites do not exist, the synthetic oligonucleotide adaptors or linkers are used in accordance with conventional practice.

The monoclonal antibodies are suitably separated from the culture medium by conventional immunoglobulin purification procedures such as, for example, protein A-Sepharose, hydroxylapatite chromatography, gel electrophoresis, dialysis, or affinity chromatography. DNA and RNA encoding the monoclonal antibodies are readily isolated and sequenced using conventional procedures. The hybridoma cells can serve as a source of such DNA and RNA. Once isolated, the DNA may be inserted into expression vectors, which are then transfected into host cells such as HEK 293 cells, CHO cells, or myeloma cells that do not otherwise produce immunoglobulin protein, to obtain the synthesis of recombinant monoclonal antibodies in the host cells.

As used herein, the expressions "cell", "cell line", and "cell culture" are used interchangeably and all such designations include progeny. Thus, the words "transformants" and "transformed cells" include the primary subject cell and cultures derived therefrom without regard for the number of transfers. It is also understood that all progeny may not be precisely identical in DNA content, due to deliberate or inadvertent mutations. Variant progeny that have the same function or biological activity as screened for in the originally transformed cell are included.

In another aspect, the present invention provides a composition, e.g. a pharmaceutical composition, containing one or a combination of monoclonal antibodies, or the antigen-binding portion thereof, of the present invention, formulated together with a pharmaceutically acceptable carrier.

As used herein, "pharmaceutically acceptable carrier" includes any and all solvents, dispersion media, coatings, antibacterial and antifungal agents, isotonic and absorption/resorption delaying agents, and the like that are physiologically compatible. Preferably, the carrier is suitable for injection or infusion.

A composition of the present invention can be administered by a variety of methods known in the art. As will be appreciated by the skilled artisan, the route and/or mode of administration will vary depending upon the desired results.

Pharmaceutically acceptable carriers include sterile aqueous solutions or dispersions and sterile powders for the preparation of sterile injectable solutions or dispersion. The use of such media and agents for pharmaceutically active substances is known in the art. In addition to water, the carrier can be, for example, an isotonic buffered saline solution.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient (effective amount). The selected dosage level will depend upon a variety of pharmacokinetic factors including the activity of the particular compositions of the present invention employed, or the ester, salt or amide thereof, the route of administration, the time of administration, the rate of excretion of the particular compound being employed, other drugs, compounds and/or materials used in combination with the particular compositions employed, the age, sex, weight, condition, general health and prior medical history of the patient being treated, and like factors well known in the medical arts.

The term "a method of treating" or its equivalent, when applied to, for example, cancer refers to a procedure or course of action that is designed to reduce or eliminate the number of cancer cells in a patient, or to alleviate the symptoms of a cancer. "A method of treating" cancer or another proliferative disorder does not necessarily mean that the cancer cells or other disorder will, in fact, be eliminated, that the number of cells or disorder will, in fact, be reduced, or that the symptoms of a cancer or other disorder will, in fact, be alleviated. Often, a method of treating cancer will be performed even with a low likelihood of success, but which, given the medical history and estimated survival expectancy of a patient, is nevertheless deemed to induce an overall beneficial course of action.

The terms "administered in combination with" or "co-administration", "coadministering", "combination therapy" or "combination treatment" refer to the administration of the anti-CSF-1R as described herein, and the anti-CD20 antibody as described herein e.g. as separate formulations/applications (or as one single formulation/application). The co-administration can be simultaneous or sequential in either order, wherein preferably there is a time period while both (or all) active agents simultaneously exert their biological activities. Said antibody and said further agent are co-administered either simultaneously or sequentially (e.g. intravenous (i.v.) through a continuous infusion. When both therapeutic agents are co-administered sequentially the dose is administered either on the same day in two separate administrations, or one of the agents is administered on day 1 and the second is co-administered on day 2 to day 7, preferably on day 2 to 4. Thus in one embodiment the term "sequentially" means within 7 days after the dose of the first component, preferably within 4 days after the dose of the first component; and the term "simultaneously" means at the same time. The terms "co-administration" with respect to the maintenance doses of anti-CSF-1R antibody and/or anti-CD20 antibody mean that the maintenance doses can be either co-administered simultaneously, if the treatment cycle is appropriate for both drugs, e.g. every week. Or the further agent is e.g. administered e.g. every first to third day and said antibody is administered every week. Or the maintenance doses are co-administered sequentially, either within one or within several days.

It is self-evident that the antibodies are administered to the patient in a "therapeutically effective amount" (or simply "effective amount") which is the amount of the respective compound or combination that will elicit the biological or medical response of a tissue, system, animal or human that is being sought by the researcher, veterinarian, medical doctor or other clinician.

The amount of co-administration and the timing of co-administration will depend on the type (species, gender, age, weight, etc.) and condition of the patient being treated and the severity of the disease or condition being treated. Said anti-CSF-1R antibody and further agent are suitably co-administered to the patient at one time or over a series of treatments e.g. on the same day or on the day after.

Depending on the type and severity of the disease, about 0.1 mg /kg to 50 mg/kg (e.g. 0.1-20 mg/kg) of said anti-CSF-1R antibody and/or anti-CD20 antibody; is an initial candidate dosage for co-administration of both drugs to the patient The invention comprises the use of the antibodies according to the invention for the treatment of a patient suffering from cancer, especially from colon, lung or pancreas cancer.

Depending on the type and severity of the disease, about 0.1 mg /kg to 50 mg/kg (e.g. 0.1-20 mg/kg) of said anti-CSF-1R antibody and/or anti-CD20 antibody; is an initial candidate dosage for co-administration of both drugs to the patient The invention comprises the use of the antibodies according to the invention for the treatment of a patient suffering from cancer, especially from colon, lung or pancreas cancer.

In addition to the anti-CSF-1R antibody in combination with the anti-CD20 antibody also a chemotherapeutic agents or targeted therapies can be administered.

In one embodiment such additional chemotherapeutic agents, which may be administered with anti-CSF-1R antibody as described herein and the anti-CD20 antibody as described herein , include, but are not limited to, anti-neoplastic agents including alkylating agents including: nitrogen mustards, such as mechlorethamine, cyclophosphamide, ifosfamide, melphalan and chlorambucil; nitrosoureas, such as carmustine (BCNU), lomustine (CCNU), and semustine (methyl-CCNU); Temodal(TM) (temozolamide), ethylenimines/methylmelamine such as thriethylenemelamine (TEM), triethylene, thiophosphoramide (thiotepa), hexamethylmelamine (HMM, altretamine); alkyl sulfonates such as busulfan; triazines such as dacarbazine (DTIC); antimetabolites including folic acid analogs such as methotrexate and trimetrexate, pyrimidine analogs such as 5-fluorouracil (5FU), fluorodeoxyuridine, gemcitabine, cytosine arabinoside (AraC, cytarabine), 5-azacytidine, 2,2'-difluorodeoxycytidine, purine analogs such as 6-merca.rho.topurine, 6-thioguamne, azathioprine, T-deoxycoformycin (pentostatin), erythrohydroxynonyladenine (EHNA), fludarabine phosphate, and 2-chlorodeoxyadenosine (cladribine, 2-CdA); natural products including antimitotic drugs such as paclitaxel, vinca alkaloids including vinblastine (VLB), vincristine, and vinorelbine, taxotere, estramustine, and estramustine phosphate; pipodophylotoxins such as etoposide and teniposide; antibiotics such as actinomycin D, daunomycin (rubidomycin), doxorubicin, mitoxantrone, idarubicin, bleomycins, plicamycin (mithramycin), mitomycin C, and actinomycin; enzymes such as L-asparaginase; biological response modifiers such as interferon-alpha, IL-2, G-CSF and GM-CSF; miscellaneous agents including platinum coordination complexes such as oxaliplatin, cisplatin and carboplatin, anthracenediones such as mitoxantrone, substituted urea such as hydroxyurea, methylhydrazine derivatives including N- methylhydrazine (MIH) and procarbazine, adrenocortical suppressants such as mitotane (o, p-DDD) and aminoglutethimide; hormones and antagonists including adrenocorticosteroid antagonists such as prednisone and equivalents, dexamethasone and aminoglutethimide; Gemzar(TM) (gemcitabine), progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide. Therapies targeting epigenetic mechanism including, but not limited to, histone deacetylase inhibitors, demethylating agents (e.g., Vidaza) and release of transcriptional repression (ATRA) therapies can also be combined with the antigen binding proteins. In one embodiment the chemotherapeutic agent is selected from the group consisting of taxanes (like e.g. paclitaxel (Taxol), docetaxel (Taxotere), modified paclitaxel (e.g., Abraxane and Opaxio), doxorubicin, sunitinib (Sutent), sorafenib (Nexavar), and other multikinase inhibitors, oxaliplatin, cisplatin and carboplatin, etoposide, gemcitabine, and vinblastine. In one embodiment the chemotherapeutic agent is selected from the group consisting of taxanes (like e.g. taxol (paclitaxel), docetaxel (Taxotere), modified paclitaxel (e.g. Abraxane and Opaxio). In one embodiment, the additional chemotherapeutic agent is selected from 5-fluorouracil (5-FU), leucovorin, irinotecan, or oxaliplatin. In one embodiment the chemotherapeutic agent is 5-fluorouracil, leucovorin and irinotecan (FOLFIRI). In one embodiment the chemotherapeutic agent is 5-fluorouracil, and oxaliplatin (FOLFOX).

Specific examples of combination therapies with additional chemotherapeutic agents include, for instance, therapies taxanes (e.g., docetaxel or paclitaxel) or a modified paclitaxel (e.g., Abraxane or Opaxio), doxorubicin), capecitabine and/or bevacizumab (Avastin) for the treatment of breast cancer; therapies with carboplatin, oxaliplatin, cisplatin, paclitaxel, doxorubicin (or modified doxorubicin (Caelyx or Doxil)), or topotecan (Hycamtin) for ovarian cancer, the therapies with a multikinase inhibitor, MKI, (Sutent, Nexavar, or 706) and/or doxorubicin for treatment of kidney cancer; therapies with oxaliplatin, cisplatin and/or radiation for the treatment of squamous cell carcinoma; therapies with taxol and/or carboplatin for the treatment of lung cancer.

Therefore, in one embodiment the additional chemotherapeutic agent is selected from the group of taxanes (docetaxel or paclitaxel or a modified paclitaxel (Abraxane or Opaxio), doxorubicin, capecitabine and/or bevacizumab for the treatment of breast cancer.

In one embodiment the CSF-1R antibody/CD20 antibody combination therapy is no chemotherapeutic agents or targeted therapies are administered.

The invention comprises also a method for the treatment of a patient suffering from such disease.

In addition to the anti-CSF-1R antibody in combination with the anti-CD20 antibody also a targeted therapies can be administered.

In addition to the anti-CSF-1R antibody in combination with the anti-CD20 antibody also an anti-PD-Ll or anti-PDl antibody can be administered.

### PD-1/PD-Ll/PD-L2 pathway:

An important negative co-stimulatory signal regulating T cell activation is provided by programmed death - 1 receptor (PD-1)(CD279), and its ligand binding partners PD-L1 (B7-H1, CD274; SEQ ID NO: 88) andPD-L2 (B7-DC, CD273). Thenegative regulatory role of PD-1 was revealed by PD-1 knock outs (Pdcd1-/-), which are prone to autoimmunity. Nishimura et al., Immunity 11: 141-51 (1999); Nishimura et al., Science 291: 319-22 (2001). PD-1 is related to CD28 and CTLA-4, but lacks the membrane proximal cysteine that allows homodimerization. The cytoplasmic domain of PD-1 contains an immunoreceptor tyrosine-based inhibition motif (ITIM, V/IxYxxL/V). PD-1 only binds to PD-L1 and PD-L2. Freeman et al., J. Exp. Med. 192: 1-9 (2000); Dong et al., Nature Med. 5: 1365-1369 (1999); Latchman et al., Nature Immunol. 2: 261-268 (2001); Tseng et al., J. Exp. Med. 193: 839-846 (2001).

PD-1 can be expressed on T cells, B cells, natural killer T cells, activated monocytes and dendritic cells (DCs). PD-1 is expressed by activated, but not by unstimulated human CD4+ and CD8+ T cells, B cells and myeloid cells. This stands in contrast to the more restricted expression of CD28 and CTLA-4. Nishimura et al., Int. Immunol. 8: 773-80 (1996); Boettler et al., J. Virol. 80: 3532-40 (2006). There are at least 4 variants of PD-1 that have been cloned from activated human T cells, including transcripts lacking (i) exon 2, (ii) exon 3, (iii) exons 2 and 3 or (iv) exons 2 through 4. Nielsen et al., Cell. Immunol. 235: 109-16 (2005). With the exception of PD-1 Δex3, all variants are expressed at similar levels as full length PD-1 in resting peripheral blood mononuclear cells (PBMCs). Expression of all variants is significantly induced upon activation of human T cells with anti-CD3 and anti-CD28. The PD-1 Δex3 variants lacks a transmembrane domain, and resembles soluble CTLA-4, which plays an important role in autoimmunity. Ueda et al., Nature 423: 506-11 (2003). This variant is enriched in the synovial fluid and sera of patients with rheumatoid arthritis. Wan et al., J. Immunol. 177: 8844-50 (2006).

The two PD-1 ligands differ in their expression patterns. PD-L1 is constitutively expressed on mouse T and B cells, CDs, macrophages, mesenchymal stem cells and bone marrow-derived mast cells. Yamazaki et al., J. Immunol. 169: 5538-45 (2002). PD-L1 is expressed on a wide range of nonhematopoietic cells (e.g., cornea, lung, vascular epithelium, liver nonparenchymal cells, mesenchymal stem cells, pancreatic islets, placental synctiotrophoblasts, keratinocytes, etc.) [Keir et al., Annu. Rev. Immunol. 26: 677-704 (2008)], and is upregulated on a number of cell types after activation. Both type I and type II interferons IFN's) upregulate PD-L1. Eppihimer et al., Microcirculation 9: 133-45 (2002); Schreiner et al., J. Neuroimmunol. 155: 172-82 (2004). PD-L1 expression in cell lines is decreased when MyD88, TRAF6 and MEK are inhibited. Liu et al., Blood 110: 296-304 (2007). JAK2 has also been implicated in PD-L1 induction. Lee et al., FEBS Lett. 580: 755-62 (2006); Liu et al., Blood 110: 296-304 (2007). Loss or inhibition of phosphatase and tensin homolog (PTEN), a cellular phosphatase that modified phosphatidylinositol 3-kinase (PI3K) and Akt signaling, increased post-transcriptional PD-L1 expression in cancers. Parsa et al., Nat. Med. 13: 84-88 (2007).

PD-L2 expression is more restricted than PD-L1. PD-L2 is inducibly expressed on DCs, macrophages, and bone marrow-derived mast cells. PD-L2 is also expressed on about half to two-thirds of resting peritoneal B1 cells, but not on conventional B2 B cells. Zhong et al., Eur. J. Immunol. 37: 2405-10 (2007). PD-L2+ B1 cells bind phosphatidylcholine and may be important for innate immune responses against bacterial antigens. Induction of PD-L2 by IFN-gamma is partially dependent upon NF-κB. Liang et al., Eur. J. Immunol. 33: 2706-16 (2003). PD-L2 can also be induced on monocytes and macrophages by GM-CF, IL-4 and IFN-gamma. Yamazaki et al., J. Immunol. 169: 5538-45 (2002); Loke et al., PNAS 100:5336-41 (2003).

PD-1 signaling typically has a greater effect on cytokine production than on cellular proliferation, with significant effects on IFN-gamma, TNF-alpha and IL-2 production. PD-1 mediated inhibitory signaling also depends on the strength of the TCR signaling, with greater inhibition delivered at low levels of TCR stimulation. This reduction can be overcome by costimulation through CD28 [Freeman et al., J. Exp. Med. 192: 1027-34 (2000)] or the presence of IL-2 [Carter et al., Eur. J. Immunol. 32: 634-43 (2002)].

Evidence is mounting that signaling through PD-L1 and PD-L2 may be bidirectional. That is, in addition to modifying TCR or BCR signaling, signaling may also be delivered back to the cells expressing PD-L1 and PD-L2. While treatment of dendritic cells with a naturally human anti-PD-L2 antibody isolated from a patient with Waldenstrom's macroglobulinemia was not found to upregulate MHC II or B7 costimulatory molecules, such cells did produce greater amount of proinflammatory cytokines, particularly TNF-alpha and IL-6, and stimulated T cell proliferation. Nguyen et al., J. Exp. Med. 196: 1393-98 (2002). Treatment of mice with this antibody also (1) enhanced resistance to transplanted b16 melanoma and rapidly induced tumor-specific CTL. Radhakrishnan et al., J. Immunol. 170: 1830-38 (2003); Radhakrishnan et al., Cancer Res. 64: 4965-72 (2004); Heckman et al., Eur. J. Immunol. 37: 1827-35 (2007); (2) blocked development of airway inflammatory disease in a mouse model of allergic asthma. Radhakrishnan et al., J. Immunol. 173: 1360-65 (2004); Radhakrishnan et al., J. Allergy Clin. Immunol. 116: 668-74 (2005).

Further evidence of reverse signaling into dendritic cells ("DC's") results from studies of bone marrow derived DC's cultured with soluble PD-1 (PD-1 EC domain fused to Ig constant region - "s-PD-1"). Kuipers et al., Eur. J. Immunol. 36: 2472-82 (2006). This sPD-1 inhibited DC activation and increased IL-10 production, in a manner reversible through administration of anti-PD-1.

Additionally, several studies show a receptor for PD-L1 or PD-L2 that is independent of PD-1. B7.1 has already been identified as a binding partner for PD-L1. Butte et al., Immunity 27: 111-22 (2007). Chemical crosslinking studies suggest that PD-L1 and B7.1 can interact through their IgV-like domains. B7.1:PD-L1 interactions can induce an inhibitory signal into T cells. Ligation of PD-L1 on CD4+ T cells by B7.1 or ligation of B7.1 on CD4+ T cells by PD-L1 delivers an inhibitory signal. T cells lacking CD28 and CTLA-4 show decreased proliferation and cytokine production when stimulated by anti-CD3 plus B7.1 coated beads. In T cells lacking all the receptors for B7.1 (i.e., CD28, CTLA-4 and PD-L1), T cell proliferation and cytokine production were no longer inhibited by anti-CD3 plus B7.1 coated beads. This indicates that B7.1 acts specifically through PD-L1 on the T-cell in the absence of CD28 and CTLA-4. Similarly, T cells lacking PD-1 showed decreased proliferation and cytokine production when stimulated in the presence of anti-CD3 plus PD-L1 coated beads, demonstrating the inhibitory effect of PD-L1 ligation on B7.1 on T cells. When T cells lacking all known receptors for PD-L1 (i.e., no PD-1 and B7.1), T cell proliferation was no longer impaired by anti-CD3 plus PD-L1 coated beads. Thus, PD-L1 can exert an inhibitory effect on T cells either through B7.1 or PD-1.

The direct interaction between B7.1 and PD-L1 suggests that the current understanding of costimulation is incomplete, and underscores the significance to the expression of these molecules on T cells. Studies of PD-L1-/- T cells indicate that PD-L1 on T cells can downregulate T cell cytokine production. Latchman et al., Proc. Natl. Acad. Sci. USA 101: 10691-96 (2004). Because both PD-L1 and B7.1 are expressed on T cells, B cells, DCs and macrophages, there is the potential for directional interactions between B7.1 and PD-L1 on these cells types. Additionally, PD-L1 on non-hematopoietic cells may interact with B7.1 as well as PD-1 on T cells, raising the question of whether PD-L1 is involved in their regulation. One possible explanation for the inhibitory effect of B7.1:PD-L1 interaction is that T cell PD-L1 may trap or segregate away APC B7.1 from interaction with CD28.

As a result, the antagonism of signaling through PD-L1, including blocking PD-L1 from interacting with either PD-1, B7.1 or both, thereby preventing PD-L1 from sending a negative co-stimulatory signal to T-cells and other antigen presenting cells is likely to enhance immunity in response to infection (e.g., acute and chronic) and tumor immunity. In addition, the anti-PD-Ll antibodies of the present invention, may be combined with antagonists of other components of PD-1:PD-L1 signaling, for example, antagonist anti-PD-1 and anti-PD-L2 antibodies.

Mechanisms of PD-L1/PD-1 mediated CD8 T-cell dysfunction in the context of aging-related immune defects in the Eµ-TCL1 CLL mouse model are described in McClanahan F, et al, .Blood. 2015 May 15. blood-2015-02-626754 (Epub ahead of print- PMID: 25979947). McClanahan F, et al , Blood. 2015 Mar 23.(pii: blood-2015-01-622936) refers to PD-L1 Checkpoint Blockade Prevents Immune Dysfunction and Leukemia Development in a Mouse Model of Chronic Lymphocytic Leukemia.

The term "human PD-L1" refers to the human protein PD-L1 (SEQ ID NO: 97, PD-1 signaling typically). As used herein, "binding to human PD-L1" or "specifically binding to human PD-L1" or "which binds to human PD-L1" or "anti- PD-L1 antibody" refers to an antibody specifically binding to the human PD-L1 antigen with a binding affinity of KD-value of 1.0 x 10⁻⁸ mol/l or lower, in one embodiment of a KD-value of 1.0 x10⁻⁹ mol/l or lower. The binding affinity is determined with a standard binding assay, such as surface plasmon resonance technique (BIAcore®, GE-Healthcare Uppsala, Sweden). Thus an "antibody binding to human PD-L1" as used herein refers to an antibody specifically binding to the human PD-L1 antigen with a binding affinity of KD 1.0 x 10⁻⁸ mol/l or lower (in one embodiment 1.0 x 10⁻⁸ mol/l - 1.0 x 10⁻¹³ mol/l), in on embodiment of a KD 1.0 x10⁻⁹ mol/l or lower (in one embodiment 1.0 x 10⁻⁹ mol/l - 1.0 x 10⁻¹³ mol/l).

In one preferred embodiment , in addition to the anti-CSF-1R antibody in combination with the anti-CD20 antibody also an anti-PD-Ll antibody can be administered.

In one embodiment the anti-PD-Ll antibody (antibody which binds to human PD-L1) used in the combination therapy described herein is selected from the group consisting of:
243.55.S70, 243.55.H1, 243.55.H12, 243.55.H37, 243.55.H70, 243.55.H89, 243.55.S1, 243.55.5, 243.55.8 , 243.55.30, 243.55.34 , 243.55.S37 , 243.55.49 , 243.55.51, 243.55.62 , and 243.55.84.

These antibodies are described in WO 2010/77634 (sequences are shown in Figure 11 of WO 2010/77634) and are characterized in comprising the following VH and VL sequences as described herein:

**Table 4:**

| anti-PD-Ll antibody | amino acid sequence of the heavy chain variable domain VH, SEQ ID NO: | amino acid sequence of the light chain variable domain VL, SEQ ID NO: |
|---|---|---|
| 243.55.S70 | 78 | 81 |
| 243.55.H1 | 79 | 82 |
| 243.55.H12 | 79 | 83 |
| 243.55.H37 | 79 | 84 |
| 243.55.H70 | 79 | 85 |
| 243.55.H89 | 79 | 86 |
| 243.55.S1 | 79 | 87 |
| 243.55.5 | 79 | 88 |
| 243.55.8 | 79 | 89 |
| 243.55.30 | 79 | 90 |
| 243.55.34 | 79 | 91 |
| 243.55.S37 | 79 | 92 |
| 243.55.49 | 79 | 93 |
| 243.55.51 | 79 | 94 |
| 243.55.62 | 79 | 95 |
| 243.55.84 | 80 | 96 |

In one embodiment of the invention, the antibody that binds to PD-L1 that is used in a combination therapy according to the invention comprises variable domain amino acid sequences, selected from the group of:
- variable heavy chain domain VH of SEQ ID NO: 78, and variable light chain domain VL of SEQ ID NO: 81 (corresponding to the VH and VL domains of <PD-L1> "243.55.S70" as disclosed herein);
- variable heavy chain domain VH of SEQ ID NO: 79, and variable light chain domain VL of SEQ ID NO: 82 (corresponding to the VH and VL domains of <PD-L1> "243.55.H1" as disclosed herein);
- variable heavy chain domain VH of SEQ ID NO: 79, and variable light chain domain VL of SEQ ID NO: 83 (corresponding to the VH and VL domains of <PD-L1> "243.55.H12" as disclosed herein);
- variable heavy chain domain VH of SEQ ID NO: 79, and variable light chain domain VL of SEQ ID NO: 84 (corresponding to the VH and VL domains of <PD-L1> "243.55.H37" as disclosed herein);
- variable heavy chain domain VH of SEQ ID NO: 79, and variable light chain domain VL of SEQ ID NO: 85 (corresponding to the VH and VL domains of <PD-L1> "243.55.H70" as disclosed herein);
- variable heavy chain domain VH of SEQ ID NO: 79, and variable light chain domain VL of SEQ ID NO: 86 (corresponding to the VH and VL domains of <PD-L1> "243.55.H89" as disclosed herein);
- variable heavy chain domain VH of SEQ ID NO: 79, and variable light chain domain VL of SEQ ID NO: 87 (corresponding to the VH and VL domains of <PD-L1> "243.55.S1" as disclosed herein);
- variable heavy chain domain VH of SEQ ID NO: 79, and variable light chain domain VL of SEQ ID NO: 88 (corresponding to the VH and VL domains of <PD-L1> "243.55.5" as disclosed herein);
- variable heavy chain domain VH of SEQ ID NO: 79, and variable light chain domain VL of SEQ ID NO: 89 (corresponding to the VH and VL domains of <PD-L1> "243.55.8" as disclosed herein);
- variable heavy chain domain VH of SEQ ID NO: 79, and variable light chain domain VL of SEQ ID NO: 90 (corresponding to the VH and VL domains of <PD-L1> "243.55.30" as disclosed herein);
- variable heavy chain domain VH of SEQ ID NO: 79, and variable light chain domain VL of SEQ ID NO: 91 (corresponding to the VH and VL domains of <PD-L1> "243.55.34" as disclosed herein);
- variable heavy chain domain VH of SEQ ID NO: 79, and variable light chain domain VL of SEQ ID NO: 92 (corresponding to the VH and VL domains of <PD-L1> "243.55.S37" as disclosed herein);
- variable heavy chain domain VH of SEQ ID NO: 79, and variable light chain domain VL of SEQ ID NO: 93 (corresponding to the VH and VL domains of <PD-L1> "243.55.49" as disclosed herein);
- variable heavy chain domain VH of SEQ ID NO: 79, and variable light chain domain VL of SEQ ID NO: 94 (corresponding to the VH and VL domains of <PD-L1> "243.55.51" as disclosed herein);
- variable heavy chain domain VH of SEQ ID NO: 79, and variable light chain domain VL of SEQ ID NO: 95 (corresponding to the VH and VL domains of <PD-L1> "243.55.62" as disclosed herein); and
- variable heavy chain domain VH of SEQ ID NO: 80, and variable light chain domain VL of SEQ ID NO: 96 (corresponding to the VH and VL domains of <PD-L1> "243.55.84" as disclosed herein).

In one preferred embodiment of the invention, the antibody that binds to PD-L1 that is used in a combination therapy according to the invention comprises the following variable domain amino acid sequences: variable heavy chain domain VH of SEQ ID NO: 78, and variable light chain domain VL of SEQ ID NO: 81 (corresponding to the VH and VL domains of <PD-L1> "243.55.S70" as disclosed herein).

In one embodiment the anti-PD-Ll antibody (antibody which binds to human PD-L1) used in the combination therapy described herein is atezolizumab (CAS Number: 1380723-44-3). In one preferred embodiment of the invention, the antibody that binds to PD-L1 that is used in a combination therapy according to the invention comprises the variable domain amino acid sequences of the variable heavy chain domain VH and variable light chain domain VL of atezolizumab (CAS Number: 1380723-44-3).

In one embodiment, in addition to the anti-CSF-1R antibody in combination with the anti-CD20 antibody also an anti-PDl antibody can be administered. In one embodiment of the invention, the antibody that binds to PD-1 that is used in a combination therapy according to the invention binds to human PD-1.

In one embodiment of the invention, the antibody that binds to PD-1 that is used in a combination therapy according to the invention comprises the following variable domain amino acid sequences: variable heavy chain domain VH of SEQ ID NO: 98, and variable light chain domain VL of SEQ ID NO: 99 (corresponding to the VH and VL domains of <PD-1> nivolumab as disclosed herein).

In one embodiment of the invention, the antibody that binds to PD-1 that is used in a combination therapy according to the invention comprises the following variable domain amino acid sequences: variable heavy chain domain VH of SEQ ID NO: 100, and variable light chain domain VL of SEQ ID NO: 101 (corresponding to the VH and VL domains of <PD-1> pembrolizumab as disclosed herein).

In another preferred embodiment in addition to the anti-CSF-1R antibody in combination with the anti-CD20 antibody also ibrutinib can be administered.

Ibrutinib (1-[(3R)-3-[4-Amino-3-(4-phenoxyphenyl)-1H-pyrazolo[3,4-d]pyrimidin-1-yl]piperidin-1-yl]prop-2-en-1-one) (USAN,[1] also known as PCI-32765 and marketed under the name Imbruvica) is an anticancer drug targeting B-cell malignancies.

Therefore in one embodiment in addition to the anti-CSF-1R antibody in combination with the anti-CD20 antibody in the above described methods and medical uses (with the specific CSF1R and CD20 antibodies) also Ibrutinib is administered and/ or used for combination. (triple combo)

The invention further provides a method for the manufacture of a pharmaceutical composition comprising an effective amount of an antibody according to the invention together with a pharmaceutically acceptable carrier and the use of the antibody according to the invention for such a method.

The invention further provides the use of an antibody according to the invention in an effective amount for the manufacture of a pharmaceutical agent, preferably together with a pharmaceutically acceptable carrier, for the treatment of a patient suffering from cancer.

The invention also provides the use of an antibody according to the invention in an effective amount for the manufacture of a pharmaceutical agent, preferably together with a pharmaceutically acceptable carrier, for the treatment of a patient suffering from cancer.

The following examples, sequence listing and figures are provided to aid the understanding of the present invention, the true scope of which is set forth in the appended claims..

**Description of the Sequences**

| | |
|---|---|
| SEQ ID NO: 1 | heavy chain CDR3, Mab 2F11 |
| SEQ ID NO: 2 | heavy chain CDR2, Mab 2F11 |
| SEQ ID NO: 3 | heavy chain CDR1, Mab 2F11 |
| SEQ ID NO: 4 | light chain CDR3, Mab 2F11 |
| SEQ ID NO: 5 | light chain CDR2, Mab 2F11 |
| SEQ ID NO: 6 | light chain CDR1, Mab 2F11 |
| SEQ ID NO: 7 | heavy chain variable domain, Mab 2F11 |
| SEQ ID NO: 8 | light chain variable domain, Mab 2F11 |
| SEQ ID NO: 9 | heavy chain CDR3, Mab 2E10 |
| SEQ ID NO: 10 | heavy chain CDR2, Mab 2E10 |
| SEQ ID NO: 11 | heavy chain CDR1, Mab 2E10 |
| SEQ ID NO: 12 | light chain CDR3, Mab 2E10 |
| SEQ ID NO: 13 | light chain CDR2, Mab 2E10 |
| SEQ ID NO: 14 | light chain CDR1, Mab 2E10 |
| SEQ ID NO: 15 | heavy chain variable domain, Mab 2E10 |
| SEQ ID NO: 16 | light chain variable domain, Mab 2E10 |
| SEQ ID NO: 17 | heavy chain CDR3, hMab 2F11-c11 |
| SEQ ID NO: 18 | heavy chain CDR2, hMab 2F11-c11 |
| SEQ ID NO: 19 | heavy chain CDR1, hMab 2F11-c11 |
| SEQ ID NO: 20 | light chain CDR3, hMab 2F11-c11 |
| SEQ ID NO: 21 | light chain CDR2, hMab 2F11-c11 |
| SEQ ID NO: 22 | light chain CDR1, hMab 2F11-c11 |
| SEQ ID NO: 23 | heavy chain variable domain, hMab 2F11-c11 |
| SEQ ID NO: 24 | light chain variable domain, hMab 2F11-c11 |
| SEQ ID NO: 25 | heavy chain CDR3, hMab 2F11-d8 |
| SEQ ID NO: 26 | heavy chain CDR2, hMab 2F11-d8 |
| SEQ ID NO: 27 | heavy chain CDR1, hMab 2F11-d8 |
| SEQ ID NO: 28 | light chain CDR3, hMab 2F11-d8 |
| SEQ ID NO: 29 | light chain CDR2, hMab 2F11-d8 |
| SEQ ID NO: 30 | light chain CDR1, hMab 2F11-d8 |
| SEQ ID NO: 31 | heavy chain variable domain, hMab 2F11-d8 |
| SEQ ID NO: 32 | light chain variable domain, hMab 2F11-d8 |
| SEQ ID NO: 33 | heavy chain CDR3, hMab 2F11-e7 |
| SEQ ID NO: 34 | heavy chain CDR2, hMab 2F11-e7 |
| SEQ ID NO: 35 | heavy chain CDR1, hMab 2F11-e7 |
| SEQ ID NO: 36 | light chain CDR3, hMab 2F11-e7 |
| SEQ ID NO: 37 | light chain CDR2, hMab 2F11-e7 |
| SEQ ID NO: 38 | light chain CDR1, hMab 2F11-e7 |
| SEQ ID NO: 39 | heavy chain variable domain, hMab 2F11-e7 |
| SEQ ID NO: 40 | light chain variable domain, hMab 2F11-e7 |
| SEQ ID NO: 41 | heavy chain CDR3, hMab 2F11-f12 |
| SEQ ID NO: 42 | heavy chain CDR2, hMab 2F11-f12 |
| SEQ ID NO: 43 | heavy chain CDR1, hMab 2F11-f12 |
| SEQ ID NO: 44 | light chain CDR3, hMab 2F11-f12 |
| SEQ ID NO: 45 | light chain CDR2, hMab 2F11-f12 |
| SEQ ID NO: 46 | light chain CDR1, hMab 2F11-f12 |
| SEQ ID NO: 47 | heavy chain variable domain, hMab 2F11-f12 |
| SEQ ID NO: 48 | light chain variable domain, hMab 2F11-f12 |
| SEQ ID NO: 49 | heavy chain CDR3, hMab 2F11-g1 |
| SEQ ID NO: 50 | heavy chain CDR2, hMab 2F11-g1 |
| SEQ ID NO: 51 | heavy chain CDR1, hMab 2F11-g1 |
| SEQ ID NO: 52 | light chain CDR3, hMab 2F11-g1 |
| SEQ ID NO: 53 | light chain CDR2, hMab 2F11-g1 |
| SEQ ID NO: 54 | light chain CDR1, hMab 2F11-g1 |
| SEQ ID NO: 55 | heavy chain variable domain, hMab 2F11-g1 |
| SEQ ID NO: 56 | light chain variable domain, hMab 2F11-g1 |
| SEQ ID NO: 57 | human kappa light chain constant region |
| SEQ ID NO: 58 | human heavy chain constant region derived from IgG1 |
| SEQ ID NO: 59 | human heavy chain constant region derived from IgG1 mutated on L234A and L235A |
| SEQ ID NO: 60 | human heavy chain constant region derived from IgG4 |
| SEQ ID NO: 61 | human heavy chain constant region derived from IgG4 mutated on S228P |
| SEQ ID NO: 62 | human wildtype CSF-1R (wt CSF-1R) (including signal sequence) |
| SEQ ID NO: 63 | human mutant CSF-1R L301S Y969F (including signal sequence) |
| SEQ ID NO: 64 | human CSF-1R Extracellular Domain (domains D1-D5) |
| SEQ ID NO: 65 | human CSF-1R fragment delD4 |
| SEQ ID NO: 66 | human CSF-1R fragment domains D1-D3 |
| SEQ ID NO: 67 | signal peptide |
| SEQ ID NO: 68 | Primer |
| SEQ ID NO: 69 | heavy chain variable domain, humanized B-Lyl variant B-HH2 |
| SEQ ID NO: 70 | heavy chain variable domain, humanized B-Lyl variant B-HH3 |
| SEQ ID NO: 71 | heavy chain variable domain, humanized B-Lyl variant B-HH6 |
| SEQ ID NO: 72 | heavy chain variable domain, humanized B-Lyl variant B-HH8 |
| SEQ ID NO: 73 | heavy chain variable domain, humanized B-Lyl variant B-HL8 |
| SEQ ID NO: 74 | heavy chain variable domain, humanized B-Lyl variant B-HL11 |
| SEQ ID NO: 75 | heavy chain variable domain, humanized B-Lyl variant B-HL13 |
| SEQ ID NO: 76 | light chain variable domain (VL), humanized B-Ly1 variant B-KV1 |
| SEQ ID NO: 77 | human CD20 |
| SEQ ID NO: 78 | variable heavy chain domain VH of <PD-L1> 243.55 variant 1 |
| SEQ ID NO: 79 | variable heavy chain domain VH of <PD-L1> 243.55 variant 2 |
| SEQ ID NO: 80 | variable heavy chain domain VH of <PD-L1> 243.55 variant 3 |
| SEQ ID NO: 81 | variable light chain domain VL of <PD-L1> 243.55 variant 1 |
| SEQ ID NO: 82 | variable light chain domain VL of <PD-L1> 243.55 variant 2 |
| SEQ ID NO: 83 | variable light chain domain VL of <PD-L1> 243.55 variant 3 |
| SEQ ID NO: 84 | variable light chain domain VL of <PD-L1> 243.55 variant 4 |
| SEQ ID NO: 85 | variable light chain domain VL of <PD-L1> 243.55 variant 5 |
| SEQ ID NO: 86 | variable light chain domain VL of <PD-L1> 243.55 variant 6 |
| SEQ ID NO: 87 | variable light chain domain VL of <PD-L1> 243.55 variant 7 |
| SEQ ID NO: 88 | variable light chain domain VL of <PD-L1> 243.55 variant 8 |
| SEQ ID NO: 89 | variable light chain domain VL of <PD-L1> 243.55 variant 9 |
| SEQ ID NO: 90 | variable light chain domain VL of <PD-L1> 243.55 variant 10 |
| SEQ ID NO: 91 | variable light chain domain VL of <PD-L1> 243.55 variant 11 |
| SEQ ID NO: 92 | variable light chain domain VL of <PD-L1> 243.55 variant 12 |
| SEQ ID NO: 93 | variable light chain domain VL of <PD-L1> 243.55 variant 13 |
| SEQ ID NO: 94 | variable light chain domain VL of <PD-L1> 243.55 variant 14 |
| SEQ ID NO: 95 | variable light chain domain VL of <PD-L1> 243.55 variant 15 |
| SEQ ID NO: 96 | variable light chain domain VL of <PD-L1> 243.55 variant 16 |
| SEQ ID NO: 97 | human programmed death ligand 1 |
| SEQ ID NO: 98 | variable heavy chain domain VH of <PD-1> nivolumab |
| SEQ ID NO: 99 | variable light chain domain VL of <PD-1> nivolumab |
| SEQ ID NO: 100 | variable heavy chain domain VH of <PD-1> pembrolizumab |
| SEQ ID NO: 101 | variable light chain domain VL of <PD-1> pembrolizumab |

### Experimental procedures

### Mice

All mice were housed and bred in a specific pathogen-free animal facility, treated in accordance with the European Union guidelines and with the approval of the San Raffaele Scientific Institute Institutional Ethical Committee. Rag2^{-/-}γ_{c}^{-/-} mice on BALB/c background were kindly provided by CIEA and Taconic, Eµ-TCL1 transgenic mice on a C57BL/6 background were kindly provided by Dr. Byrd and wild-type C57BL/6 mice were supplied by Charles River Laboratories.

### Cells and reagents

Human primary samples were obtained from RAI stage 0-1 CLL patients, after informed consent as approved by the Institutional Ethical Committee (protocol VIVI-CLL) of San Raffaele Scientific Institute (Milan, Italy) in accordance with the Declaration of Helsinki. MEC1 CLL cell line was obtained from Deutsche Sammlung von Mikroorganismen und Zellkulturen (DMSZ) and cultured in RPMI 1640 medium (Invitrogen) with 10% fetal bovine serum and gentamicin (15 µg/mL; Sigma-Aldrich). Clodrolip and phosphate buffer solution (PBS) liposomes were purchased from ClodLip B.V. Anti-mouse CSF1R antibody 2G2 and anti-human CSF1R antibody huMab 2F11-2e 7 were provided by Roche Innovation Center Penzberg, Germany. Anti-human CD20 GA101(glycoengineered humanized B-Lyl antibody BHH6-B-KV1 GE with an amount of fucose between 40 and 60%) was provided by Roche Innovation Center, Schlieren, Switzerland.

### In vivo studies

For xenograft studies, eight-week-old Rag2^{-/-}γ_{c}^{-/-} mice were challenged either i.v. or s.c. with 10x106 MEC1 cells in 0.1 mL of saline through a 27-gauge needle.

Depending on the experiments mice were injected with clodrolip, anti-mouse CSF1R moAb, anti-human CD20 GA101 moAb. For transgenic transplantation studies, C57BL/6 male mice were 16 challenged i.p. (day 0) with 10x106 cells purified from the spleen of leukemic male Eµ- TCL1 transgenic mice. Depending on the experiments, mice were treated with clodrolip or anti-CSFIR moAb at different doses and schedules.

### Murine cell preparations

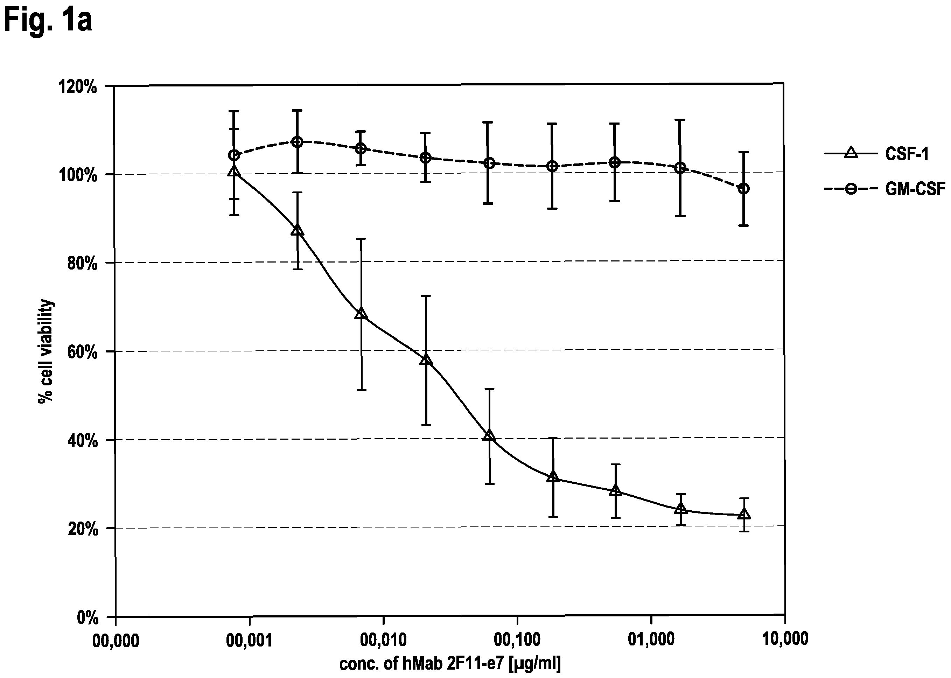

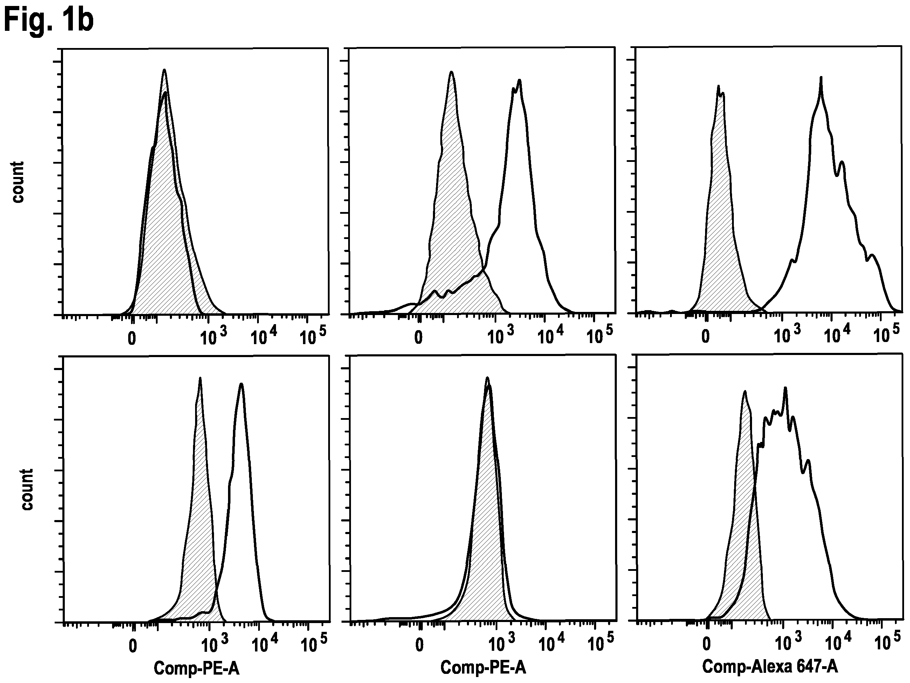

PB, PE, SP, and femurs were collected from mice and cells were isolated. Erythrocytes from BM, PE, SP and PB samples were lysed by incubation for 5 min at room temperature in ammonium chloride solution (ACK) lysis buffer (NH4C10,15 M, KHCO3 10 mM, Na2EDTA 0,1 mM, pH 7,2-7,4). After blocking fragment crystallizable (Fc) receptors with Fc block (BD Biosciences) for 10 minutes at room temperature, cells from PB, BM, PE, SP were stained with the antibodies (15 min. at 4°C) listed below and analyzed with a Beckman Coulter FC500 flow cytometer. For intracellular cytokine detection see below. Absolute numbers were obtained by multiplying the percentage of the cells by the total number of splenocytes, peritoneal cells or BM cells flushed from one femur (except for Figure 7 (Figure 1 of paper)B-C-D-E, where the absolute number refers to the total BM cells flushed from both femurs and tibias).

### Murine cell flow cytometry

Phenotype analysis was performed with the following antibodies: PE-Cy7 or PE Rat Anti-Mouse CD11b (M1/70), APC Rat Anti-Mouse CD5 (53-7.3), PE-Cy7 Rat Anti-Mouse CD19 (1D3), APC or FITC Rat Anti-Mouse CD45 (30-F11), FITC Rat Anti-Mouse CD206 (MR5D3), FITC Hamster Anti-Mouse CD54 (3E2), PE Rat Anti-Mouse CD86 (GL1), FITC or PE-Cy7 Rat Anti-Mouse CD8 (53-6.7), FITC Rat Anti-Mouse CD44 (IM7), PE Rat Anti-Mouse CD62L (MEL-14), PE-Cy7 Rat-Anti-Mouse CD4 (GK1.5), APC Rat-Anti-Mouse CD25 (PC61), FITC Rat Anti-mouse Gr1 (RB6-8C5), FITC Annexin V Apoptosis Detection Kit obtained by BD Biosciences; FITC Rat Anti-Mouse Kappa (187.1), PE Rat Anti-Mouse Lambda (JC5-1) purchased by Beckman Coulter; PE Anti-mouse F4/80 (BM8), FITC Rat Anti-Mouse Ly6C (HK1.4), APC Rat Anti-Mouse Ly6G (1A8) obtained by BioLegend; APC Anti-Mouse CSF1R (AFS98) purchased by eBioscience; PE Rat Anti-Mouse CSF1R (604B5 2E11) purchased by AbD Serotec.

### Intracellular cytokine detection

For IFNγ detection on CD8 T cells, splenocytes were stimulated in vitro with ionomycin and Brefeldin A, and stained with FITC anti-CD44, PE-Cy7 anti-CD8 and APC Anti-Mouse IFNγ (XMG1.2, BD Biosciences). For IL17a and IL2 detection on stromal cells, the cell surface was labeled with anti-CD45, anti-Grl, anti-CD11b, then cells were washed, fixed and permeabilized with Cytofix/Cytoperm-Perm/Wash (BD Biosciences) and labeled with PE Anti-Mouse IL17a (TC11-18H10) or Biotin-Streptavidin-PE Anti-Mouse IL2 (JES6-5H4) purchased by BD Biosciences.

### In vitro cultures and cell-depletion assays from CLL samples

Fresh PBMCs from untreated CLL patients were seeded, as triplicates, at 3x106 cells/ml in culture medium and treated with clodrolip or PBS liposomes (100, 500, 1000 µM) for 30 min and 24h, in presence or absence of etanercept (10µg/ml), a TNF-α antagonist from Pfizer or anti-TRAIL-R2 (human, 1µg/ml) moAb (HS201) from Adipogen AG. For CSF1R inhibition studies, PBMCs from CLL patients were treated 48h with anti-human CSF1R moAb (1-10µg/ml) or with anti-human CSF1R moAb + anti-CD20 moAb GA101 (10µg/ml). The specific percentage of remaining leukemic CD19+CD5+ or CD14+ cells in treated samples was calculated as (absolute number in treated samples/absolute number in control samples) x 100. For each condition, the absolute number of remaining cells was calculated as total viable cell number (trypan blue exclusion determination) x % of viable cells (flow cytometry determination). Then, specific cell depletion was calculated as follow: 100 - % specific remaining cells, as described {Laprevotte, 2013 #46}.

### Gene Expression Profiling analysis

hCD19- cells from murine BM were secondary enriched of monocytes/macrophages by depletion of T, NK, dendritic cells, progenitors, granulocytes and red blood cells through the Easysep negative selection monocyte enrichment kit on EasySep Magnet (StemCell Technologies), following the manufacturer's indications. RNA extraction was performed using RNeasy Mini Kit (QIAGEN). Two hundred nanograms of total RNA samples were processed by Illumina TotalPrep Amplification kit (Ambion-Life technologies) strictly adhering to manufacture protocol. Subsequently, biotin-labeled cRNA was hybridized on MouseWG-6 v2.0 Expression BeadChip (harboring probes for ∼45,000 transcript) or HumanHT-12 v4 BeadChip (harboring probes for ∼47,000 transcript) (Illumina®) for 16 hours, followed by Cy3 staining. Following hybridization, the BeadChip underwent a washing and streptavidin-Cy3 (GE Healthcare Bio-Sciences) conjugate staining protocol. Once the BeadChip was processed it was scanned with an Illumina BeadArray Reader. In brief, arrays were scanned on a BeadScan instrument, and fluorescence intensities were extracted and summarized using the BeadStudio software (Illumina) resulting in a set of summarized fluorescence measurements.

### Statistical analysis

Statistical analyses were performed with the use of the Student test. Data were expressed as the mean value ± SD, and comparison of growth curves was considered statistically significant for P less than .05. Comparison of survival curves was performed with the use of the log-rank test.

### Mice genotyping

Eµ-TCL1 transgenic mice were genotyped for hTCL1 transgene by PCR-based screening assay as previously described {Bertilaccio, 2011 #1}.

### Xenograft studies

Eight-week-old Rag2-/-γc-/- mice male mice were challenged i.v. (day 0) with 10x106 MEC1 cells in 0.1 mL of saline through a 27-gauge needle, as described (Bertilaccio MTS, Blood, 2010). In some experiments, mice were i.v. injected with clodrolip (200 µl) every 3 days, starting at day -1 of the leukemic challenge or with PBS liposomes (200 µl), as control. Depending on the experiments, mice were monitored once a week for weight and killed at early stage of leukemia (days 18-21, 7 clodrolip injections) or at late stage of leukemia (day 28-31, 5 clodrolip injections). PE, PB, SP and femoral BM were analyzed. In selected experiments, BM cells were flushed from mice femurs for magnetic separation of leukemic cells and monocytes/macrophages. In preclinical experiments Rag2-/-γc-/- mice were challenged s.c. (day 0) in the left flank with 10x106 MEC1 cells in 0.1 mL of saline. Once mice bearing subcutaneous tumor developed a palpable tumor draining axillary LN (TDAL), they were s.c. injected with clodrolip (60 µl) at the TDAL site (day +39, +45, +52, +56, +60, +63, +66) or with PBS liposomes (60 µl), as control. Mice were monitored once a week for weight, tumor and LN growth (measuring three perpendicular diameters by a caliper) and killed when the mean LN volume reached 1000 mm3 or larger before reaching clinical signs and symptoms, to avoid unnecessary pain and discomfort according to the ethical guidelines. For pre-clinical purpose, Rag2-/-γc-/- mice transplanted i.v. (day 0) with 10x106 MEC1 cells, were also i.v. injected with clodrolip (60 µl) starting at day 11 of the leukemic challenge and sacrificed at day 26-32 (after bi-weekly clodrolip injection, two weeks treatment; after two clodrolip injections, every other week) or monitored for survival (after two clodrolip injections, every other week). For in vivo CSF1R inhibition studies, xeno-transplanted mice were i.v. injected with anti- CSF1R moAb at days +11, +25 and sacrificed at day 27-29. For survival experiment xenotransplanted mice were i.v. injected with anti-CSFIR or GA101 moAbs (monoclonal antibodies) at days +11, +25 as single agents or in combination setting. For functional studies xeno-transplanted mice, either injected i.v. with clodrolip or anti- CSF1R moAb (at days +11, +25), were repeatedly administered i.p. with etanercept (Pfizer, 10mg/Kg) and killed at day 26 or 29, respectively. In one experiment xenotransplanted mice were pre-treated starting from day -1 every 3/4 days with 2mg/Kg of anti-ICAM1 blocking moAb (YN1/1.7.4, Biolegend) and killed at day +19. Transgenic studies Eight-week-old syngeneic immunocompetent C57BL/6 male mice were challenged i.p. (day 0) with 10x106 cells purified from the spleen of leukemic male Eµ-TCL1 transgenic mice by the EasySep mouse B-cell enrichment kit. The purity of transplanted CD19+ CD5+ Igk+ cells was assessed by flow cytometry. Mice were i.p. injected with clodrolip (200 µl) every 3 days, starting at day -1 of the leukemic challenge (day -1, +2, +5, +8, +11, +14, +18) or with PBS liposomes (200 µl), as control. In preclinical studies, transplanted mice were administered with clodrolip (50 µl) starting at day +16 or +17 of the leukemic challenge every 3/4 days or with PBS liposomes (50 µl), as control. Mice were monitored weekly for weight and leukemia development by flow cytometric analysis of the PB samples and killed at day 24-28. PE, PB, and organs (SP, LN, femoral BM) were analyzed. For in vivo CSF1R inhibition studies, transplanted mice were treated i.p. (day +17, +23) with 30mg/Kg of anti-CSFIR moAb and monitored as described above.

### Examples

### Example 1

### Inhibition of CSF-1-induced CSF-1R phosphorylation in NIH3T3-CSF-1R recombinant cells

4.5x10³ NIH 3T3 cells, retrovirally infected with an expression vector for full-length CSF-1R, were cultured in DMEM (PAA Cat. No.E15-011), 2mM L-glutamine (Sigma, Cat.No.G7513, 2mM Sodium pyruvate, 1x nonessential amino acids, 10% FKS (PAA, Cat.No.A15-649) and 100µg/ml PenStrep (Sigma, Cat.No. P4333 [10mg/ml]) until they reached confluency. Thereafter cells were washed with serum-free DMEM media (PAA Cat.No.E15-011) supplemented with sodium selenite [5ng/ml] (Sigma, Cat.No. S9133), transferrin [10µg/ml] (Sigma, Cat.No. T8158), BSA [400µg/ml] (Roche Diagnostics GmbH, Cat.No. 10735078), 4mM L-glutamine (Sigma, Cat.No.G7513), 2mM sodium pyruvate (Gibco, Cat.No. 11360), 1x nonessential amino acids (Gibco, Cat: 11140-035), 2-mercaptoethanol [0,05mM] (Merck, Cat.No. M7522), 100µg/ml and PenStrep (Sigma, Cat. No. P4333) and incubated in 30 µl of the same medium for 16 hours to allow for receptor up-regulation. 10 µl of diluted anti-CSR-1R antibodies were added to the cells for 1.5 h. Then cells were stimulated with 10 µl of 100 ng/ml hu CSF-1 (active 149 aa fragment of human CSF-1 (aa 33-181 of SEQ ID NO: 86) ;Biomol, DE, Cat.No.60530)for 5 min. After the incubation, supernatant was removed, cells were washed twice with 80 µl of ice-cold PBS and 50 µl of freshly prepared ice-cold lysis buffer (150mM NaCl/ 20mM Tris pH 7.5 / 1mM EDTA/ 1mM EGTA/ 1% Triton X-100/1 protease inhibitor tablet (Roche Diagnostics GmbH Cat.No. 1 836 170) per 10 ml buffer/10µl/ml phosphatase inhibitor cocktail 1 (Sigma Cat.No. P-2850, 100x Stock)/10µl/ml protease inhibitor 1 (Sigma Cat.No.P-5726, 100x Stock) 10µl/ml 1 M NaF) was added. After 30 minutes on ice the plates were shaken vigorously on a plateshaker for 3 minutes and then centrifuged 10 minutes at 2200 rpm (Heraeus Megafuge 10).

The presence of phosphorylated and total CSF-1 receptor in the cell lysate was analyzed with Elisa. For detection of the phosphorylated receptor the kit from R&D Systems (Cat. No. DYC3268-2) was used according to the instructions of the supplier. For detection of total CSF-1R 10 µl of the lysate was immobilized on plate by use of the capture antibody contained in the kit. Thereafter 1:750 diluted biotinylated anti CSF-1R antibody BAF329 (R&D Systems) and 1:1000 diluted streptavidin-HRP conjugate was added. After 60 minutes plates were developed with freshly prepared ABTS® solution and the absorbance was detected. Data were calculated as % of positive control without antibody and the ratio value phospho/total receptor expressed. The negative control was defined without addition of M-CSF-1. Anti CSF-1R SC 2-4A5 (Santa Cruz Biotechnology, US, see also Sherr, C.J. et al., Blood 73 (1989) 1786-1793), which inhibits the ligand- receptor interaction, was used as reference control.

**Table 3 :**

| **Calculated IC50 values for the inhibition of CSF-1 receptor phosphorylation.** | |
|---|---|
| **CSF-1R Mab** | **IC50 CSF-1R Phosphorylation [ng/ml]** |
| Mab 2F11 | 219.4 |
| Mab 2E10 | 752.0 |
| Mab 2H7 | 703.4 |
| Mab 1G10 | 56.6 |
| SC-2-4A5 | 1006.6 |

### Example 2

### Growth inhibition of NIH3T3-CSF-1R recombinant cells in 3D culture under treatment with anti-CSF-1R monoclonal antibodies (CellTiterGlo-assay)

NIH 3T3 cells, retrovirally infected with either an expression vector for full-length wildtype CSF-1R (SEQ ID NO: 62) or mutant CSF-1R L301S Y969F (SEQ ID NO: 63), were cultured in DMEM high glucose media (PAA, Pasching, Austria) supplemented with 2mM L-glutamine, 2mM sodium pyruvate and non-essential amino acids and 10% fetal bovine serum (Sigma, Taufkirchen, Germany) on poly-HEMA (poly(2-hydroxyethylmethacrylate)) (Polysciences, Warrington, PA, USA)) coated dishes to prevent adherence to the plastic surface. Cells are seeded in medium replacing serum with 5ng/ml sodium selenite, 10mg/ml transferrin, 400µg/ml BSA and 0.05 mM 2-mercaptoethanol. When treated with 100ng/ml hu CSF-1 (active 149 aa fragment of human CSF-1 (aa 33-181 of SEQ ID NO: 86); Biomol, DE, Cat.No.60530) wtCSF-1R (expressing cells form dense spheroids that grow three dimensionally, a property that is called anchorage independence. These spheroids resemble closely the three dimensional architecture and organization of solid tumors in situ. Mutant CSF-1R recombinant cells are able to form spheroids independent of the CSF-1 ligand. The anti-CSF-1R antibody according to the invention hMab 2F11-e7 and the anti-CSF-1R antibodies 1.2.SM (ligand displacing CSF-1R antibody described in WO 2009/026303), CXIIG6 (ligand displacing CSF-1R antibody described in WO 2009/112245), the goat polyclonal anti-CSF-1R antibody ab10676 (abcam), and SC 2-4A5 (Santa Cruz Biotechnology, US- see also Sherr, C.J. et al., Blood 73 (1989) 1786-1793) and Mab R&D-Systems 3291 were investigated. Reference control Mab R&D-Systems 3291 did not show inhibition of mutant CSF-1R recombinant cell proliferation.

Spheroid cultures were incubated for 3 days in the presence of different concentrations of antibody in order to determine an IC30 (concentration with 30 percent inhibition of cell viability). Maximum concentration was 20 µg/ml The CellTiterGlo assay was used to detect cell viability by measuring the ATP-content of the cells.

**Table 4 :**

| **CSF-1R Mab** | **wtCSF-1R IC₃₀ [µg/ml]** | **Mutant CSF-1R IC₃₀ [µg/ml]** |
|---|---|---|
| hMab 2F11-e7 | 4.91 | 0.54 |
| 1.2.SM | 1.19 | > 20 µg/ml (-19% inhibition at 20 µg/ml = 19% stimulation) |
| CXIIG6 | > 20 µg/ml (21% inhibition at 20 µg/ml) | > 20 µg/ml (-36% inhibition at 20 µg/ml = 36% stimulation) |
| ab10676 | 14.15 | > 20 µg/ml (0% inhibition at 20 µg/ml) |
| SC 2-4A5 | 16.62 | 2.56 |

### Example 3

### Inhibition of human macrophage differentiation under treatment with anti-CSF-1R monoclonal antibodies (CellTiterGlo-assay)

Human monocytes were isolated from peripheral blood using the RosetteSep™ Human Monocyte Enrichment Cocktail (StemCell Tech. - Cat. No. 15028). Enriched monocyte populations were seeded into 96 well microtiterplates (2.5x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 150 ng/ml huCSF-1 was added to the medium, a clear differentiation into adherent macrophages could be observed. This differentiation could be inhibited by addition of anti-CSF-1R antibodies. Furthermore, the monocyte survival is affected and could be analyzed by CellTiterGlo (CTG) analysis. From the concentration dependent inhibition of the survival of monocytes by antibody treatment, an IC₅₀ was calculated (see Table below).

**Table 5 :**

| **CSF-1R Mab** | **IC₅₀ [µg/ml]** |
|---|---|
| Mab 2F11 | 0.08 |

In a separate test series humanized versions of Mab 2 F11, e.g. hMab 2F11-c11, hMab 2F11-d8, hMab 2F11-e7, hMab 2F11-f12, showed IC50 values of 0.07 µg/ml (hMab 2F11-c11), 0.07 µg/ml (hMab 2F11-d8), 0.04 µg/ml (hMab 2F11-e7) and 0.09 µg/ml (hMab 2F11-f12).

### Example 4

### Inhibition of human macrophage differentiation under treatment with anti-CSF-1R monoclonal antibodies (CellTiterGlo-assay)

Human monocytes were isolated from peripheral blood using the RosetteSep™ Human Monocyte Enrichment Cocktail (StemCell Tech. - Cat. No. 15028). Enriched monocyte populations were seeded into 96 well microtiterplates (2.5x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 150 ng/ml huCSF-1 was added to the medium, a clear differentiation into adherent macrophages could be observed. This differentiation could be inhibited by addition of anti-CSF-1R antibodies. Furthermore, the monocyte survival is affected and could be analyzed by CellTiterGlo (CTG) analysis. From the concentration dependent inhibition of the survival of monocytes by antibody treatment, an IC₅₀ was calculated. Humanized versions of Mab 2 F11, e.g. hMab 2F11-c11, hMab 2F11-d8, hMab 2F11-e7, hMab 2F11-f12, showed IC50 values of 0.07 µg/ml (hMab 2F11-c11), 0.07 µg/ml (hMab 2F11-d8), 0.04 µg/ml (hMab 2F11-e7) and 0.09 µg/ml (hMab 2F11-f12).

### Example 5

### Inhibition of human M1 and M2 macrophage differentiation under treatment with anti-CSF-1R monoclonal antibodies (CellTiterGlo-assay)

Human monocytes were isolated from peripheral blood using the RosetteSep™ Human Monocyte Enrichment Cocktail (StemCell Tech. - Cat. No. 15028). Enriched monocyte populations were seeded into 96 well microtiterplates (2.5x10⁴ cells/well) in 100 µl RPMI 1640 (Gibco - Cat. No.31870) supplemented with 10% FCS (GIBCO - Cat. No.011-090014M), 4 mM L-glutamine (GIBCO - Cat. No.25030) and 1x PenStrep (Roche Cat. No.1 074 440) at 37°C and 5% CO₂ in a humidified atmosphere. When 100 ng/ml huCSF-1 was added for 6 days to the medium, a clear differentiation into adherent, M2 macrophages with elongated morphology could be observed. When 100 ng/ml huGM-CSF was added to the medium for 6 days, a clear differentiation into adherent, M1 macrophages with round morphology could be observed. This differentiation was associated with the expression of certain markers such as CD163 for M2 macrophages and CD80 or high MHC class II for M1 macrophages as assessed by flow cytometry. Cells were washed with PBS and, if adherent, detached using a 5mM EDTA solution in PBS (20min at 37°C). Cells were then well resuspended, washed with staining buffer (5% FCS in PBS) and centrifuged at 300xg for 5min. Pellets were resuspended in 1ml staining buffer and cells counted in a Neubauer chamber. Approximately 1x10e5 cells were transferred in each FACS tube, centrifuged at 300xg for 5min and resuspended in staining buffer. Fcγ receptors were blocked by incubation with 1µg human IgG/2,5x10e4 cells (JIR Cat.No.009-000-003) in staining buffer for 20 min on ice. Cells were then mixed with 1,5µl antibody/2,5x10e4 cells for CD80 and CD163 detection whereas 5 µl antibody/2,5x10e4 cells for MHC class II detection was used: PE labeled mouse anti human CD163 (BD Bioscience Cat.No.556018), PE labeled mouse anti human CD80 (BD Bioscience Cat.No. 557227) and Alexa 647 labeled mouse anti human MHC class II (Dako- Cat.No. M0775). The Alexa 647 label was conjugated to the antibody by using the Zenon Alexa 647 mouse IgG labeling kit (Invitrogen Cat.No. Z25008) After a 1-hour incubation on ice cells were washed twice with staining buffer, resuspended and measured at a FACS Canto II.

Exclusively M2 macrophage differentiation which is characterized by the expression of CD163, absence of CD80 and low MHC class II expression could be inhibited by addition of humanized anti-CSF-1R antibody hMab 2F11-e7. Furthermore, the M2 but not M1 macrophage survival is affected and could be analyzed by CellTiterGlo (CTG) analysis. Concentration dependent inhibition of the survival of macrophages by antibody treatment for 7 days is depicted in Figure 1a. Expression of M1 and M2 macrophage markers assessed by flow cytometry is shown in Figure 1b.

### Example 6

### Relationship between M2 subtype tumor associated macrophages (TAMs) and T cells - Rationale for combining anti-CSF-R1 antibody and a T cell engaging agents

To investigate the functional relationship between TAMs and T cells we isolated TAMs from the MC38 tumor and cocultured them with CD8+ T cells.

### TAM suppression assay

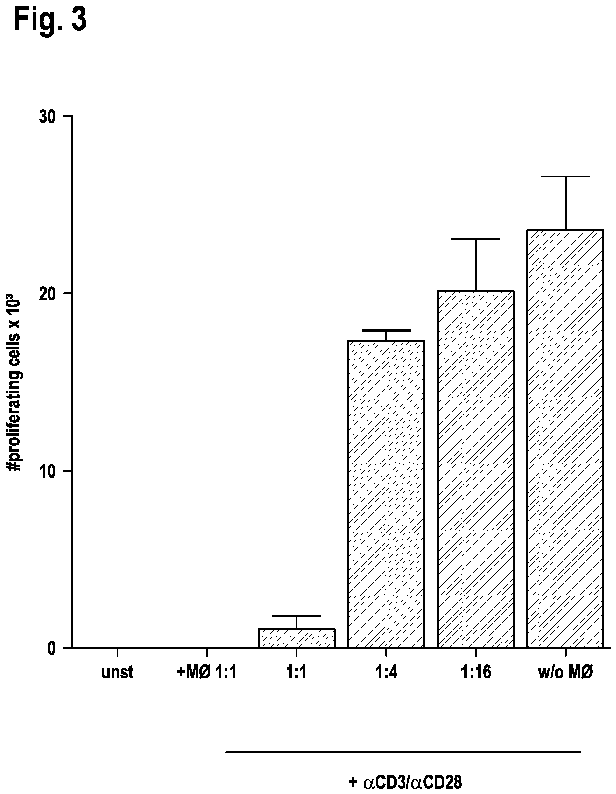

TAMs were enriched from single cell suspensions of MC38 tumors after enzymatic digest using a two-step protocol: Single cells were stained with CD11b-FITC (clone M1/70) and positively enriched over MACS columns by anti-FITC beads (Miltenyi). Upon removal from the column, anti-FITC beads were detached using release buffer protocol as provided the manufacturer. Finally, TAM were isolated by adding anti-Ly6G and anti-Ly6C positive selection beads in order to remove granulocytic and monocytic cells from TAM preparations. Final cell purity was analyzed and was usually > 90%. Subsequently, TAM were titrated in the indicated ratios to total CD3+ T cells labeled with CFSE in U-bottom plates coated with anti-CD3 and soluble anti-CD28 was added. Cell proliferation was determined from CFSElow cells using blank Sphero beads as previously described after 3 days of incubation (Hoves, S. et al. Monocyte-derived human macrophages mediate anergy in allogeneic T cells and induce regulatory T cells. J. Immunol. 177, 2691-2698 (2006)). In the presence of TAMs, T cell expansion induced by activation of CD3 and CD28 was suppressed. (see Figure 3).

### Example 7

### TARGETING MONOCYTES/MACROPHAGES BY CSF-1R INHIBITION CAN BE EXPLOITED AS A THERAPEUTIC STRATEGY IN CLL

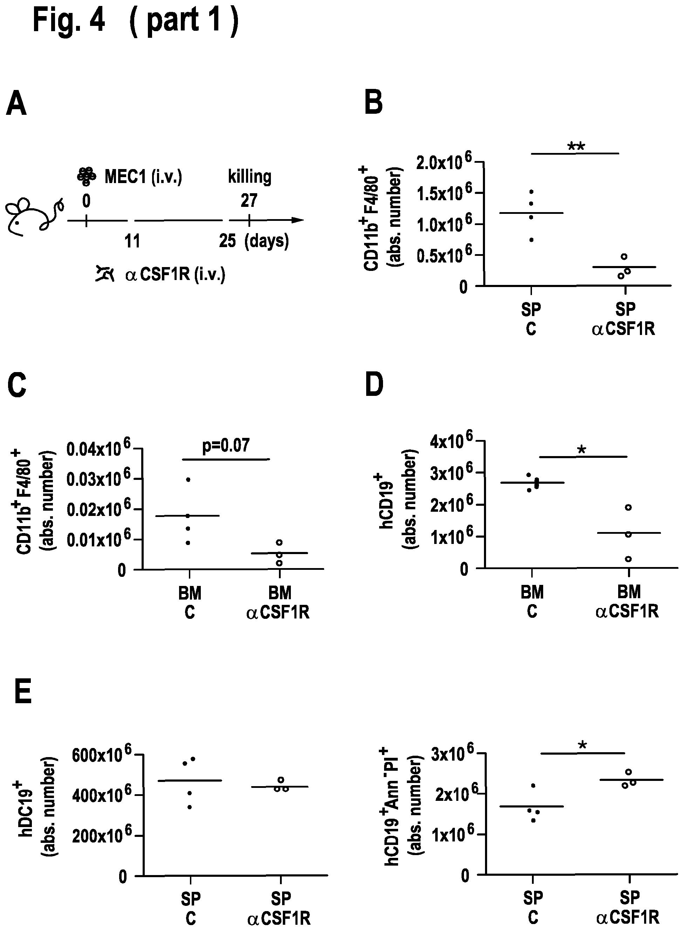

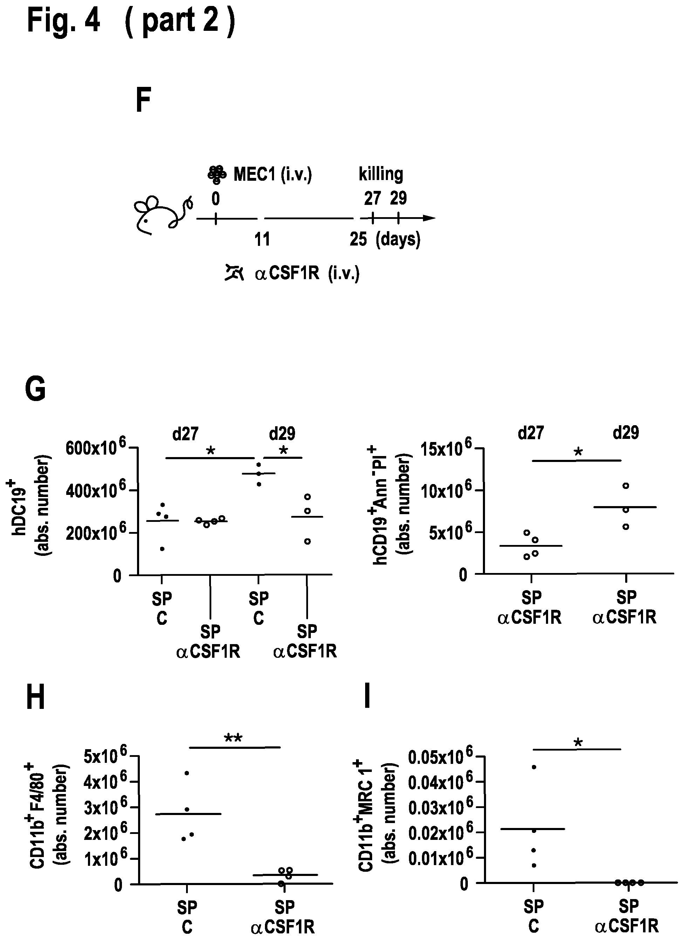

To evaluate the therapeutic potential of macrophage targeting, we investigated the anti-leukemic effects of a monoclonal antibody that inhibits CSF1R signaling and prevents macrophage differentiation from monocyte precursors {Ries, 2014 #16}. Rag2^{-/-}γ_{c}^{-/-} mice transplanted i.v. with MEC1 cells were injected i.v. with the anti-CSF1R moAb (30 mg/kg, days +11, +25) and sacrificed at day 27, 48 hours after the last moAb injection (Figure 4A). Macrophage depletion (Figure 4B-C) significantly reduced the number of leukemic B cells in the BM (Figure 4D) and induced the appearance of CD19+ AnnV-PI+ necrotic leukemic cells in the SP (Figure 4E). To confirm that CSF1R blockade reduces disease severity in the SP, we performed a time course experiment, where mice were sacrificed 48h and 96h after the last moAb injection, at days 27 and 29, respectively (Figure 4F). CSF1R blockade was able to stabilize the disease and induce increasing necrosis of the splenic leukemic cells over time (Figure 4G). This anti-leukemic effect was associated with a remarkable and selective depletion of CSF1R+ MRC1+ M2-like TAMs (Figure 4H-I). As already shown in solid tumors {Ries, 2014 #16}, CSF1R blockade, together with monocyte depletion (Figure 6A-B), induced a relative increase in the PB of SSChigh neutrophil granulocytes (Figure S4C) and Gr1+ myeloid cells (Figure 6D) encompassing both monocytic (Ly6C+) and granulocytic (Ly6G+) subsets (Figure 6E-F). Of note, this effect was not observed with clodrolip (Figure 6G-H-I-J-K). In the BM, CSF1R blockade decreased monocytes, identified as either CD11b+CSF1R+ (Figure 6L) or Ly6C+ (Figure 6M-N). In the Eµ-TCL1 tg transplantation model (Figure 6O), we observed a pronounced increase of CD8+ effector T cells in the PB (Figure 6P), BM (Figure 6Q) and SP (Figure 6R-S), accompanied by a marked increase of apoptotic leukemic cells (Figure 6T) and a decrease of splenic CD4+CD25+ T cells (Figure 6U). Conversely, clodrolip did not impact on T cells (Figure 6V-W-X). Two administrations of the anti-CSFIR moAb (Figure 5A) induced significant lymphocytosis (Figure 5B-C), a treatment-related process already seen in CLL patients with agents targeting BCR signaling (e.g. ibrutinib), not associated to disease progression (Byrd J.C. NEJM 2013). As moAb treatment increased the percentage of CD19+CD20+ circulating leukemic cells (Figure 5C), we tested a two-pronged approach by combining the anti-CSFIR moAb with the glycoengineered type II CD20 moAb, GA101 (Moessner E Blood, 2010) (Figure 5D). Of note, anti-mouse CSF1R moAb impacted on the survival of mice as single agent (p=0.01, αCSF1R moAb vs untreated) and, even more significantly in the combination setting (p<0.0001, αCD20 + αCSF1R moAb vs untreated) (Figure 5E). Together, these findings indicate that CSF1R blockade provides therapeutic benefits in mouse models of CLL. Furthermore, they show that macrophage depletion-associated increase of circulating CD20+ leukemic cells represents a therapeutic opportunity for combination with anti-CD20 antibodies.

### CHARACTERIZATION OF BM MONOCYTES AND MACROPHAGES IN CLL XENOTRANSPLANTED MICE

We asked whether monocytes/macrophages influence CLL growth in the BM. Eightweek-old Rag2^{-/-}γ_{c}^{-/-} mice were injected intravenously (i.v.; day 0) with MEC1 cells (a CLL cell line) {Stacchini, 1999 #50} and killed either in the early (day 21) or overt (day 31) phase of leukemia growth (Figure 7A), when the percentage of human CD19+ leukemic cells in the BM was 33.83 ± 15.03 and 86 ± 9.13, respectively (Figure 7B). We found lower numbers of CD11b+CSF1R+ monocytes and CD11b+F4/80+ macrophages in the BM of frank leukemic Rag2^{-/-}γ_{c}^{-/-} mice when compared to early leukemic mice (Figure 7C-D). Of note, the abundance of CD11b+ cells coexpressing the macrophage mannose receptor (MRC1/CD206), CSF1R and CD86 increased along with leukemia progression (Figure 7E-F), a feature also associated with progression of solid tumors {De Palma, 2013 #48} (Noy & Pollard Immunity 2014).

We next evaluated the expression of chemokines, cytokines and growth factors in the non-leukemic cells of the BM microenvironment of Rag2^{-/-}γ_{c}^{-/-} mice with early leukemia compared to uninjected mice. BM cells of xeno-transplanted mice were depleted of human CD19+ leukemic cells by magnetic beads to perform RNA extraction and qPCR array analysis (RT² Profiler™). As shown in Figure 7G, a distinct inflammatory profile was induced by the presence of leukemic cells. We observed a significant upregulation of cytokines with leukemia-growth-promoting capacity (Yan XJ Blood 2011), such as I16, Cd401g, I12 and I117a. Gr1+ myeloid-derived suppressor cells (MDSCs) were identified as the source of IL-17a (Figure 8A) and non-hematopoietic CD45- cells, as the source of IL-17a (Figure 8B) and IL-2 (Figure 8C) protein production. Also upregulated were I110, a cytokine with immunosuppressive activity; Csf1, which promotes monocyte/macrophage differentiation and survival; and various chemokines/stromal cell chemoattractants, like Ccl1, Ccl3, and Ccl4 {De Palma, 2013 #48} (Noy & Pollard Immunity 2014). Such cytokine/chemokine profile is suggestive of a BM microenvironment conducive to leukemic cell growth.

To specifically characterize the molecular profile of CLL-associated monocytes/macrophages (Figure 7C-D), a whole genome transcriptional profile analysis was performed by Illumina hybridization system on monocytes/macrophages isolated from the BM of Rag2^{-/-}γ_{c}^{-/-} mice xeno-transplanted mice, sacrificed at day 21 (early leukemia).

A complex network of gene regulation involving the modulation of 164 transcripts (84 up- and 80 downregulated; 1.6% of total transcripts; adjusted P-value < 0.05) distinguished monocytes/macrophages of mice with leukemia compared to age-matched, uninjected mice. Differentially expressed genes were organized into five putative functional categories including inflammation and intracellular/extracellular function, shown in Figure 9A-E. In parallel, we also analyzed the transcriptional changes occurring in leukemic MEC1 cells purified from the same mice. Particularly relevant was the differential expression of genes supporting the existence of monocyte/macrophage-leukemic cell cross talk, including IL10, RNASET2 and CCL2 (a potent monocyte chemoattractant (Noy & Pollard Immunity 2014)), besides genes involved in CLL progression, like NOTCH1, BIRC3, and PTEN (Figure 9F). We also confirmed by qPCR the differential expression of a panel of selected genes in both murine monocytes/macrophages (Figure 10A) and human MEC1 cells (Figure 10B). Due to its role in B cell adhesion, antigen presentation and activation (Batista F Nat Rev Immunol 2009), we validated ICAM1 upregulation at the protein level, both in mouse macrophages from spleen (SP), BM or peritoneal exudate (PE) (Figure 9G) and human classical monocytes of CLL patients (Figure 10C). Xeno-transplantation studies using an ICAM1 blocking moAb, administered every 3/4 days from day -1 (Figure 10D), demonstrated that its inhibition increased the number of leukemic cells in the PB, SP and PE, but not in the BM (Figure 10E), thus suggesting a functional relevance of ICAM1 upregulation in different tissues.

Taken together, these findings indicate that CLL cells profoundly sculpt the BM microenvironment and modulate the expression of multiple gene transcripts involved in CLL cell-monocyte/macrophage interaction.

### LEUKEMIC CELL DEATH INDUCED BY MACROPHAGE TARGETING IS TNF DEPENDENT

As a next step we evaluated how leukemic cells were affected by macrophage targeting. Clodrolip and anti-human CSF-1R moAb (hMab 2F11-e7) have no direct toxicity on leukemic B cells in vitro, as shown by cytotoxicity assays performed on MEC1 cells (Figure 11A). We therefore investigated alternative mechanisms whereby clodrolip and anti-CSFIR may induce leukemic cell death in vivo. To address this question, Rag2^{-/-}γ_{c}^{-/-} mice transplanted i.v. with MEC1 cells were macrophage-depleted by i.v. clodrolip injection every 3 days starting at day -1 (7 consecutive injections) and sacrificed 1 day after the last treatment (Figure 12A). The reduction of the leukemic (Figure 12B) and monocyte/macrophage (Figure 12C-D-E) cell burden induced by clodrolip paralleled the induction of apoptosis of leukemic cells in all tissues, as shown by the significantly increased frequency of CD19+ AnnV+PI+ late apoptotic cells in the SP and BM and of CD19+ AnnV-PI+ necrotic cells in the PB (Figure 12F-G). These results confirmed the increase of CD19+ AnnV-PI+ necrotic cells already observed in the SP of anti-CSFIR moAb-treated mice (Figure 4G). The interaction between leukemic B cells and monocytes/macrophages and the induction of leukemic B cells apoptosis upon clodrolip were also visualized in a time-course experiment whereby GFP-labelled monocytes/macrophages were added to leukemic cells from Eµ-TCL1 transgenic mice in the absence or presence of clodrolip. Interestingly, AnnV/PI staining and flow cytometry analysis showed that the induction of leukemic cell apoptosis by clodrolip occurred only in the presence of monocytes and macrophages (Figure 11B).

To investigate the cell death pathways induced in leukemic cells via clodrolip-induced macrophage killing, we purified CD19+ MEC1 cells from the BM of transplanted Rag2^{-/-}γ_{c}^{-/-} mice and evaluated, at the RNA level by qPCR, the expression of key effector molecules involved in TNF, TRAIL, ROS, and FAS/FASL regulated cell death pathways. As for the TNF pathway, we observed upregulated expression of TNFR1, FADD, BID, BAX and CASP3 (Figure S5C). Interestingly we also found upregulated levels of TRAIL-R2 and AIFM1 (Figure S5C), the latter being involved in ROS-mediated dell death {Joza, 2009 #49}.

To confirm the involvement of TNF (Figure 11C) in the mechanism of leukemia cell death induced by macrophage targeting, we utilized etanercept, a soluble TNF receptor fusion protein {Deeg, 2002 #44}, in xeno-transplanted Rag2^{-/-}γ_{c}^{-/-} mice treated either with clodrolip or CSF1R moAb. Rag2^{-/-}γ_{c}^{-/-} mice transplanted i.v. with MEC1 cells and injected with clodrolip (i.v., days +11, +25) and with etanercept (i.p., starting at day +10) were sacrificed 24 hours after the last clodrolip injection (Figure 12H). Etanercept abated clodrolip-induced leukemic cell depletion in the BM (Figure 12I). In xeno-transplanted mice treated i.v. with the CSF1R moAb (days +11, +25) and sacrificed 96h after the last injection of the moAb (Figure 7J), the anti-leukemic effect was abrogated in the SP when TNF pathway was blocked in vivo by etanercept (Figure 12K).

Furthermore, we functionally inactivated the FAS/FASL signaling by using the blocking Ab (clone Kay10, Biolegend) in the TCL1-transplantation system. FASL blocking did not abrogate the leukemic cell depletion of transplanted mice treated with clodrolip (Figure 11D-E-F-G) or with aCSF1R (hMab 2F11-e7) (Figure 11H-I-J). The same clodrolip results were confirmed by transplanting leukemic B cells into Faslgld mice, carrying a null-function mutation of the Fas1 gene (Figure 11E-F-G).

These findings let us conclude that macrophage killing sensitizes leukemic cells to apoptosis mainly via induction of TNF signaling.

### TARGETING HUMAN PRIMARY CLL CELLS BY MONOCYTE/MACROPHAGE KILLING

Finally, we applied the relevant molecular and functional information gathered in mouse models to human samples. We first analyzed by immunohistochemistry LN sections from CLL patients and observed the proximity of CD68+ macrophages to proliferating (Ki67+) CLL cells in the proliferation centers (Figure 13A). As a next step we evaluated whether human primary leukemic cells could be affected by clodrolip. Clodrolip had no direct toxic effect in vitro on leukemic B cells, as shown by cytotoxicity studies performed on MEC1 cells (Figure 11A) as well as on purified human primary CLL cells (Figure 13B). However, when unfractionated peripheral blood mononuclear cells (PBMCs) of CLL patients (which contained both leukemic and normal hematopoietic cells) were treated with different doses of clodrolip, we observed a marked depletion of both CD14+ monocytes and leukemic B cells, as early as 30 minutes after treatment (Figure 13C) and even more markedly after 24h (Figure 13D). The massive leukemic cell death induced by monocyte killing is shown in Figure 13E. Transwell experiments, performed by seeding PBMCs from CLL patients depleted of monocytes, showed that CLL death is not necessarily induced by cell-cell contacts (Figure 13F). We then evaluated the expression of key effector molecules involved in cell death mechanisms in human primary leukemic cells. To this aim we purified by magnetic negative selection leukemic cells from PBMCs of 24h-clodrolip cultures and untreated control cultures. We observed a significant upregulation of FAS in all the treated samples analyzed (Figure 14A, n=3). Besides FAS, we observed the upregulation of TNFR1, FADD, BID, TRAIL-R2 in one patient's sample (Figure 14B), suggesting that several pathways of cell death (e.g. FAS/FASL, TRAIL and TNF) may be involved also in human primary CLL cell killing induced by monocytes/macrophages.

To investigate the involvement of TNF and TRAIL in the mechanism of leukemia cell death, we utilized etanercept and a blocking anti-human TRAIL-R2 moAb (Germano G CC 2013). As shown in Figure 14C-D, in 3 out of 4 samples, the leukemic cell depletion induced by clodrolip was reduced when TNF (Figure S6C) and TRAIL signaling (Figure 14D) were blocked. To conclusively test the efficacy of a macrophage targeting strategy on primary cells from CLL patients, PBMCs were treated with anti-human CSF1R moAb (hMab 2F1 1-e7) (Figure 13G). We observed a relevant depletion of both CD14+ monocytes and leukemic B cells after 48h. More strikingly, the leukemic cell depletion increased significantly when the anti-human CSF1R moAb (hMab 2F11-e7) was associated to GA101 (Figure 13G).

Together, these findings indicate that macrophage killing either restores CLL sensitivity to apoptosis or directly induces their death. They further support the rationale translating these findings into novel combination therapies.

Our data on the anti-CSFIR moAb, known to prevent the formation of new macrophages by inducing apoptosis or inhibiting monocyte differentiation, substantiate the dependence of the leukemic clone on monocytes/macrophages especially in the BM niche, where normally CSF1 induces differentiation and maturation of monocytes {Ries, 2014 #16;MacDonald, 2010 #36}. The proof of principle that monocyte/macrophage depletion results in an anti-leukemic effect was obtained by clodrolip killing. The results obtained with the therapeutically relevant anti-CSFIR moAb paralleled those obtained with clodrolip. In different mouse models macrophage targeting impairs CLL cell engraftment and, even more interestingly associates with a striking anti-leukemic effect and a significant improvement of mouse survival.

The depletion of the monocyte/macrophage pool goes along with the apoptosis of leukemic cells. The molecular mechanisms accounting for the leukemic cell death in vivo appear to entail the RNA upregulation of key molecules of the TNF pathway. Macrophage targeting sensitizes leukemic cells to apoptosis via induction of TNF signaling and triggers their death through a TNF-dependent mechanism. Our in vitro findings on human primary CLL cells and the survival improvement of xeno-transplanted mice upon the combined treatment, corroborated the strong potential of this innovative strategy.

## Claims

1. A combination of an antibody which binds to CSF-1R and an antibody which binds CD 20 for use in inducing lymphocytosis of leukemic cells in lymphomas or leukemias,
wherein the lymphocytosis increases the percentage of CD20 expressing circulating leukemic cells and renders the lymphoma or leukemia susceptible to a treatment with an anti-CD20 antibody, and wherein
wherein the antibody which binds to human CSF-1R used in the combination therapy comprises
a) a heavy chain variable domain VH of SEQ ID NO:23 and a light chain variable domain VL of SEQ ID NO:24, or
b) a heavy chain variable domain VH of SEQ ID NO:31 and a light chain variable domain VL of SEQ ID NO:32, or
c) a heavy chain variable domain VH of SEQ ID NO:39 and a light chain variable domain VL of SEQ ID NO:40, or
d) a heavy chain variable domain VH of SEQ ID NO:47 and a light chain variable domain VL of SEQ ID NO:48, or
e) a heavy chain variable domain VH of SEQ ID NO:55 and a light chain variable domain VL of SEQ ID NO:56; and
wherein the antibody which binds to human CD20 used in the combination therapy comprises
a) a heavy chain variable domain VH of SEQ ID NO:69 and a light chain variable domain VL of SEQ ID NO:76, or
b) a heavy chain variable domain VH of SEQ ID NO:70 and a light chain variable domain VL of SEQ ID NO:76, or
c) a heavy chain variable domain VH of SEQ ID NO:71 and a light chain variable domain VL of SEQ ID NO:76, or
d) a heavy chain variable domain VH of SEQ ID NO:72 and a light chain variable domain VL of SEQ ID NO:76, or
e) a heavy chain variable domain VH of SEQ ID NO:73 and a light chain variable domain VL of SEQ ID NO:76, or
f) a heavy chain variable domain VH of SEQ ID NO:74 and a light chain variable domain VL of SEQ ID NO:76, or
g) a heavy chain variable domain VH of SEQ ID NO:75 and a light chain variable domain VL of SEQ ID NO:76.

2. The combination for use according to claim 1,
wherein the antibody which binds to human CSF-1R used in the combination therapy comprises
a heavy chain variable domain VH of SEQ ID NO:39 and a light chain variable domain VL of SEQ ID NO:40,and
wherein the antibody which binds to human CD20 used in the combination therapy comprises
a heavy chain variable domain VH of SEQ ID NO:71 and a light chain variable domain VL of SEQ ID NO:76

3. The combination for use according to any of the preceding claims, wherein the antibody which binds to human CSF-1R used in the combination therapy comprises
a heavy chain variable domain VH of SEQ ID NO:39 and a light chain variable domain VL of SEQ ID NO:40,and
wherein the antibody which binds to human CD20 used in the combination therapy is an afucosylated antibody of IgG1 isotype having an altered pattern of glycosylation in the Fc region wherein the amount of fucose containing oligosaccharides is between 40% and 60% of the total amount of oligosaccharides at Asn297; and comprises a heavy chain variable domain VH of SEQ ID NO:71 and a light chain variable domain VL of SEQ ID NO:76.

4. A combination of an antibody which binds to human CSF-1R and an antibody which binds to human CD20 wherein the combination is
i) for use in the treatment of a CD20 expressing cancer; or
ii) for use in stimulating an immune response or function, such as T cell activity; or
iii) for use in stimulating a cell mediated immune response, particularly stimulating cytotoxic T-lymphocytes, stimulating T cell activity, or stimulating macrophage activity; or
iv) for use in delaying progression of cancer; or
v) for use in prolonging the survival of a patient suffering from cancer.
and wherein the antibody which binds to human CSF-1R used in the combination therapy is **characterized in** comprising
a) a heavy chain variable domain VH of SEQ ID NO:23 and a light chain variable domain VL of SEQ ID NO:24, or
b) a heavy chain variable domain VH of SEQ ID NO:31 and a light chain variable domain VL of SEQ ID NO:32, or
c) a heavy chain variable domain VH of SEQ ID NO:39 and a light chain variable domain VL of SEQ ID NO:40, or
d) a heavy chain variable domain VH of SEQ ID NO:47 and a light chain variable domain VL of SEQ ID NO:48, or
e) a heavy chain variable domain VH of SEQ ID NO:55 and a light chain variable domain VL of SEQ ID NO:56;
and wherein the antibody which binds to human CD20 used in the combination therapy is **characterized in** comprising
a) a heavy chain variable domain VH of SEQ ID NO:69 and a light chain variable domain VL of SEQ ID NO:76, or
b) a heavy chain variable domain VH of SEQ ID NO:70 and a light chain variable domain VL of SEQ ID NO:76, or
c) a heavy chain variable domain VH of SEQ ID NO:71 and a light chain variable domain VL of SEQ ID NO:76, or
d) a heavy chain variable domain VH of SEQ ID NO:72 and a light chain variable domain VL of SEQ ID NO:76, or
e) a heavy chain variable domain VH of SEQ ID NO:73 and a light chain variable domain VL of SEQ ID NO:76, or
f) a heavy chain variable domain VH of SEQ ID NO:74 and a light chain variable domain VL of SEQ ID NO:76, or
g) a heavy chain variable domain VH of SEQ ID NO:75 and a light chain variable domain VL of SEQ ID NO:76.

5. The combination for use according to claim 4, wherein the antibody which binds to human CSF-1R used in the combination therapy comprises
a heavy chain variable domain VH of SEQ ID NO:39 and a light chain variable domain VL of SEQ ID NO:40,and
wherein the antibody which binds to human CD20 used in the combination therapy comprises
a heavy chain variable domain VH of SEQ ID NO:71 and a light chain variable domain VL of SEQ ID NO:76

6. The combination for use according to claim 5, wherein the antibody which binds to human CSF-1R used in the combination therapy comprises
a heavy chain variable domain VH of SEQ ID NO:39 and a light chain variable domain VL of SEQ ID NO:40,and
wherein the antibody which binds to human CD20 used in the combination therapy is an afucosylated antibody of IgG1 isotype having an altered pattern of glycosylation in the Fc region wherein the amount of fucose containing oligosaccharides is between 40% and 60% of the total amount of oligosaccharides at Asn297; and comprises a heavy chain variable domain VH of SEQ ID NO:71 and a light chain variable domain VL of SEQ ID NO:76.

7. The combination for use according to any one of the preceding claims for use in the treatment of lymphomas and lymphocytic leukemias.

8. The combination for use according to any one of the preceding claims, for use in the treatment of B-Cell Non-Hodgkin's lymphomas (NHL).

9. The combination for use according to any one of the preceding claims, for use in the treatment of multiple myeloma, of follicular lymphoma, or of Hodgkin's disease.

10. The combination for use according to any one of the preceding claims, wherein the cancer, lymphoma and leukemia expressed CD20.

11. The combination for use according to any one of the preceding claims, for use in treating or delaying progression of an immune related disease such as tumor immunity.

12. The combination for use according to any one of the preceding claims, for use in stimulating an immune response or function, such as T cell activity.

13. The combination for use according to any one of the preceding claims, for use in the prevention or treatment of metastasis.

14. The combination for use according to any one of the preceding claims, for use in the treatment of inflammatory diseases.

15. The combination for use according to any one of the preceding claims, wherein the antibody which binds to human CSF-R and the antibody which binds to human CD20 are of human IgG1 subclass.

16. The combination for use according to any one of the preceding claims, wherein no additional chemotherapeutic agents and/or targeted therapy is administered in addition to the anti-CSF-1R antibody and anti-CD20 antibody combination therapy.

17. The combination for use according to any of the preceding claims, wherein the antibody that binds to CSF-1R and the antibody that binds to human CD20 are co-administered simultaneously.

18. The combination for use according to any of the preceding claims, wherein the antibody that binds to CSF-1R and the antibody that binds to human CD20 are co-administered sequentially.

19. The combination for use according to any one of the preceding claims, wherein in addition to the anti-CSF-1R antibody in combination with the anti-CD20 antibody also an anti-PD-Ll antibody is administered.

20. The combination for use according to claim 19, wherein the antibody that binds to PD-L1 that is used comprises variable domain amino acid sequences, selected from the group of:
- variable heavy chain domain VH of SEQ ID NO: 78, and variable light chain domain VL of SEQ ID NO: 81 (corresponding to the VH and VL domains of <PD-L1> "243.55.S70" as disclosed herein);
- variable heavy chain domain VH of SEQ ID NO: 79, and variable light chain domain VL of SEQ ID NO: 82 (corresponding to the VH and VL domains of <PD-L1> "243.55.H1" as disclosed herein);
- variable heavy chain domain VH of SEQ ID NO: 79, and variable light chain domain VL of SEQ ID NO: 83 (corresponding to the VH and VL domains of <PD-L1> "243.55.H12" as disclosed herein);
- variable heavy chain domain VH of SEQ ID NO: 79, and variable light chain domain VL of SEQ ID NO: 84 (corresponding to the VH and VL domains of <PD-L1> "243.55.H37" as disclosed herein);
- variable heavy chain domain VH of SEQ ID NO: 79, and variable light chain domain VL of SEQ ID NO: 85 (corresponding to the VH and VL domains of <PD-L1> "243.55.H70" as disclosed herein);
- variable heavy chain domain VH of SEQ ID NO: 79, and variable light chain domain VL of SEQ ID NO: 86 (corresponding to the VH and VL domains of <PD-L1> "243.55.H89" as disclosed herein);
- variable heavy chain domain VH of SEQ ID NO: 79, and variable light chain domain VL of SEQ ID NO: 87 (corresponding to the VH and VL domains of <PD-L1> "243.55.S1" as disclosed herein);
- variable heavy chain domain VH of SEQ ID NO: 79, and variable light chain domain VL of SEQ ID NO: 88 (corresponding to the VH and VL domains of <PD-L1> "243.55.5" as disclosed herein);
- variable heavy chain domain VH of SEQ ID NO: 79, and variable light chain domain VL of SEQ ID NO: 89 (corresponding to the VH and VL domains of <PD-L1> "243.55.8" as disclosed herein);
- variable heavy chain domain VH of SEQ ID NO: 79, and variable light chain domain VL of SEQ ID NO: 90 (corresponding to the VH and VL domains of <PD-L1> "243.55.30" as disclosed herein);
- variable heavy chain domain VH of SEQ ID NO: 79, and variable light chain domain VL of SEQ ID NO: 91 (corresponding to the VH and VL domains of <PD-L1> "243.55.34" as disclosed herein);
- variable heavy chain domain VH of SEQ ID NO: 79, and variable light chain domain VL of SEQ ID NO: 92 (corresponding to the VH and VL domains of <PD-L1> "243.55.S37" as disclosed herein);
- variable heavy chain domain VH of SEQ ID NO: 79, and variable light chain domain VL of SEQ ID NO: 93 (corresponding to the VH and VL domains of <PD-L1> "243.55.49" as disclosed herein);
- variable heavy chain domain VH of SEQ ID NO: 79, and variable light chain domain VL of SEQ ID NO: 94 (corresponding to the VH and VL domains of <PD-L1> "243.55.51" as disclosed herein);
- variable heavy chain domain VH of SEQ ID NO: 79, and variable light chain domain VL of SEQ ID NO: 95 (corresponding to the VH and VL domains of <PD-L1> "243.55.62" as disclosed herein); and
- variable heavy chain domain VH of SEQ ID NO: 80, and variable light chain domain VL of SEQ ID NO: 96 (corresponding to the VH and VL domains of <PD-L1> "243.55.84" as disclosed herein).

21. The combination for use according to claim 19 wherein the antibody that binds to PD-L1 is atezolizumab.

22. The combination for use according to any one of claims 19 to 21, wherein the antibody that binds to CSF-1R, the antibody that binds to human CD20, and the antibody that binds to human PD-L1 are co-administered simultaneously.

23. The combination for use according to any one of claims 19 to 21, wherein the antibody that binds to CSF-1R, the antibody that binds to human CD20, and the antibody that binds to human PD-L1 are co-administered sequentially.

24. The combination for use according to any one of the preceding claims, wherein in addition to the anti-CSF-1R antibody in combination with the anti-CD20 antibody also an anti-PD-1 antibody is administered.

25. The combination for use according to claim 24, wherein the antibody that binds to CSF-1R, the antibody that binds to human CD20, and the antibody that binds to human PD-1 are co-administered simultaneously.

26. The combination for use according to claim 24, wherein the antibody that binds to CSF-1R, the antibody that binds to human CD20, and the antibody that binds to human PD-1 are co-administered sequentially.

## Patentansprüche

1. Kombination aus einem Antikörper, der an CSF-1R bindet, und einem Antikörper, der an CD20 bindet, zur Verwendung beim Induzieren von Lymphozytose von Leukämiezellen in Lymphomen oder Leukämien,
wobei die Lymphozytose den Prozentsatz an CD20-exprimierenden zirkulierenden Leukämiezellen erhöht und das Lymphom oder die Leukämie für eine Behandlung mit einem Anti-CD20-Antikörper empfänglich macht, und wobei
wobei der Antikörper, der an humanes CSF-1R bindet, das in der Kombinationstherapie verwendet wird,
a) eine variable Domäne der schweren Kette VH von SEQ ID NO:23 und eine variable Domäne der leichten Kette VL von SEQ ID NO:24, oder
b) eine variable Domäne der schweren Kette VH von SEQ ID NO:31 und eine variable Domäne der leichten Kette VL von SEQ ID NO:32, oder
c) eine variable Domäne der schweren Kette VH von SEQ ID NO:39 und eine variable Domäne der leichten Kette VL von SEQ ID NO:40, oder
d) eine variable Domäne der schweren Kette VH von SEQ ID NO:47 und eine variable Domäne der leichten Kette VL von SEQ ID NO:48, oder
e) eine variable Domäne der schweren Kette VH von SEQ ID NO:55 und eine variable Domäne der leichten Kette VL von SEQ ID NO:56 umfasst; und
wobei der Antikörper, der an humanes CD20 bindet, das in der Kombinationstherapie verwendet wird,
a) eine variable Domäne der schweren Kette VH von SEQ ID NO:69 und eine variable Domäne der leichten Kette VL von SEQ ID NO:76, oder
b) eine variable Domäne der schweren Kette VH von SEQ ID NO:70 und eine variable Domäne der leichten Kette VL von SEQ ID NO:76, oder
c) eine variable Domäne der schweren Kette VH von SEQ ID NO:71 und eine variable Domäne der leichten Kette VL von SEQ ID NO:76, oder
d) eine variable Domäne der schweren Kette VH von SEQ ID NO:72 und eine variable Domäne der leichten Kette VL von SEQ ID NO:76, oder
e) eine variable Domäne der schweren Kette VH von SEQ ID NO:73 und eine variable Domäne der leichten Kette VL von SEQ ID NO:76, oder
f) eine variable Domäne der schweren Kette VH von SEQ ID NO:74 und eine variable Domäne der leichten Kette VL von SEQ ID NO:76, oder
g) eine variable Domäne der schweren Kette VH von SEQ ID NO:75 und eine variable Domäne der leichten Kette VL von SEQ ID NO:76 umfasst.

2. Kombination zur Verwendung nach Anspruch 1,
wobei der Antikörper, der an humanes CSF-1R bindet, das in der Kombinationstherapie verwendet wird,
eine variable Domäne der schweren Kette VH von SEQ ID NO:39 und eine variable Domäne der leichten Kette VL von SEQ ID NO:40 umfasst, und
wobei der Antikörper, der an humanes CD20 bindet, das in der Kombinationstherapie verwendet wird,
eine variable Domäne der schweren Kette VH von SEQ ID NO:71 und eine variable Domäne der leichten Kette VL von SEQ ID NO:76 umfasst.

3. Kombination zur Verwendung nach einem der vorhergehenden Ansprüche,
wobei der Antikörper, der an humanes CSF-1R bindet, das in der Kombinationstherapie verwendet wird,
eine variable Domäne der schweren Kette VH von SEQ ID NO:39 und eine variable Domäne der leichten Kette VL von SEQ ID NO:40 umfasst, und
wobei der Antikörper, der an humanes CD20 bindet, das in der Kombinationstherapie verwendet wird, ein afucosylierter Antikörper des IgG1-Isotyps mit einem veränderten Glycosylierungsmuster in der Fc-Region ist, wobei die Menge an Fucose enthaltenden Oligosacchariden zwischen 40 % und 60 % der Gesamtmenge an Oligosacchariden an Asn297 liegt; und eine variable Domäne der schweren Kette VH von SEQ ID NO:71 und eine variable Domäne der leichten Kette VL von SEQ ID NO:76 umfasst.

4. Kombination aus einem Antikörper, der an humanes CSF-1R bindet, und einem Antikörper, der an humanes CD20 bindet, wobei die Kombination
i) zur Verwendung bei der Behandlung eines CD20-exprimierenden Krebses; oder
ii) zur Verwendung bei der Stimulierung einer Immunantwort oder -funktion, wie T-Zellenaktivität; oder
iii) zur Verwendung bei der Stimulierung einer zellvermittelten Immunantwort, insbesondere Stimulierung zytotoxischer T-Lymphozyten, Stimulierung von T-Zellenaktivität oder Stimulierung von Makrophagenaktivität; oder
iv) zur Verwendung bei der Verzögerung des Fortschreitens des Krebses; oder
v) zur Verwendung bei der Verlängerung des Überlebens eines Patienten, der an Krebs leidet, vorgesehen ist;
und wobei der Antikörper, der an humanes CSF-1R bindet, das in der Kombinationstherapie verwendet wird, **dadurch gekennzeichnet ist, dass** er
a) eine variable Domäne der schweren Kette VH von SEQ ID NO:23 und eine variable Domäne der leichten Kette VL von SEQ ID NO:24, oder
b) eine variable Domäne der schweren Kette VH von SEQ ID NO:31 und eine variable Domäne der leichten Kette VL von SEQ ID NO:32, oder
c) eine variable Domäne der schweren Kette VH von SEQ ID NO:39 und eine variable Domäne der leichten Kette VL von SEQ ID NO:40, oder
d) eine variable Domäne der schweren Kette VH von SEQ ID NO:47 und eine variable Domäne der leichten Kette VL von SEQ ID NO:48, oder
e) eine variable Domäne der schweren Kette VH von SEQ ID NO:55 und eine variable Domäne der leichten Kette VL von SEQ ID NO:56 umfasst;
und wobei der Antikörper, der an humanes CD20 bindet, das in der Kombinationstherapie verwendet wird, **dadurch gekennzeichnet ist, dass** er
a) eine variable Domäne der schweren Kette VH von SEQ ID NO:69 und eine variable Domäne der leichten Kette VL von SEQ ID NO:76, oder
b) eine variable Domäne der schweren Kette VH von SEQ ID NO:70 und eine variable Domäne der leichten Kette VL von SEQ ID NO:76, oder
c) eine variable Domäne der schweren Kette VH von SEQ ID NO:71 und eine variable Domäne der leichten Kette VL von SEQ ID NO:76, oder
d) eine variable Domäne der schweren Kette VH von SEQ ID NO:72 und eine variable Domäne der leichten Kette VL von SEQ ID NO:76, oder
e) eine variable Domäne der schweren Kette VH von SEQ ID NO:73 und eine variable Domäne der leichten Kette VL von SEQ ID NO:76, oder
f) eine variable Domäne der schweren Kette VH von SEQ ID NO:74 und eine variable Domäne der leichten Kette VL von SEQ ID NO:76, oder
g) eine variable Domäne der schweren Kette VH von SEQ ID NO:75 und eine variable Domäne der leichten Kette VL von SEQ ID NO:76 umfasst.

5. Kombination zur Verwendung nach Anspruch 4,
wobei der Antikörper, der an humanes CSF-1R bindet, das in der Kombinationstherapie verwendet wird,
eine variable Domäne der schweren Kette VH von SEQ ID NO:39 und eine variable Domäne der leichten Kette VL von SEQ ID NO:40 umfasst, und
wobei der Antikörper, der an humanes CD20 bindet, das in der Kombinationstherapie verwendet wird,
eine variable Domäne der schweren Kette VH von SEQ ID NO:71 und eine variable Domäne der leichten Kette VL von SEQ ID NO:76 umfasst.

6. Kombination zur Verwendung nach Anspruch 5,
wobei der Antikörper, der an humanes CSF-1R bindet, das in der Kombinationstherapie verwendet wird,
eine variable Domäne der schweren Kette VH von SEQ ID NO:39 und eine variable Domäne der leichten Kette VL von SEQ ID NO:40 umfasst, und
wobei der Antikörper, der an humanes CD20 bindet, das in der Kombinationstherapie verwendet wird, ein afucosylierter Antikörper des IgG1-Isotyps mit einem veränderten Glycosylierungsmuster in der Fc-Region ist, wobei die Menge an Fucose enthaltenden Oligosacchariden zwischen 40 % und 60 % der Gesamtmenge an Oligosacchariden an Asn297 liegt; und eine variable Domäne der schweren Kette VH von SEQ ID NO:71 und eine variable Domäne der leichten Kette VL von SEQ ID NO:76 umfasst.

7. Kombination zur Verwendung nach einem der vorhergehenden Ansprüche zur Verwendung bei der Behandlung von Lymphomen und lymphatischen Leukämien.

8. Kombination zur Verwendung nach einem der vorhergehenden Ansprüche zur Verwendung bei der Behandlung von B-Zellen-Non-Hodgkin-Lymphomen (NHL).

9. Kombination zur Verwendung nach einem der vorhergehenden Ansprüche zur Verwendung bei der Behandlung von multiplem Myelom, follikulärem Lymphom oder der Hodgkin-Krankheit.

10. Kombination zur Verwendung nach einem der vorhergehenden Ansprüche, wobei der Krebs, das Lymphom und die Leukämie CD20 exprimierten.

11. Kombination zur Verwendung nach einem der vorhergehenden Ansprüche zur Verwendung bei der Behandlung oder Verzögerung des Fortschreitens einer Immunkrankheit wie Tumorimmunität.

12. Kombination zur Verwendung nach einem der vorhergehenden Ansprüche zur Verwendung bei der Stimulierung einer Immunantwort oder -funktion, wie T-Zellenaktivität.

13. Kombination zur Verwendung nach einem der vorhergehenden Ansprüche zur Verwendung bei der Vorbeugung oder Behandlung von Metastasen.

14. Kombination zur Verwendung nach einem der vorhergehenden Ansprüche zur Verwendung bei der Behandlung von Entzündungskrankheiten.

15. Kombination zur Verwendung nach einem der vorhergehenden Ansprüche, wobei der Antikörper, der an humanes CSF-R bindet, und der Antikörper, der an humanes CD20 bindet, von der humanen IgG1-Unterklasse sind.

16. Kombination zur Verwendung nach einem der vorhergehenden Ansprüche, wobei neben der Anti-CSF-1R-Antikörper- und Anti-CD20-Antikörper-Kombinationstherapie keine zusätzlichen Chemotherapeutika und/oder zielgerichtete Therapie verabreicht werden.

17. Kombination zur Verwendung nach einem der vorhergehenden Ansprüche, wobei der Antikörper, der an CSF-1R bindet, und der Antikörper, der an humanes CD20 bindet, gleichzeitig verabreicht werden.

18. Kombination zur Verwendung nach einem der vorhergehenden Ansprüche, wobei der Antikörper, der an CSF-1R bindet, und der Antikörper, der an humanes CD20 bindet, nacheinander verabreicht werden.

19. Kombination zur Verwendung nach einem der vorhergehenden Ansprüche, wobei neben dem Anti-CSF-IR-Antikörper in Kombination mit dem Anti-CD20-Antikörper auch ein Anti-PD-Ll-Antikörper verabreicht wird.

20. Kombination zur Verwendung nach Anspruch 19, wobei der Antikörper, der an PD-L1 bindet, der verwendet wird, Aminosäuresequenzen einer variablen Domäne umfasst, die aus der folgenden Gruppe ausgewählt sind:
- der variablen Domäne der schweren Kette VH von SEQ ID NO:78 und der variablen Domäne der leichten Kette VL von SEQ ID NO:81 (die den VH- und VL-Domänen von <PD-L1> "243.55.S70", wie hierin offenbart, entsprechen);
- der variablen Domäne der schweren Kette VH von SEQ ID NO:79 und der variablen Domäne der leichten Kette VL von SEQ ID NO:82 (die den VH- und VL-Domänen von <PD-L1> "243.55.H1", wie hierin offenbart, entsprechen);
- der variablen Domäne der schweren Kette VH von SEQ ID NO:79 und der variablen Domäne der leichten Kette VL von SEQ ID NO:83 (die den VH- und VL-Domänen von <PD-L1> "243.55.H12", wie hierin offenbart, entsprechen);
- der variablen Domäne der schweren Kette VH von SEQ ID NO:79 und der variablen Domäne der leichten Kette VL von SEQ ID NO:84 (die den VH- und VL-Domänen von <PD-L1> "243.55.H37", wie hierin offenbart, entsprechen);
- der variablen Domäne der schweren Kette VH von SEQ ID NO:79 und der variablen Domäne der leichten Kette VL von SEQ ID NO:85 (die den VH- und VL-Domänen von <PD-L1> "243.55.H70", wie hierin offenbart, entsprechen);
- der variablen Domäne der schweren Kette VH von SEQ ID NO:79 und der variablen Domäne der leichten Kette VL von SEQ ID NO:86 (die den VH- und VL-Domänen von <PD-L1> "243.55.H89", wie hierin offenbart, entsprechen);
- der variablen Domäne der schweren Kette VH von SEQ ID NO:79 und der variablen Domäne der leichten Kette VL von SEQ ID NO:87 (die den VH- und VL-Domänen von <PD-L1> "243.55.S1", wie hierin offenbart, entsprechen);
- der variablen Domäne der schweren Kette VH von SEQ ID NO:79 und der variablen Domäne der leichten Kette VL von SEQ ID NO:88 (die den VH- und VL-Domänen von <PD-L1> "243.55.5", wie hierin offenbart, entsprechen);
- der variablen Domäne der schweren Kette VH von SEQ ID NO:79 und der variablen Domäne der leichten Kette VL von SEQ ID NO:89 (die den VH- und VL-Domänen von <PD-L1> "243.55.8", wie hierin offenbart, entsprechen);
- der variablen Domäne der schweren Kette VH von SEQ ID NO:79 und der variablen Domäne der leichten Kette VL von SEQ ID NO:90 (die den VH- und VL-Domänen von <PD-L1> "243.55.30", wie hierin offenbart, entsprechen);
- der variablen Domäne der schweren Kette VH von SEQ ID NO:79 und der variablen Domäne der leichten Kette VL von SEQ ID NO:91 (die den VH- und VL-Domänen von <PD-L1> "243.55.34", wie hierin offenbart, entsprechen);
- der variablen Domäne der schweren Kette VH von SEQ ID NO:79 und der variablen Domäne der leichten Kette VL von SEQ ID NO:92 (die den VH- und VL-Domänen von <PD-L1> "243.55.S37", wie hierin offenbart, entsprechen);
- der variablen Domäne der schweren Kette VH von SEQ ID NO:79 und der variablen Domäne der leichten Kette VL von SEQ ID NO:93 (die den VH- und VL-Domänen von <PD-L1> "243.55.49", wie hierin offenbart, entsprechen);
- der variablen Domäne der schweren Kette VH von SEQ ID NO:79 und der variablen Domäne der leichten Kette VL von SEQ ID NO:94 (die den VH- und VL-Domänen von <PD-L1> "243.55.51", wie hierin offenbart, entsprechen);
- der variablen Domäne der schweren Kette VH von SEQ ID NO:79 und der variablen Domäne der leichten Kette VL von SEQ ID NO:95 (die den VH- und VL-Domänen von <PD-L1> "243.55.62", wie hierin offenbart, entsprechen); und
- der variablen Domäne der schweren Kette VH von SEQ ID NO:80 und der variablen Domäne der leichten Kette VL von SEQ ID NO:96 (die den VH- und VL-Domänen von <PD-L1> "243.55.84", wie hierin offenbart, entsprechen).

21. Kombination zur Verwendung nach Anspruch 19, wobei der Antikörper, der an PD-L1 bindet, Atezolizumab ist.

22. Kombination zur Verwendung nach einem der Ansprüche 19 bis 21, wobei der Antikörper, der an CSF-1R bindet, der Antikörper, der an humanes CD20 bindet, und der Antikörper, der an humanes PD-L1 bindet, gleichzeitig verabreicht werden.

23. Kombination zur Verwendung nach einem der Ansprüche 19 bis 21, wobei der Antikörper, der an CSF-1R bindet, der Antikörper, der an humanes CD20 bindet, und der Antikörper, der an humanes PD-L1 bindet, nacheinander verabreicht werden.

24. Kombination zur Verwendung nach einem der vorhergehenden Ansprüche, wobei neben dem Anti-CSF-IR-Antikörper in Kombination mit dem Anti-CD20-Antikörper auch ein Anti-PD-1-Antikörper verabreicht wird.

25. Kombination zur Verwendung nach Anspruch 24, wobei der Antikörper, der an CSF-1R bindet, der Antikörper, der an humanes CD20 bindet, und der Antikörper, der an humanes PD-1 bindet, gleichzeitig verabreicht werden.

26. Kombination zur Verwendung nach Anspruch 24, wobei der Antikörper, der an CSF-1R bindet, der Antikörper, der an humanes CD20 bindet, und der Antikörper, der an humanes PD-1 bindet, nacheinander verabreicht werden.

## Revendications

1. Association d'un anticorps qui se lie au CSF-1R et d'un anticorps qui se lie au CD20 pour une utilisation dans l'induction d'une lymphocytose de cellules leucémiques dans des lymphomes ou des leucémies,
dans laquelle la lymphocytose augmente le pourcentage de cellules leucémiques circulantes exprimant le CD20 et rend le lymphome ou la leucémie sensible à un traitement avec un anticorps anti-CD20, et dans laquelle
dans laquelle l'anticorps qui se lie au CSF-1R humain utilisé dans l'association thérapeutique comprend
a) un domaine variable de chaîne lourde VH de SEQ ID NO : 23 et un domaine variable de chaîne légère VL de SEQ ID NO : 24, ou
b) un domaine variable de chaîne lourde VH de SEQ ID NO : 31 et un domaine variable de chaîne légère VL de SEQ ID NO : 32, ou
c) un domaine variable de chaîne lourde VH de SEQ ID NO : 39 et un domaine variable de chaîne légère VL de SEQ ID NO : 40, ou
d) un domaine variable de chaîne lourde VH de SEQ ID NO : 47 et un domaine variable de chaîne légère VL de SEQ ID NO : 48, ou
e) un domaine variable de chaîne lourde VH de SEQ ID NO : 55 et un domaine variable de chaîne légère VL de SEQ ID NO : 56 ; et
dans laquelle l'anticorps qui se lie au CD20 humain utilisé dans l'association thérapeutique comprend
a) un domaine variable de chaîne lourde VH de SEQ ID NO : 69 et un domaine variable de chaîne légère VL de SEQ ID NO : 76, ou
b) un domaine variable de chaîne lourde VH de SEQ ID NO : 70 et un domaine variable de chaîne légère VL de SEQ ID NO : 76, ou
c) un domaine variable de chaîne lourde VH de SEQ ID NO : 71 et un domaine variable de chaîne légère VL de SEQ ID NO : 76, ou
d) un domaine variable de chaîne lourde VH de SEQ ID NO : 72 et un domaine variable de chaîne légère VL de SEQ ID NO : 76, ou
e) un domaine variable de chaîne lourde VH de SEQ ID NO : 73 et un domaine variable de chaîne légère VL de SEQ ID NO : 76, ou
f) un domaine variable de chaîne lourde VH de SEQ ID NO : 74 et un domaine variable de chaîne légère VL de SEQ ID NO : 76, ou
g) un domaine variable de chaîne lourde VH de SEQ ID NO : 75 et un domaine variable de chaîne légère VL de SEQ ID NO : 76.

2. Association pour une utilisation selon la revendication 1,
dans laquelle l'anticorps qui se lie au CSF-1R humain utilisé dans l'association thérapeutique comprend
un domaine variable de chaîne lourde VH de SEQ ID NO : 39 et un domaine variable de chaîne légère VL de SEQ ID NO : 40, et
dans laquelle l'anticorps qui se lie au CD20 humain utilisé dans l'association thérapeutique comprend
un domaine variable de chaîne lourde VH de SEQ ID NO : 71 et un domaine variable de chaîne légère VL de SEQ ID NO : 76.

3. Association pour une utilisation selon l'une quelconque des revendications précédentes,
dans laquelle l'anticorps qui se lie au CSF-1R humain utilisé dans l'association thérapeutique comprend
un domaine variable de chaîne lourde VH de SEQ ID NO : 39 et un domaine variable de chaîne légère VL de SEQ ID NO : 40, et
dans laquelle l'anticorps qui se lie au CD20 humain utilisé dans l'association thérapeutique est un anticorps afucosylé d'isotype IgG1 ayant un motif de glycosylation altéré dans la région Fc dans laquelle la quantité d'oligosaccharides contenant du fucose est entre 40 % et 60 % de la quantité totale d'oligosaccharides en Asn297 ; et comprend un domaine variable de chaîne lourde VH de SEQ ID NO : 71 et un domaine variable de chaîne légère VL de SEQ ID NO : 76.

4. Association d'un anticorps qui se lie au CSF-1R humain et d'un anticorps qui se lie au CD20 humain dans laquelle l'association est
i) pour une utilisation dans le traitement d'un cancer exprimant le CD20 ; ou
ii) pour une utilisation dans la stimulation d'une réponse ou d'une fonction immunitaire, telle qu'une activité des lymphocytes T ; ou
iii) pour une utilisation dans la stimulation d'une réponse immunitaire à médiation cellulaire, en particulier la stimulation des lymphocytes T cytotoxiques, la stimulation de l'activité des lymphocytes T ou la stimulation de l'activité des macrophages ; ou
iv) pour une utilisation dans le retardement de la progression du cancer ; ou
v) pour une utilisation dans la prolongation de la survie d'un patient souffrant du cancer ;
et dans laquelle l'anticorps qui se lie au CSF-1R humain utilisé dans l'association thérapeutique est **caractérisé en ce qu'**il comprend
a) un domaine variable de chaîne lourde VH de SEQ ID NO : 23 et un domaine variable de chaîne légère VL de SEQ ID NO : 24, ou
b) un domaine variable de chaîne lourde VH de SEQ ID NO : 31 et un domaine variable de chaîne légère VL de SEQ ID NO : 32, ou
c) un domaine variable de chaîne lourde VH de SEQ ID NO : 39 et un domaine variable de chaîne légère VL de SEQ ID NO : 40, ou
d) un domaine variable de chaîne lourde VH de SEQ ID NO : 47 et un domaine variable de chaîne légère VL de SEQ ID NO : 48, ou
e) un domaine variable de chaîne lourde VH de SEQ ID NO : 55 et un domaine variable de chaîne légère VL de SEQ ID NO : 56 ;
et dans laquelle l'anticorps qui se lie au CD20 humain utilisé dans l'association thérapeutique est **caractérisé en ce qu'**il comprend
a) un domaine variable de chaîne lourde VH de SEQ ID NO : 69 et un domaine variable de chaîne légère VL de SEQ ID NO : 76, ou
b) un domaine variable de chaîne lourde VH de SEQ ID NO : 70 et un domaine variable de chaîne légère VL de SEQ ID NO : 76, ou
c) un domaine variable de chaîne lourde VH de SEQ ID NO : 71 et un domaine variable de chaîne légère VL de SEQ ID NO : 76, ou
d) un domaine variable de chaîne lourde VH de SEQ ID NO : 72 et un domaine variable de chaîne légère VL de SEQ ID NO : 76, ou
e) un domaine variable de chaîne lourde VH de SEQ ID NO : 73 et un domaine variable de chaîne légère VL de SEQ ID NO : 76, ou
f) un domaine variable de chaîne lourde VH de SEQ ID NO : 74 et un domaine variable de chaîne légère VL de SEQ ID NO : 76, ou
g) un domaine variable de chaîne lourde VH de SEQ ID NO : 75 et un domaine variable de chaîne légère VL de SEQ ID NO : 76.

5. Association pour une utilisation selon la revendication 4,
dans laquelle l'anticorps qui se lie au CSF-1R humain utilisé dans l'association thérapeutique comprend
un domaine variable de chaîne lourde VH de SEQ ID NO : 39 et un domaine variable de chaîne légère VL de SEQ ID NO : 40, et
dans laquelle l'anticorps qui se lie au CD20 humain utilisé dans l'association thérapeutique comprend
un domaine variable de chaîne lourde VH de SEQ ID NO : 71 et un domaine variable de chaîne légère VL de SEQ ID NO : 76.

6. Association pour une utilisation selon la revendication 5,
dans laquelle l'anticorps qui se lie au CSF-1R humain utilisé dans l'association thérapeutique comprend
un domaine variable de chaîne lourde VH de SEQ ID NO : 39 et un domaine variable de chaîne légère VL de SEQ ID NO : 40, et
dans laquelle l'anticorps qui se lie au CD20 humain utilisé dans l'association thérapeutique est un anticorps afucosylé d'isotype IgG1 ayant un motif de glycosylation altéré dans la région Fc dans laquelle la quantité d'oligosaccharides contenant du fucose est entre 40 % et 60 % de la quantité totale d'oligosaccharides en Asn297 ; et comprend un domaine variable de chaîne lourde VH de SEQ ID NO : 71 et un domaine variable de chaîne légère VL de SEQ ID NO : 76.

7. Association pour une utilisation selon l'une quelconque des revendications précédentes pour une utilisation dans le traitement de lymphomes et de leucémies lymphoïdes.

8. Association pour une utilisation selon l'une quelconque des revendications précédentes, pour une utilisation dans le traitement de lymphomes non hodgkiniens (LNH) à cellules B.

9. Association pour une utilisation selon l'une quelconque des revendications précédentes, pour une utilisation dans le traitement d'un myélome multiple, d'un lymphome folliculaire ou d'une maladie de Hodgkin.

10. Association pour une utilisation selon l'une quelconque des revendications précédentes, dans laquelle le cancer, le lymphome et la leucémie ont exprimé le CD20.

11. Association pour une utilisation selon l'une quelconque des revendications précédentes, pour une utilisation dans le traitement ou le retardement de la progression d'une maladie d'origine immunitaire telle qu'une immunité tumorale.

12. Association pour une utilisation selon l'une quelconque des revendications précédentes, pour une utilisation dans la stimulation d'une réponse ou d'une fonction immunitaire, telle qu'une activité des lymphocytes T.

13. Association pour une utilisation selon l'une quelconque des revendications précédentes, pour une utilisation dans la prévention ou le traitement de métastases.

14. Association pour une utilisation selon l'une quelconque des revendications précédentes, pour une utilisation dans le traitement de maladies inflammatoires.

15. Association pour une utilisation selon l'une quelconque des revendications précédentes, dans laquelle l'anticorps qui se lie au CSF-R humain et l'anticorps qui se lie au CD20 humain sont de la sous-classe IgG1 humaine.

16. Association pour une utilisation selon l'une quelconque des revendications précédentes, dans laquelle aucun agent chimiothérapeutique supplémentaire et/ou aucune thérapie ciblée n'est administré(e) en plus de l'association thérapeutique d'anticorps anti-CSF-1R et d'anticorps anti-CD20.

17. Association pour une utilisation selon l'une quelconque des revendications précédentes, dans laquelle l'anticorps qui se lie au CSF-1R et l'anticorps qui se lie au CD20 humain sont co-administrés simultanément.

18. Association pour une utilisation selon l'une quelconque des revendications précédentes, dans laquelle l'anticorps qui se lie au CSF-1R et l'anticorps qui se lie au CD20 humain sont co-administrés séquentiellement.

19. Association pour une utilisation selon l'une quelconque des revendications précédentes, dans laquelle en plus de l'anticorps anti-CSF-1R en association avec l'anticorps anti-CD20 un anticorps anti-PD-Ll est également administré.

20. Association pour une utilisation selon la revendication 19, dans laquelle l'anticorps qui se lie au PD-L1 qui est utilisé comprend des séquences d'acides aminés de domaines variables, choisies dans le groupe des :
- domaine variable de chaîne lourde VH de SEQ ID NO : 78 et domaine variable de chaîne légère VL de SEQ ID NO : 81 (correspondant aux domaines VH et VL de <PD-L1> « 243.55.S70 » comme décrit ici) ;
- domaine variable de chaîne lourde VH de SEQ ID NO : 79 et domaine variable de chaîne légère VL de SEQ ID NO : 82 (correspondant aux domaines VH et VL de <PD-L1> « 243.55.H1 » comme décrit ici) ;
- domaine variable de chaîne lourde VH de SEQ ID NO : 79 et domaine variable de chaîne légère VL de SEQ ID NO : 83 (correspondant aux domaines VH et VL de <PD-L1> « 243.55.H12 » comme décrit ici) ;
- domaine variable de chaîne lourde VH de SEQ ID NO : 79 et domaine variable de chaîne légère VL de SEQ ID NO : 84 (correspondant aux domaines VH et VL de <PD-L1> « 243.55.H37 » comme décrit ici) ;
- domaine variable de chaîne lourde VH de SEQ ID NO : 79 et domaine variable de chaîne légère VL de SEQ ID NO : 85 (correspondant aux domaines VH et VL de <PD-L1> « 243.55.H70 » comme décrit ici) ;
- domaine variable de chaîne lourde VH de SEQ ID NO : 79 et domaine variable de chaîne légère VL de SEQ ID NO : 86 (correspondant aux domaines VH et VL de <PD-L1> « 243.55.H89 » comme décrit ici) ;
- domaine variable de chaîne lourde VH de SEQ ID NO : 79 et domaine variable de chaîne légère VL de SEQ ID NO : 87 (correspondant aux domaines VH et VL de <PD-L1> « 243.55.S1 » comme décrit ici) ;
- domaine variable de chaîne lourde VH de SEQ ID NO : 79 et domaine variable de chaîne légère VL de SEQ ID NO : 88 (correspondant aux domaines VH et VL de <PD-L1> « 243.55.5 » comme décrit ici) ;
- domaine variable de chaîne lourde VH de SEQ ID NO : 79 et domaine variable de chaîne légère VL de SEQ ID NO : 89 (correspondant aux domaines VH et VL de <PD-L1> « 243.55.8 » comme décrit ici) ;
- domaine variable de chaîne lourde VH de SEQ ID NO : 79 et domaine variable de chaîne légère VL de SEQ ID NO : 90 (correspondant aux domaines VH et VL de <PD-L1> « 243.55.30 » comme décrit ici) ;
- domaine variable de chaîne lourde VH de SEQ ID NO : 79 et domaine variable de chaîne légère VL de SEQ ID NO : 91 (correspondant aux domaines VH et VL de <PD-L1> « 243.55.34 » comme décrit ici) ;
- domaine variable de chaîne lourde VH de SEQ ID NO : 79 et domaine variable de chaîne légère VL de SEQ ID NO : 92 (correspondant aux domaines VH et VL de <PD-L1> « 243.55.S37 » comme décrit ici) ;
- domaine variable de chaîne lourde VH de SEQ ID NO : 79 et domaine variable de chaîne légère VL de SEQ ID NO : 93 (correspondant aux domaines VH et VL de <PD-L1> « 243.55.49 » comme décrit ici) ;
- domaine variable de chaîne lourde VH de SEQ ID NO : 79 et domaine variable de chaîne légère VL de SEQ ID NO : 94 (correspondant aux domaines VH et VL de <PD-L1> « 243.55.51 » comme décrit ici) ;
- domaine variable de chaîne lourde VH de SEQ ID NO : 79 et domaine variable de chaîne légère VL de SEQ ID NO : 95 (correspondant aux domaines VH et VL de <PD-L1> « 243.55.62 » comme décrit ici) ; et
- domaine variable de chaîne lourde VH de SEQ ID NO : 80 et domaine variable de chaîne légère VL de SEQ ID NO : 96 (correspondant aux domaines VH et VL de <PD-L1> « 243.55.84 » comme décrit ici).

21. Association pour une utilisation selon la revendication 19 dans laquelle l'anticorps qui se lie au PD-L1 est l'atézolizumab.

22. Association pour une utilisation selon l'une quelconque des revendications 19 à 21,
dans laquelle l'anticorps qui se lie au CSF-1R, l'anticorps qui se lie au CD20 humain et l'anticorps qui se lie au PD-L1 humain sont co-administrés simultanément.

23. Association pour une utilisation selon l'une quelconque des revendications 19 à 21,
dans laquelle l'anticorps qui se lie au CSF-1R, l'anticorps qui se lie au CD20 humain et l'anticorps qui se lie au PD-L1 humain sont co-administrés séquentiellement.

24. Association pour une utilisation selon l'une quelconque des revendications précédentes,
dans laquelle en plus de l'anticorps anti-CSF-1R en association avec l'anticorps anti-CD20 un anticorps anti-PD-1 est également administré.

25. Association pour une utilisation selon la revendication 24,
dans laquelle l'anticorps qui se lie au CSF-1R, l'anticorps qui se lie au CD20 humain et l'anticorps qui se lie au PD-1 humain sont co-administrés simultanément.

26. Association pour une utilisation selon la revendication 24,
dans laquelle l'anticorps qui se lie au CSF-1R, l'anticorps qui se lie au CD20 humain et l'anticorps qui se lie au PD-1 humain sont co-administrés séquentiellement.
